# EUROPEAN PATENT APPLICATION

(11) **EP 3 705 574 A1**
(43) Date of publication of application: **09.09.2020**
(21) Application number: 18873933.8
(22) Date of filing: 31.10.2018
(51) Int. Cl.: C12N 15/62, C07K 14/705, C07K 19/00, C12N 5/10, C12N 9/02, C12N 15/63, C12Q 1/02, C12Q 1/66

(54) **SPLIT LUCIFERASE, AND HIGHLY SENSITIVE VITAMIN D RECEPTOR LIGAND DETECTION METHOD USING SAME**

(30) Priority: 31.10.2017 JP 2017210604
(71) Applicant: Public University Corporation Toyama Prefectural University, Imizu-shi, Toyama 939-0398 (JP)
(72) Inventor: SAKAKI, Toshiyuki, Imizu-shi Toyama 939-0398 (JP); NISHIKAWA, Miyu, Imizu-shi Toyama 939-0398 (JP); MANO, Hiroki, Imizu-shi Toyama 939-0398 (JP)
(74) Representative: Appleyard Lees IP LLP
(86) International application number: PCT/JP2018/040583
(87) International publication number: WO 2019/088200

(57) **Abstract**

The present invention pertains to any fusion protein in (1) to (3): (1) a fusion protein containing, in no particular order, the luciferase N-terminal domain (LucN), at least one vitamin D-binding domain of a vitamin D receptor (VDR), and the luciferase C-terminal domain (LucC), and able to additionally include, in no particular order, an LX1X2LL sequence and/or a linker sequence; (2) a fusion protein containing, in no particular order, LucN or LucC, and an LX1X2LL sequence, and able to additionally include, in no particular order, a linker sequence; and (3) a fusion protein containing, in no particular order, at least one vitamin D-binding domain of a VDR, and LucC or LucN, and able to additionally include, in no particular order, a linker sequence. The present invention also pertains to a method for screening, using the fusion protein, for substances which demonstrate the ability to bind to VDRs. The present invention provides a biosensor in which the luminescence increases when an agonist binds thereto, and provides a method for easily screening for substances which bind strongly to vitamin D receptors (VDRs).

### [Sequence Listing]

SEQUENCE LISTING

<110>  PUBLIC UNIVERSITY CORPORATION TOYAMA PREFECTURAL UNIVERSITY

<120>  Split luciferase and a sensitive detection method of vitamin D
       receptor ligand using thereof

<130>  181799H

<160>  119

<170>  PatentIn version 3.5

<210>  1
<211>  36
<212>  DNA
<213>  Artificial sequence

<220>
<223>  Artificial sequence

<400>  1
aattctcgag atggaggcaa tggcggccag cacttc                                36


<210>  2
<211>  36
<212>  DNA
<213>  Artificial sequence

<220>
<223>  Artificial sequence

<400>  2
atatgcggcc gctcaggaga tctcattgcc aaacac                                36


<210>  3
<211>  50
<212>  DNA
<213>  Artificial sequence

<220>
<223>  Artificial sequence

<400>  3
tacaaacgct ctcatcgaca aggaccggcc caagctgtct gaggagcagc                 50


<210>  4
<211>  50
<212>  DNA
<213>  Artificial sequence

<220>
<223>  Artificial sequence

<400>  4
cgatgtcgcc gctgtgcagc cagccggaga tctcattgcc aaacacttcg                 50


<210>  5
<211>  34

<212>  DNA
<213>  Artificial sequence

<220>
<223>  Artificial sequence

<400>  5
aattctcgag atggaagatg ccaaaaacat taag                                    34


<210>  6
<211>  50
<212>  DNA
<213>  Artificial sequence

<220>
<223>  Artificial sequence

<400>  6
gctgctcctc agacagcttg ggccggtcct tgtcgatgag agcgtttgta                  50


<210>  7
<211>  50
<212>  DNA
<213>  Artificial sequence

<220>
<223>  Artificial sequence

<400>  7
cgaagtgttt ggcaatgaga tctccggctg gctgcacagc ggcgacatcg                  50


<210>  8
<211>  40
<212>  DNA
<213>  Artificial sequence

<220>
<223>  Artificial sequence

<400>  8
atatgcggcc gcttacacgg cgatcttgcc gcccttcttg                              40


<210>  9
<211>  50
<212>  DNA
<213>  Artificial sequence

<220>
<223>  Artificial sequence

<400>  9
ggaggtagcg gtggcggtgg tagtctgccc cacctggctg acctggtcag                  50


<210>  10
<211>  49
<212>  DNA
<213>  Artificial sequence

<220>
<223>  Artificial sequence

<400>  10
ccctccacta cccccacctc cgtccttgtc gatgagagcg tttgtagcc                49


<210>  11
<211>  45
<212>  DNA
<213>  Artificial sequence

<220>
<223>  Artificial sequence

<400>  11
catgagcatc gggtggttgt ccttgtcgat gagagcgttt gtagc                    45


<210>  12
<211>  44
<212>  DNA
<213>  Artificial sequence

<220>
<223>  Artificial sequence

<400>  12
aaccttctta aagataatgg aggtgggggt agtggagggg gagg                     44


<210>  13
<211>  59
<212>  DNA
<213>  Artificial sequence

<220>
<223>  Artificial sequence

<400>  13
acaagccttc ttcagaatgg agttgaccat gtgggaggtg ggggtagtgg agggggagg      59


<210>  14
<211>  56
<212>  DNA
<213>  Artificial sequence

<220>
<223>  Artificial sequence

<400>  14
gaggatcggg tgcatctctg tgaggtcgac gtccttgtcg atgagagcgt ttgtag        56


<210>  15
<211>  59
<212>  DNA
<213>  Artificial sequence

<220>
<223>  Artificial sequence

<400>  15
tggacacttc ttagcagcga aggggacagc atgggaggtg ggggtagtgg agggggagg        59


<210>  16
<211>  56
<212>  DNA
<213>  Artificial sequence

<220>
<223>  Artificial sequence

<400>  16
aagaagcggg tgtgtctcgc tgaggtcgac gtccttgtcg atgagagcgt ttgtag          56


<210>  17
<211>  34
<212>  DNA
<213>  Artificial sequence

<220>
<223>  Artificial sequence

<400>  17
atagtcgaca accacccgat gctcatgaac cttc                                  34


<210>  18
<211>  40
<212>  DNA
<213>  Artificial sequence

<220>
<223>  Artificial sequence

<400>  18
atatgcggcc gcttacacgg cgatcttgcc gcccttcttg                            40


<210>  19
<211>  30
<212>  DNA
<213>  Artificial sequence

<220>
<223>  Artificial sequence

<400>  19
catcatgttg ctctccaatg agtccttcac                                       30


<210>  20
<211>  32
<212>  DNA
<213>  Artificial sequence

<220>
<223>  Artificial sequence

<400>  20
actcattgga gagcaacatg atgacctcaa tg                                    32

<210>  21
<211>  25
<212>  DNA
<213>  Artificial sequence

<220>
<223>  Artificial sequence

<400>  21
tctgtgaccc tagagctgtc ccagc                                          25


<210>  22
<211>  24
<212>  DNA
<213>  Artificial sequence

<220>
<223>  Artificial sequence

<400>  22
ccctccacca tcattcacac gaac                                           24


<210>  23
<211>  34
<212>  DNA
<213>  Artificial sequence

<220>
<223>  Artificial sequence

<400>  23
aattctcgag atggaagatg ccaaaaacat taag                                34


<210>  24
<211>  39
<212>  DNA
<213>  Artificial sequence

<220>
<223>  Artificial sequence

<400>  24
atatgcggcc gctaattatc tttaagaagg ttcatgagc                           39


<210>  25
<211>  38
<212>  DNA
<213>  Artificial sequence

<220>
<223>  Artificial sequence

<400>  25
atctcgagat gcggcccaag ctgtctgagg agcagcag                            38


<210>  26
<211>  40

<212>  DNA
<213>  Artificial sequence

<220>
<223>  Artificial sequence

<400>  26
atatgcggcc gcttacacgg cgatcttgcc gcccttcttg                              40


<210>  27
<211>  49
<212>  DNA
<213>  Artificial sequence

<220>
<223>  Artificial sequence

<400>  27
catgcgcgga agccatcacc atcaccatca cggatccctc gagaggcct                    49


<210>  28
<211>  49
<212>  DNA
<213>  Artificial sequence

<220>
<223>  Artificial sequence

<400>  28
gatcaggcct ctcgagggat ccgtgatggt gatggtgatg gcttccgcg                    49


<210>  29
<211>  26
<212>  DNA
<213>  Artificial sequence

<220>
<223>  Artificial sequence

<400>  29
tcgaggaatt cgcggccgcg tcgacg                                             26


<210>  30
<211>  26
<212>  DNA
<213>  Artificial sequence

<220>
<223>  Artificial sequence

<400>  30
tcgacgtcga cgcggccgcg aattcc                                             26


<210>  31
<211>  56
<212>  DNA
<213>  Artificial sequence

<220>
<223>  Artificial sequence

<400>  31
cacaacgctg caggtgatga cgatgataag ggatccctcg agaggcctga tccggc          56


<210>  32
<211>  62
<212>  DNA
<213>  Artificial sequence

<220>
<223>  Artificial sequence

<400>  32
attatgatta tgattatgat tatgattatg acccatggta tatctccttc ttaaagttaa      60

ac                                                                      62


<210>  33
<211>  65
<212>  DNA
<213>  Artificial sequence

<220>
<223>  Artificial sequence

<400>  33
cacgctcatg cccacaacaa gatcgatgac gatgacaaag gatccctcga gaggcctgat      60

ccggc                                                                   65


<210>  34
<211>  62
<212>  DNA
<213>  Artificial sequence

<220>
<223>  Artificial sequence

<400>  34
attatgatta tgattatgat tatgattatg acccatggta tatctccttc ttaaagttaa      60

ac                                                                      62


<210>  35
<211>  1653
<212>  DNA
<213>  Firefly

<400>  35
atggaagatg ccaaaaacat taagaagggc ccagcgccat tctacccact cgaagacggg      60

accgccggcg agcagctgca caaagccatg aagcgctacg ccctggtgcc cggcaccatc     120

gcctttaccg acgcacatat cgaggtggac attacctacg ccgagtactt cgagatgagc     180

gttcggctgg cagaagctat gaagcgctat gggctgaata caaaccatcg gatcgtggtg     240

tgcagcgaga atagcttgca gttcttcatg cccgtgttgg gtgccctgtt catcggtgtg 300

gctgtggccc cagctaacga catctacaac gagcgcgagc tgctgaacag catgggcatc 360

agccagccca ccgtcgtatt cgtgagcaag aaagggctgc aaaagatcct caacgtgcaa 420

aagaagctac cgatcataca aaagatcatc atcatggata gcaagaccga ctaccagggc 480

ttccaaagca tgtacacctt cgtgacttcc catttgccac ccggcttcaa cgagtacgac 540

ttcgtgcccg agagcttcga ccgggacaaa accatcgccc tgatcatgaa cagtagtggc 600

agtaccggat tgcccaaggg cgtagcccta ccgcaccgca ccgcttgtgt ccgattcagt 660

catgcccgcg accccatctt cggcaaccag atcatccccg acaccgctat cctcagcgtg 720

gtgccatttc accacggctt cggcatgttc accacgctgg gctacttgat ctgcggcttt 780

cgggtcgtgc tcatgtaccg cttcgaggag gagctattct tgcgcagctt gcaagactat 840

aagattcaat ctgccctgct ggtgcccaca ctatttagct tcttcgctaa gagcactctc 900

atcgacaagt acgacctaag caacttgcac gagatcgcca gcggcggggc gccgctcagc 960

aaggaggtag gtgaggccgt ggccaaacgc ttccacctac caggcatccg ccagggctac 1020

ggcctgacag aaacaaccag cgccattctg atcacccccg aaggggacga caagcctggc 1080

gcagtaggca aggtggtgcc cttcttcgag gctaaggtgg tggacttgga caccggtaag 1140

acactgggtg tgaaccagcg cggcgagctg tgcgtccgtg gccccatgat catgagcggc 1200

tacgttaaca accccgaggc tacaaacgct ctcatcgaca aggacggctg gctgcacagc 1260

ggcgacatcg cctactggga cgaggacgag cacttcttca tcgtggaccg gctgaagagc 1320

ctgatcaaat acaagggcta ccaggtagcc ccagccgaac tggagagcat cctgctgcaa 1380

caccccaaca tcttcgacgc cggggtcgcc ggcctgcccg acgacgatgc cggcgagctg 1440

cccgccgcag tcgtcgtgct ggaacacggt aaaaccatga ccgagaagga gatcgtggac 1500

tatgtggcca gccaggttac aaccgccaag aagctgcgcg gtggtgttgt gttcgtggac 1560

gaggtgccta aaggactgac cggcaagttg gacgcccgca agatccgcga gattctcatt 1620

aaggccaaga agggcggcaa gatcgccgtg taa 1653

<210> 36
<211> 550
<212> PRT
<213> Firefly

<400> 36

Met Glu Asp Ala Lys Asn Ile Lys Lys Gly Pro Ala Pro Phe Tyr Pro
1               5                   10                  15

Leu Glu Asp Gly Thr Ala Gly Glu Gln Leu His Lys Ala Met Lys Arg
            20                  25                  30

Tyr Ala Leu Val Pro Gly Thr Ile Ala Phe Thr Asp Ala His Ile Glu
        35                  40                  45

Val Asp Ile Thr Tyr Ala Glu Tyr Phe Glu Met Ser Val Arg Leu Ala
    50                  55                  60

Glu Ala Met Lys Arg Tyr Gly Leu Asn Thr Asn His Arg Ile Val Val
65                  70                  75                  80

Cys Ser Glu Asn Ser Leu Gln Phe Phe Met Pro Val Leu Gly Ala Leu
                85                  90                  95

Phe Ile Gly Val Ala Val Ala Pro Ala Asn Asp Ile Tyr Asn Glu Arg
            100                 105                 110

Glu Leu Leu Asn Ser Met Gly Ile Ser Gln Pro Thr Val Val Phe Val
        115                 120                 125

Ser Lys Lys Gly Leu Gln Lys Ile Leu Asn Val Gln Lys Lys Leu Pro
    130                 135                 140

Ile Ile Gln Lys Ile Ile Ile Met Asp Ser Lys Thr Asp Tyr Gln Gly
145                 150                 155                 160

Phe Gln Ser Met Tyr Thr Phe Val Thr Ser His Leu Pro Pro Gly Phe
                165                 170                 175

Asn Glu Tyr Asp Phe Val Pro Glu Ser Phe Asp Arg Asp Lys Thr Ile
            180                 185                 190

Ala Leu Ile Met Asn Ser Ser Gly Ser Thr Gly Leu Pro Lys Gly Val
        195                 200                 205

Ala Leu Pro His Arg Thr Ala Cys Val Arg Phe Ser His Ala Arg Asp
    210                 215                 220

Pro Ile Phe Gly Asn Gln Ile Ile Pro Asp Thr Ala Ile Leu Ser Val
225                 230                 235                 240

Val Pro Phe His His Gly Phe Gly Met Phe Thr Thr Leu Gly Tyr Leu
                245                 250                 255

Ile Cys Gly Phe Arg Val Val Leu Met Tyr Arg Phe Glu Glu Glu Leu
            260                 265                 270

Phe Leu Arg Ser Leu Gln Asp Tyr Lys Ile Gln Ser Ala Leu Leu Val
        275                 280                 285

Pro Thr Leu Phe Ser Phe Phe Ala Lys Ser Thr Leu Ile Asp Lys Tyr
    290                 295                 300

Asp Leu Ser Asn Leu His Glu Ile Ala Ser Gly Gly Ala Pro Leu Ser
305                 310                 315                 320

Lys Glu Val Gly Glu Ala Val Ala Lys Arg Phe His Leu Pro Gly Ile
                325                 330                 335

Arg Gln Gly Tyr Gly Leu Thr Glu Thr Thr Ser Ala Ile Leu Ile Thr
            340                 345                 350

Pro Glu Gly Asp Asp Lys Pro Gly Ala Val Gly Lys Val Val Pro Phe
        355                 360                 365

Phe Glu Ala Lys Val Val Asp Leu Asp Thr Gly Lys Thr Leu Gly Val
    370                 375                 380

Asn Gln Arg Gly Glu Leu Cys Val Arg Gly Pro Met Ile Met Ser Gly
385                 390                 395                 400

Tyr Val Asn Asn Pro Glu Ala Thr Asn Ala Leu Ile Asp Lys Asp Gly
                405                 410                 415

Trp Leu His Ser Gly Asp Ile Ala Tyr Trp Asp Glu Asp Glu His Phe
            420                 425                 430

Phe Ile Val Asp Arg Leu Lys Ser Leu Ile Lys Tyr Lys Gly Tyr Gln
        435                 440                 445

Val Ala Pro Ala Glu Leu Glu Ser Ile Leu Leu Gln His Pro Asn Ile
    450                 455                 460

Phe Asp Ala Gly Val Ala Gly Leu Pro Asp Asp Asp Ala Gly Glu Leu
465                 470                 475                 480

Pro Ala Ala Val Val Val Leu Glu His Gly Lys Thr Met Thr Glu Lys
                485                 490                 495

Glu Ile Val Asp Tyr Val Ala Ser Gln Val Thr Thr Ala Lys Lys Leu
            500                 505                 510

Arg Gly Gly Val Val Phe Val Asp Glu Val Pro Lys Gly Leu Thr Gly
        515                 520                 525

Lys Leu Asp Ala Arg Lys Ile Arg Glu Ile Leu Ile Lys Ala Lys Lys

530                 535                 540

Gly Gly Lys Ile Ala Val
545                 550

<210>  37
<211>  936
<212>  DNA
<213>  Renilla

<400>  37
atgacttcga aagtttatga tccagaacaa aggaaacgga tgataactgg tccgcagtgg      60

tgggccagat gtaaacaaat gaatgttctt gattcattta ttaattatta tgattcagaa     120

aaacatgcag aaaatgctgt tattttttta catggtaacg cggcctcttc ttatttatgg     180

cgacatgttg tgccacatat tgagccagta gcgcggtgta ttataccaga ccttattggt     240

atgggcaaat caggcaaatc tggtaatggt tcttataggt tacttgatca ttacaaatat     300

cttactgcat ggtttgaact tcttaattta ccaaagaaga tcatttttgt cggccatgat     360

tggggtgctt gtttggcatt tcattatagc tatgagcatc aagataagat caaagcaata     420

gttcacgctg aaagtgtagt agatgtgatt gaatcatggg atgaatggcc tgatattgaa     480

gaagatattg cgttgatcaa atctgaagaa ggagaaaaaa tggttttgga gaataacttc     540

ttcgtggaaa ccatgttgcc atcaaaaatc atgagaaagt tagaaccaga agaatttgca     600

gcatatcttg aaccattcaa agagaaaggt gaagttcgtc gtccaacatt atcatggcct     660

cgtgaaatcc cgttagtaaa aggtggtaaa cctgacgttg tacaaattgt taggaattat     720

aatgcttatc tacgtgcaag tgatgattta ccaaaaatgt ttattgaatc ggacccagga     780

ttcttttcca atgctattgt tgaaggtgcc aagaagtttc ctaatactga atttgtcaaa     840

gtaaaaggtc ttcatttttc gcaagaagat gcacctgatg aaatgggaaa atatatcaaa     900

tcgttcgttg agcgagttct caaaaatgaa caataa                               936

<210>  38
<211>  311
<212>  PRT
<213>  Renilla

<400>  38

Met Thr Ser Lys Val Tyr Asp Pro Glu Gln Arg Lys Arg Met Ile Thr
1               5                   10                  15

Gly Pro Gln Trp Trp Ala Arg Cys Lys Gln Met Asn Val Leu Asp Ser
            20                  25                  30

Phe Ile Asn Tyr Tyr Asp Ser Glu Lys His Ala Glu Asn Ala Val Ile
        35                  40                  45

Phe Leu His Gly Asn Ala Ala Ser Ser Tyr Leu Trp Arg His Val Val
    50                  55                  60

Pro His Ile Glu Pro Val Ala Arg Cys Ile Ile Pro Asp Leu Ile Gly
65                  70                  75                  80

Met Gly Lys Ser Gly Lys Ser Gly Asn Gly Ser Tyr Arg Leu Leu Asp
                85                  90                  95

His Tyr Lys Tyr Leu Thr Ala Trp Phe Glu Leu Leu Asn Leu Pro Lys
            100                 105                 110

Lys Ile Ile Phe Val Gly His Asp Trp Gly Ala Cys Leu Ala Phe His
        115                 120                 125

Tyr Ser Tyr Glu His Gln Asp Lys Ile Lys Ala Ile Val His Ala Glu
    130                 135                 140

Ser Val Val Asp Val Ile Glu Ser Trp Asp Glu Trp Pro Asp Ile Glu
145                 150                 155                 160

Glu Asp Ile Ala Leu Ile Lys Ser Glu Glu Gly Glu Lys Met Val Leu
                165                 170                 175

Glu Asn Asn Phe Phe Val Glu Thr Met Leu Pro Ser Lys Ile Met Arg
            180                 185                 190

Lys Leu Glu Pro Glu Glu Phe Ala Ala Tyr Leu Glu Pro Phe Lys Glu
        195                 200                 205

Lys Gly Glu Val Arg Arg Pro Thr Leu Ser Trp Pro Arg Glu Ile Pro
    210                 215                 220

Leu Val Lys Gly Gly Lys Pro Asp Val Val Gln Ile Val Arg Asn Tyr
225                 230                 235                 240

Asn Ala Tyr Leu Arg Ala Ser Asp Asp Leu Pro Lys Met Phe Ile Glu
                245                 250                 255

Ser Asp Pro Gly Phe Phe Ser Asn Ala Ile Val Glu Gly Ala Lys Lys
            260                 265                 270

Phe Pro Asn Thr Glu Phe Val Lys Val Lys Gly Leu His Phe Ser Gln
        275                 280                 285

Glu Asp Ala Pro Asp Glu Met Gly Lys Tyr Ile Lys Ser Phe Val Glu

290                 295                 300

Arg Val Leu Lys Asn Glu Gln
305                 310

<210>  39
<211>  1629
<212>  DNA
<213>  Pyrophorus plagiophthalamus

<400>  39
atggagagag agaagaacgt ggtgtacggc cccgagccca agcaccctct gggcaacttc      60

accgccggcg agatgctgta caacgctctg cacaagcact cccacatccc ccaggccatc     120

ctggacgtga tgggcaacga gtccctttcc taccaggagt tcttcgacac tactgtgaag     180

ctgggccaga gcctccagaa ctgtggctac aagatgaacg atgtcgtgtc gatctgtgca     240

gagaacaaca agagattctt catccccatc atctccgcct ggtacatcgg catggtggtg     300

gcccctgtga acgaggacta tatcccagac gagctgtgta aagtgaccgg catctccaag     360

ccgatcctgg tcttcaccac taggaagatc ctgcctaagg ttttggaggt taaagacaga     420

accaactaca taaagaggat catcatactg gactctgaag agaacctgct gggctgcgag     480

agcctgcaca acttcatgtc caggtactcc gacaacaacc tccaaacatt caagcctctg     540

cactacgacc ctgtggacca ggtagccgcc atcctgtgct cctccggcac aaccggcctg     600

cctaaaggcg tgatgcagac ccacaggaac atctgtgtga gactcacaca cgcatctgac     660

cccagagtgg gtacacaact catccccggc gtatccgtgc tggcctacct gccattcttc     720

cacgccttcg gcttcagtat caacctgggc tatttcatgg tgggcctgag agtggtgatg     780

ctccgaaggt ttaaccagga ggtgttcctg aaggccatcc aggactacga ggtgaggagc     840

gtgatcaacg ttccctccac aatcctgttc ctgtccaaga gccctctggt ggacaagtac     900

gacctatcca ccctggcgga gctgtgctgt ggagccgctc ctctggcgaa ggaggtggcc     960

gagatcgccg tgaagaggct gaacctgcca gggatacggt gtggctacgg tctaacagag    1020

tctacctccg ccaacatcca tactctgcac aacgagttca agtccggctc cctgggcaag    1080

gtgacacctt acatggccgc caagatcatc gacaggaaca ccggcgaggc cctgggtcca    1140

aaccaggtgg gcgagctgtg catctgggga cctatggtaa caaaaggcta tgtgaacaac    1200

ccacaggcta ctaaggaggc catcgacgac gacggctggc tgcactctgg cgacttcggc    1260

tactacgacg aggacgagta tttctacatc gtggaccggt acaaggagct gatcaaatac    1320

aagggctatc aggtcgcccc tgtggagctg gaggagatcc tccttcagca cccaggcatc    1380

agggacgtgg ccgtcgtggg tatccctgac atcgaggccg gcgagctgcc agccggcttc    1440

gtggtgaagc agcccggcgc ccaactcacc gctaaggagg tgtacgactt cctggcccag    1500

agggtgtctc actccaagta cctgaggggc ggcgtaaggt tcgtggactc tatccccagg 1560

aacgtgacag gcaagattag tcgaaaagag ctgagggagg ccctgatgga gaaggcttct 1620

aagctgtaa 1629

<210> 40
<211> 542
<212> PRT
<213> Pyrophorus plagiophthalamus

<400> 40

Met Glu Arg Glu Lys Asn Val Val Tyr Gly Pro Glu Pro Lys His Pro
1               5                   10                  15

Leu Gly Asn Phe Thr Ala Gly Glu Met Leu Tyr Asn Ala Leu His Lys
            20                  25                  30

His Ser His Ile Pro Gln Ala Ile Leu Asp Val Met Gly Asn Glu Ser
        35                  40                  45

Leu Ser Tyr Gln Glu Phe Phe Asp Thr Thr Val Lys Leu Gly Gln Ser
    50                  55                  60

Leu Gln Asn Cys Gly Tyr Lys Met Asn Asp Val Val Ser Ile Cys Ala
65                  70                  75                  80

Glu Asn Asn Lys Arg Phe Phe Ile Pro Ile Ile Ser Ala Trp Tyr Ile
                85                  90                  95

Gly Met Val Val Ala Pro Val Asn Glu Asp Tyr Ile Pro Asp Glu Leu
            100                 105                 110

Cys Lys Val Thr Gly Ile Ser Lys Pro Ile Leu Val Phe Thr Thr Arg
        115                 120                 125

Lys Ile Leu Pro Lys Val Leu Glu Val Lys Asp Arg Thr Asn Tyr Ile
    130                 135                 140

Lys Arg Ile Ile Ile Leu Asp Ser Glu Glu Asn Leu Leu Gly Cys Glu
145                 150                 155                 160

Ser Leu His Asn Phe Met Ser Arg Tyr Ser Asp Asn Asn Leu Gln Thr
                165                 170                 175

Phe Lys Pro Leu His Tyr Asp Pro Val Asp Gln Val Ala Ala Ile Leu
            180                 185                 190

Cys Ser Ser Gly Thr Thr Gly Leu Pro Lys Gly Val Met Gln Thr His

195                 200                 205

Arg Asn Ile Cys Val Arg Leu Thr His Ala Ser Asp Pro Arg Val Gly
    210                 215                 220

Thr Gln Leu Ile Pro Gly Val Ser Val Leu Ala Tyr Leu Pro Phe Phe
225                 230                 235                 240

His Ala Phe Gly Phe Ser Ile Asn Leu Gly Tyr Phe Met Val Gly Leu
                245                 250                 255

Arg Val Val Met Leu Arg Arg Phe Asn Gln Glu Val Phe Leu Lys Ala
            260                 265                 270

Ile Gln Asp Tyr Glu Val Arg Ser Val Ile Asn Val Pro Ser Thr Ile
        275                 280                 285

Leu Phe Leu Ser Lys Ser Pro Leu Val Asp Lys Tyr Asp Leu Ser Thr
    290                 295                 300

Leu Ala Glu Leu Cys Cys Gly Ala Ala Pro Leu Ala Lys Glu Val Ala
305                 310                 315                 320

Glu Ile Ala Val Lys Arg Leu Asn Leu Pro Gly Ile Arg Cys Gly Tyr
                325                 330                 335

Gly Leu Thr Glu Ser Thr Ser Ala Asn Ile His Thr Leu His Asn Glu
            340                 345                 350

Phe Lys Ser Gly Ser Leu Gly Lys Val Thr Pro Tyr Met Ala Ala Lys
        355                 360                 365

Ile Ile Asp Arg Asn Thr Gly Glu Ala Leu Gly Pro Asn Gln Val Gly
    370                 375                 380

Glu Leu Cys Ile Trp Gly Pro Met Val Thr Lys Gly Tyr Val Asn Asn
385                 390                 395                 400

Pro Gln Ala Thr Lys Glu Ala Ile Asp Asp Asp Gly Trp Leu His Ser
                405                 410                 415

Gly Asp Phe Gly Tyr Tyr Asp Glu Asp Glu Tyr Phe Tyr Ile Val Asp
            420                 425                 430

Arg Tyr Lys Glu Leu Ile Lys Tyr Lys Gly Tyr Gln Val Ala Pro Val
        435                 440                 445

Glu Leu Glu Glu Ile Leu Leu Gln His Pro Gly Ile Arg Asp Val Ala
    450                 455                 460

Val Val Gly Ile Pro Asp Ile Glu Ala Gly Glu Leu Pro Ala Gly Phe
465                 470                 475                 480

Val Val Lys Gln Pro Gly Ala Gln Leu Thr Ala Lys Glu Val Tyr Asp
                485                 490                 495

Phe Leu Ala Gln Arg Val Ser His Ser Lys Tyr Leu Arg Gly Gly Val
            500                 505                 510

Arg Phe Val Asp Ser Ile Pro Arg Asn Val Thr Gly Lys Ile Ser Arg
        515                 520                 525

Lys Glu Leu Arg Glu Ala Leu Met Glu Lys Ala Ser Lys Leu
    530                 535                 540

<210>  41
<211>  1284
<212>  DNA
<213>  Homo Sapiens

<400>  41
atggaggcaa tggcggccag cacttccctg cctgaccctg gagactttga ccggaacgtg      60

ccccggatct gtggggtgtg tggagaccga gccactggct ttcacttcaa tgctatgacc     120

tgtgaaggct gcaaaggctt cttcaggcga agcatgaagc ggaaggcact attcacctgc     180

cccttcaacg gggactgccg catcaccaag gacaaccgac gccactgcca ggcctgccgg     240

ctcaaacgct gtgtggacat cggcatgatg aaggagttca ttctgacaga tgaggaagtg     300

cagaggaagc gggagatgat cctgaagcgg aaggaggagg aggccttgaa ggacagtctg     360

cggcccaagc tgtctgagga gcagcagcgc atcattgcca tactgctgga cgcccaccat     420

aagacctacg accccaccta ctccgacttc tgccagttcc ggcctccagt tcgtgtgaat     480

gatggtggag ggagccatcc ttccaggccc aactccagac acactcccag cttctctggg     540

gactcctcct cctcctgctc agatcactgt atcacctctt cagacatgat ggactcgtcc     600

agcttctcca atctggatct gagtgaagaa gattcagatg acccttctgt gaccctagag     660

ctgtcccagc tctccatgct gccccacctg gctgacctgg tcagttacag catccaaaag     720

gtcattggct ttgctaagat gataccagga ttcagagacc tcacctctga ggaccagatc     780

gtactgctga agtcaagtgc cattgaggtc atcatgttgc gctccaatga gtccttcacc     840

atggacgaca tgtcctggac ctgtggcaac caagactaca agtaccgcgt cagtgacgtg     900

accaaagccg gacacagcct ggagctgatt gagcccctca tcaagttcca ggtgggactg     960

aagaagctga acttgcatga ggaggagcat gtcctgctca tggccatctg catcgtctcc    1020

ccagatcgtc ctggggtgca ggacgccgcg ctgattgagg ccatccagga ccgcctgtcc 1080

aacacactgc agacgtacat ccgctgccgc cacccgcccc cgggcagcca cctgctctat 1140

gccaagatga tccagaagct agccgacctg cgcagcctca atgaggagca ctccaagcag 1200

taccgctgcc tctccttcca gcctgagtgc agcatgaagc taacgcccct tgtgctcgaa 1260

gtgtttggca atgagatctc ctga 1284

<210> 42
<211> 427
<212> PRT
<213> Homo Sapiens

<400> 42

<210>  43
<211>  1269
<212>  DNA
<213>  Mouse

<400>  43
atggaggcaa tggcagccag cacctccctg cctgaccctg gtgactttga ccggaatgtg      60

cctcggatct gtggagtgtg tggagaccga gccacgggct tccacttcaa cgctatgacc     120

tgtgaaggct gcaagggttt cttcaggcgg agcatgaagc gcaaggccct gttcacctgc     180

cccttcaatg gagattgccg catcaccaag gacaaccggc gacactgcca ggcctgccgg     240

ctcaaacgct gcgtggacat tggcatgatg aaggagttca tcctcacaga tgaggaggtg     300

cagcgtaagc gagagatgat catgaagagg aaggaggaag aggccttgaa ggacagtctg     360

aggcccaagc tgtctgagga gcaacagcac attatcgcca tcctgctcga tgcccaccac     420

aagacctacg accccaccta tgccgacttc cgggacttcc ggcctccaat tcgtgcagac     480

gtaagtacag ggagctattc tccaaggccc acactcagct tctccggaga ctcctcctca     540

aactctgatc tgtacacccc ctcactggac atgatggaac cggccagctt ttccacgatg     600

gatctgaatg aagaaggctc cgatgacccc tctgtgaccc tggacctgtc tccgctctcc     660

atgctgcccc acctggctga tcttgtcagt tacagcatcc aaaaggtcat cggctttgcc     720

aagatgatcc ctggcttcag ggacctcacc tctgatgacc agattgtcct gcttaagtca     780

agtgccattg aggtgatcat gttgcgctcc aaccagtctt ttaccttgga tgacatgtcc     840

tgggactgtg gcagccaaga ctacaaatat gacatcactg atgtctccag agctgggcac     900

accctggagc tgatcgaacc cctcataaag ttccaggtgg ggctgaagaa gctgaacctc     960

catgaggaag aacatgtgct gctcatggcc atctgcattg tctccccaga ccgacctggg    1020

gtacaggatg ctaagctggt tgaagccatt caggaccgcc tatccaacac actgcagacc    1080

tacatccgct gccgccaccc gcccccgggc agccaccagc tctacgccaa gatgatccag    1140

aagctggctg acctgcgaag cctcaatgag gagcactcca aacagtaccg ttccctctcc    1200

ttccagccgg agaacagcat gaagctcaca ccccttgtgc tagaggtgtt cggcaatgag    1260

atctcctga                                                            1269


<210>  44
<211>  422
<212>  PRT
<213>  Mouse

<400>  44

Met Glu Ala Met Ala Ala Ser Thr Ser Leu Pro Asp Pro Gly Asp Phe
1               5                   10                  15

Asp Arg Asn Val Pro Arg Ile Cys Gly Val Cys Gly Asp Arg Ala Thr
            20                  25                  30

Gly Phe His Phe Asn Ala Met Thr Cys Glu Gly Cys Lys Gly Phe Phe
        35                  40                  45

Arg Arg Ser Met Lys Arg Lys Ala Leu Phe Thr Cys Pro Phe Asn Gly
    50                  55                  60

Asp Cys Arg Ile Thr Lys Asp Asn Arg Arg His Cys Gln Ala Cys Arg
65                  70                  75                  80

Leu Lys Arg Cys Val Asp Ile Gly Met Met Lys Glu Phe Ile Leu Thr
                85                  90                  95

Asp Glu Glu Val Gln Arg Lys Arg Glu Met Ile Met Lys Arg Lys Glu
            100                 105                 110

Glu Glu Ala Leu Lys Asp Ser Leu Arg Pro Lys Leu Ser Glu Glu Gln
        115                 120                 125

Gln His Ile Ile Ala Ile Leu Leu Asp Ala His His Lys Thr Tyr Asp
    130                 135                 140

Pro Thr Tyr Ala Asp Phe Arg Asp Phe Arg Pro Pro Ile Arg Ala Asp
145                 150                 155                 160

Val Ser Thr Gly Ser Tyr Ser Pro Arg Pro Thr Leu Ser Phe Ser Gly
                165                 170                 175

Asp Ser Ser Ser Asn Ser Asp Leu Tyr Thr Pro Ser Leu Asp Met Met
            180                 185                 190

Glu Pro Ala Ser Phe Ser Thr Met Asp Leu Asn Glu Glu Gly Ser Asp
        195                 200                 205

Asp Pro Ser Val Thr Leu Asp Leu Ser Pro Leu Ser Met Leu Pro His
    210                 215                 220

Leu Ala Asp Leu Val Ser Tyr Ser Ile Gln Lys Val Ile Gly Phe Ala
225                 230                 235                 240

Lys Met Ile Pro Gly Phe Arg Asp Leu Thr Ser Asp Asp Gln Ile Val
                245                 250                 255

Leu Leu Lys Ser Ser Ala Ile Glu Val Ile Met Leu Arg Ser Asn Gln
            260                 265                 270

Ser Phe Thr Leu Asp Asp Met Ser Trp Asp Cys Gly Ser Gln Asp Tyr
        275                 280                 285

Lys Tyr Asp Ile Thr Asp Val Ser Arg Ala Gly His Thr Leu Glu Leu
    290                 295                 300

Ile Glu Pro Leu Ile Lys Phe Gln Val Gly Leu Lys Lys Leu Asn Leu
305                 310                 315                 320

His Glu Glu Glu His Val Leu Leu Met Ala Ile Cys Ile Val Ser Pro
                325                 330                 335

Asp Arg Pro Gly Val Gln Asp Ala Lys Leu Val Glu Ala Ile Gln Asp
            340                 345                 350

Arg Leu Ser Asn Thr Leu Gln Thr Tyr Ile Arg Cys Arg His Pro Pro
        355                 360                 365

Pro Gly Ser His Gln Leu Tyr Ala Lys Met Ile Gln Lys Leu Ala Asp
    370                 375                 380

Leu Arg Ser Leu Asn Glu Glu His Ser Lys Gln Tyr Arg Ser Leu Ser
385                 390                 395                 400

Phe Gln Pro Glu Asn Ser Met Lys Leu Thr Pro Leu Val Leu Glu Val
                405                 410                 415

Phe Gly Asn Glu Ile Ser
            420

<210>  45
<211>  1272
<212>  DNA
<213>  Rabbit

<400>  45
atggaggcaa cagcggccag cacctccctg cccgaccctg gtgactttga ccggaacgtg      60

ccccggatct gtggagtgtg tggagaccga gccacaggct tccacttcaa tgctatgacc     120

tgtgaaggct gcaaaggttt cttcaggcgg agcatgaagc ggaaggccct gttcacctgt     180

cccttcaatg gagattgccg catcaccaag gacaaccggc gacactgcca ggcctgccgg     240

ctcaaacgct gtgtggacat cggcatgatg aaggagttca tcctgacaga tgaggaggta     300

cagcgtaaga gggagatgat aatgaagaga aaagaggaag aggccttgaa ggacagtctg     360

aggcccaagc tatctgaaga acaacagcac atcatagcca tcctgctgga cgcccaccac     420

aagacctatg accccaccta cgctgacttc agggacttcc ggcctccagt tcgtatggac     480

ggaagtacag ggagctattc tccaaggccc acactcagct tctccgggaa ctcctcctcc 540

tccagctctg acctgtacac cacctcacta gacatgatgg aaccatccgg cttttccaac 600

ctggatctga acggagagga ttctgatgac ccgtctgtga ctctggacct gtctcctctc 660

tccatgctgc cccacctggc tgaccttgtc agttacagca tccaaaaggt catcggcttt 720

gccaagatga tcccaggatt cagggatctc acctccgatg accagattgt cctgcttaag 780

tcaagcgcca ttgaggtgat catgttacgc tccaaccagt ctttcaccat ggatgatatg 840

tcctgggact gtggcagcca ggactacaag tacgacgtca ccgatgtctc caaagctggg 900

cacaccctgg agctgatcga gcccctcata aagttccagg tggggctgaa gaagctgaac 960

ttacatgagg aagagcatgt ccttctcatg gccatctgca ttgtctcccc ggaccgacct 1020

ggggtccagg acgccaagct ggtggaagcc attcaggacc gcctatccaa cacgctgcag 1080

acctacatcc gctgccgcca cccgccccca ggcagccacc agctctatgc caagatgatc 1140

cagaaactgg ccgacctgcg gagcctcaac gaggaacact ccaaacaata ccgctccctc 1200

tccttccagc ccgagaatag catgaagctc acaccccttg tgctggaggt gttcggcaat 1260

gagatctcct ga 1272

<210> 46
<211> 423
<212> PRT
<213> Rabbit

<400> 46

Met Glu Ala Thr Ala Ala Ser Thr Ser Leu Pro Asp Pro Gly Asp Phe
1               5                   10                  15

Asp Arg Asn Val Pro Arg Ile Cys Gly Val Cys Gly Asp Arg Ala Thr
            20                  25                  30

Gly Phe His Phe Asn Ala Met Thr Cys Glu Gly Cys Lys Gly Phe Phe
        35                  40                  45

Arg Arg Ser Met Lys Arg Lys Ala Leu Phe Thr Cys Pro Phe Asn Gly
    50                  55                  60

Asp Cys Arg Ile Thr Lys Asp Asn Arg Arg His Cys Gln Ala Cys Arg
65                  70                  75                  80

Leu Lys Arg Cys Val Asp Ile Gly Met Met Lys Glu Phe Ile Leu Thr
                85                  90                  95

Asp Glu Glu Val Gln Arg Lys Arg Glu Met Ile Met Lys Arg Lys Glu
            100                 105                 110

Glu Glu Ala Leu Lys Asp Ser Leu Arg Pro Lys Leu Ser Glu Glu Gln
        115                 120                 125

Gln His Ile Ile Ala Ile Leu Leu Asp Ala His His Lys Thr Tyr Asp
    130                 135                 140

Pro Thr Tyr Ala Asp Phe Arg Asp Phe Arg Pro Pro Val Arg Met Asp
145                 150                 155                 160

Gly Ser Thr Gly Ser Tyr Ser Pro Arg Pro Thr Leu Ser Phe Ser Gly
                165                 170                 175

Asn Ser Ser Ser Ser Ser Ser Asp Leu Tyr Thr Thr Ser Leu Asp Met
            180                 185                 190

Met Glu Pro Ser Gly Phe Ser Asn Leu Asp Leu Asn Gly Glu Asp Ser
        195                 200                 205

Asp Asp Pro Ser Val Thr Leu Asp Leu Ser Pro Leu Ser Met Leu Pro
    210                 215                 220

His Leu Ala Asp Leu Val Ser Tyr Ser Ile Gln Lys Val Ile Gly Phe
225                 230                 235                 240

Ala Lys Met Ile Pro Gly Phe Arg Asp Leu Thr Ser Asp Asp Gln Ile
                245                 250                 255

Val Leu Leu Lys Ser Ser Ala Ile Glu Val Ile Met Leu Arg Ser Asn
            260                 265                 270

Gln Ser Phe Thr Met Asp Asp Met Ser Trp Asp Cys Gly Ser Gln Asp
        275                 280                 285

Tyr Lys Tyr Asp Val Thr Asp Val Ser Lys Ala Gly His Thr Leu Glu
    290                 295                 300

Leu Ile Glu Pro Leu Ile Lys Phe Gln Val Gly Leu Lys Lys Leu Asn
305                 310                 315                 320

Leu His Glu Glu Glu His Val Leu Leu Met Ala Ile Cys Ile Val Ser
                325                 330                 335

Pro Asp Arg Pro Gly Val Gln Asp Ala Lys Leu Val Glu Ala Ile Gln
            340                 345                 350

Asp Arg Leu Ser Asn Thr Leu Gln Thr Tyr Ile Arg Cys Arg His Pro
        355                 360                 365

Pro Pro Gly Ser His Gln Leu Tyr Ala Lys Met Ile Gln Lys Leu Ala
    370                 375                 380

Asp Leu Arg Ser Leu Asn Glu Glu His Ser Lys Gln Tyr Arg Ser Leu
385                 390                 395                 400

Ser Phe Gln Pro Glu Asn Ser Met Lys Leu Thr Pro Leu Val Leu Glu
                405                 410                 415

Val Phe Gly Asn Glu Ile Ser
            420

<210>  47
<211>  434
<212>  PRT
<213>  Monkey

<400>  47

Met Pro Cys Ala Pro Ser Gly Met Glu Ala Met Ala Ala Ser Thr Ser
1               5                   10                  15

Leu Pro Asp Pro Gly Asp Phe Asp Arg Asn Val Pro Arg Ile Cys Gly
            20                  25                  30

Val Cys Gly Asp Arg Ala Thr Gly Phe His Phe Asn Ala Met Thr Cys
        35                  40                  45

Glu Gly Cys Lys Gly Phe Phe Arg Arg Ser Met Lys Arg Lys Ala Leu
    50                  55                  60

Phe Thr Cys Pro Phe Asn Gly Asp Cys Arg Ile Thr Lys Asp Asn Arg
65                  70                  75                  80

Arg His Cys Gln Ala Cys Arg Leu Lys Arg Cys Val Asp Ile Gly Met
                85                  90                  95

Met Lys Glu Phe Ile Leu Thr Asp Glu Glu Val Gln Arg Lys Arg Glu
            100                 105                 110

Met Ile Leu Lys Arg Lys Glu Glu Glu Ala Leu Lys Asp Ser Leu Arg
        115                 120                 125

Pro Lys Leu Ser Glu Glu Gln Gln Arg Ile Ile Ala Ile Leu Leu Asp
    130                 135                 140

Ala His His Lys Thr Tyr Asp Pro Thr Tyr Ser Asp Phe Cys Gln Phe
145                 150                 155                 160

Arg Pro Pro Val Arg Val Asn Asp Gly Gly Gly Ser His Pro Ser Arg
                165                 170                 175

Pro Asn Ser Ser His Thr Pro Ser Phe Ser Gly Asp Ser Ser Ser Cys
            180                 185                 190

Cys Ser Asp His Tyr Ile Ala Ser Pro Asp Met Met Asp Ser Ser Ser
        195                 200                 205

Phe Ser Asn Leu Asp Leu Ser Glu Glu Asp Ser Asp Asp Pro Ser Leu
    210                 215                 220

Thr Leu Glu Leu Ser Gln Leu Ser Met Leu Pro His Leu Ala Asp Leu
225                 230                 235                 240

Val Ser Tyr Ser Ile Gln Lys Val Ile Gly Phe Ala Lys Met Ile Pro
                245                 250                 255

Gly Phe Arg Asp Leu Thr Ser Glu Asp Gln Ile Val Leu Leu Lys Ser
            260                 265                 270

Ser Ala Ile Glu Val Ile Met Leu Arg Ser Asn Glu Ser Phe Thr Met
        275                 280                 285

Asp Asp Met Ser Trp Thr Cys Gly Asn Gln Asp Tyr Lys Tyr Arg Val
    290                 295                 300

Ser Asp Val Thr Lys Ala Gly His Asn Leu Glu Leu Ile Glu Pro Leu
305                 310                 315                 320

Ile Lys Phe Gln Val Gly Leu Lys Lys Leu Asn Leu His Glu Glu Glu
                325                 330                 335

His Val Leu Leu Met Ala Ile Cys Ile Val Ser Pro Asp Arg Pro Gly
            340                 345                 350

Val Gln Asp Ala Ala Leu Ile Glu Ala Ile Gln Asp Arg Leu Ser Asn
        355                 360                 365

Thr Leu Gln Thr Tyr Ile Arg Cys Arg His Pro Pro Pro Gly Ser His
    370                 375                 380

Leu Leu Tyr Ala Lys Met Ile Gln Lys Leu Ala Asp Leu Arg Ser Leu
385                 390                 395                 400

Asn Glu Glu His Ser Lys Gln Tyr Arg Cys Leu Ser Phe Gln Pro Glu

405                 410                 415

Cys Ser Met Lys Leu Thr Pro Leu Val Leu Glu Val Phe Gly Asn Glu
            420                 425                 430

Ile Ser

<210>  48
<211>  427
<212>  PRT
<213>  Dog

<400>  48

Met Glu Ala Thr Ala Ala Ser Thr Ser Leu Pro Asp Pro Gly Asp Phe
1               5                   10                  15

Asp Arg Asn Val Pro Arg Ile Cys Gly Val Cys Gly Asp Arg Ala Thr
            20                  25                  30

Gly Phe His Phe Asn Ala Met Thr Cys Glu Gly Cys Lys Gly Phe Phe
        35                  40                  45

Arg Arg Ser Met Lys Arg Lys Ala Leu Phe Thr Cys Pro Phe Asn Gly
    50                  55                  60

Asp Cys Arg Ile Thr Lys Asp Asn Arg Arg His Cys Gln Ala Cys Arg
65                  70                  75                  80

Leu Lys Arg Cys Val Asp Ile Gly Met Met Lys Glu Phe Ile Leu Thr
                85                  90                  95

Asp Glu Glu Val Gln Arg Lys Arg Glu Met Ile Leu Lys Arg Lys Glu
            100                 105                 110

Glu Glu Ala Leu Arg Asp Ser Leu Arg Pro Lys Leu Ser Glu Glu Gln
        115                 120                 125

Gln Arg Ile Ile Ala Ile Leu Leu Asp Ala His His Lys Thr Tyr Asp
    130                 135                 140

Pro Thr Tyr Ala Asp Phe Thr Gln Phe Arg Pro Pro Val Arg Gly Asp
145                 150                 155                 160

Gly Gly Gly Gly Ser His Ser Ser Arg Pro Ser Ser Ala His Thr Pro
                165                 170                 175

Ser Val Ser Gly Asp Ser Ser Ser Ser Cys Ser Asp His Tyr Pro Ala

            180                 185                 190

Leu Leu Asp Val Met Glu Pro Thr Ser Phe Ser Asn Leu Asp Leu Arg
        195                 200                 205

Glu Glu Asp Ser Asp Asp Ser Ser Leu Thr Leu Asp Leu Ser Gln Leu
    210                 215                 220

Ser Met Leu Pro His Leu Ala Asp Leu Val Ser Tyr Ser Ile Gln Lys
225                 230                 235                 240

Val Ile Gly Phe Ala Lys Met Ile Pro Gly Phe Arg Asp Leu Thr Ser
                245                 250                 255

Glu Asp Gln Ile Val Leu Leu Lys Ser Ser Ala Ile Glu Val Ile Met
            260                 265                 270

Leu Arg Ser Asn Gln Ser Phe Ile Met Asp Asp Met Ser Trp Thr Cys
        275                 280                 285

Gly Ser Gln Asp Tyr Lys Tyr Arg Val Ser Asp Val Ala Lys Ala Gly
    290                 295                 300

His Ser Leu Glu Leu Ile Glu Pro Leu Ile Lys Phe Gln Val Gly Leu
305                 310                 315                 320

Lys Lys Leu Asn Leu His Glu Glu Glu His Val Leu Leu Met Ala Ile
                325                 330                 335

Cys Ile Leu Ser Pro Asp Arg Pro Gly Val Gln Asp Ala Ala Leu Val
            340                 345                 350

Glu Ala Leu Gln Asp Arg Leu Ser Asn Thr Leu Gln Thr Tyr Ile Arg
        355                 360                 365

Cys Arg His Pro Pro Pro Gly Ser His Leu Leu Tyr Ala Lys Met Ile
    370                 375                 380

Gln Lys Leu Ala Asp Leu Arg Ser Leu Asn Glu Glu His Ser Lys Gln
385                 390                 395                 400

Tyr Arg Cys Leu Ser Phe Gln Pro Glu Cys Ser Met Lys Leu Thr Pro
                405                 410                 415

Leu Val Leu Glu Val Phe Gly Asn Glu Ile Ser
            420                 425

<210>  49
<211>  426
<212>  PRT
<213>  Cattle

<400>  49

Met Glu Ala Thr Ala Ala Ser Thr Ser Leu Pro Asp Pro Gly Asp Phe
1               5                   10                  15

Asp Arg Asn Val Pro Arg Ile Cys Gly Val Cys Gly Asp Arg Ala Thr
            20                  25                  30

Gly Phe His Phe Asn Ala Met Thr Cys Glu Gly Cys Lys Gly Phe Phe
        35                  40                  45

Arg Arg Ser Met Lys Arg Lys Ala Leu Phe Thr Cys Pro Phe Asn Gly
    50                  55                  60

Asp Cys Arg Ile Thr Lys Asp Asn Arg Arg His Cys Gln Ala Cys Arg
65                  70                  75                  80

Leu Lys Arg Cys Ile Asp Ile Gly Met Met Lys Glu Phe Ile Leu Thr
                85                  90                  95

Asp Glu Glu Val Gln Arg Lys Arg Glu Met Ile Leu Lys Arg Lys Glu
            100                 105                 110

Glu Glu Ala Leu Lys Asp Ser Leu Arg Pro Lys Leu Ser Glu Glu Gln
        115                 120                 125

Gln Arg Ile Ile Thr Thr Leu Leu Glu Ala His His Lys Thr Tyr Asp
    130                 135                 140

Asp Thr Tyr Ser Asp Phe Ser Gln Phe Arg Pro Pro Val Arg Asn Ser
145                 150                 155                 160

Glu Asp Glu Gly Asn Arg Pro Leu Arg Ser Ile Leu Thr Pro Ser Phe
                165                 170                 175

Ser Gly Asn Ser Ser Ser Ser Cys Ser Asp His Cys Thr Ser Ser Pro
            180                 185                 190

Asp Thr Met Glu Pro Thr Ser Phe Ser Asn Gln Asp Leu Asn Glu Glu
        195                 200                 205

Asp Ser Asp Asp Pro Ser Val Thr Leu Asp Leu Ser Gln Leu Ser Met
    210                 215                 220

Leu Pro His Leu Ala Asp Leu Val Ser Tyr Ser Ile Gln Lys Val Ile
225                 230                 235                 240

Gly Phe Ala Lys Met Ile Pro Gly Phe Arg Asp Leu Thr Pro Glu Asp
                245                 250                 255

Gln Ile Val Leu Leu Lys Ser Ser Ala Ile Glu Val Ile Met Leu Arg
            260                 265                 270

Ser Asn Gln Ser Phe Thr Leu Asp Asp Asp Met Ser Trp Thr Cys Gly
        275                 280                 285

Ser Pro Asp Tyr Lys Tyr Gln Val Ser Asp Val Thr Arg Ala Gly His
    290                 295                 300

Ser Leu Glu Leu Ile Glu Pro Leu Ile Lys Phe Gln Val Gly Leu Lys
305                 310                 315                 320

Lys Leu Asn Leu His Glu Glu Glu His Val Leu Leu Met Ala Ile Cys
                325                 330                 335

Ile Val Ser Pro Asp Arg Pro Gly Val Gln Asp Ala Ala Leu Val Glu
            340                 345                 350

Ala Ile Gln Asp Arg Leu Ser Asn Thr Leu Gln Thr Tyr Ile Arg Cys
        355                 360                 365

Arg His Pro Pro Pro Gly Ser His Leu Leu Tyr Ala Lys Met Ile Gln
    370                 375                 380

Lys Leu Ala Asp Leu Arg Ser Leu Asn Glu Glu His Ser Lys Gln Tyr
385                 390                 395                 400

Arg Cys Leu Ser Phe Gln Pro Glu Ser Ser Met Lys Leu Thr Pro Leu
                405                 410                 415

Leu Phe Glu Val Phe Gly Asn Glu Ile Ser
            420                 425

<210>  50
<211>  12
<212>  PRT
<213>  artificial sequence

<220>
<223>  artificial sequence

<400>  50

Asn His Pro Met Leu Met Asn Leu Leu Lys Asp Asn

1               5                   10


<210>  51
<211>  20
<212>  PRT
<213>  Artificial sequence

<220>
<223>  Artificial sequence

<400>  51

Leu Ser Glu Thr His Pro Leu Leu Trp Thr Leu Leu Ser Ser Thr Glu
1               5                   10                  15


Gly Asp Ser Met
            20


<210>  52
<211>  19
<212>  PRT
<213>  Artificial sequence

<220>
<223>  Artificial sequence

<400>  52

Leu Thr Glu Met His Pro Ile Leu Thr Ser Leu Leu Gln Asn Gly Val
1               5                   10                  15


Asp His Val


<210>  53
<211>  34
<212>  DNA
<213>  Artificial sequence

<220>
<223>  Artificial sequence

<400>  53
atatctcgag atggtcttca cactcgaaga tttc                                  34


<210>  54
<211>  34
<212>  DNA
<213>  Artificial sequence

<220>
<223>  Artificial sequence

<400>  54
atatgtcgac actgttgatg gttactcgga acag                                  34


<210>  55

<211>  35
<212>  DNA
<213>  Artificial sequence

<220>
<223>  Artificial sequence

<400>  55
atatggatcc gtcttcacac tcgaagattt cgttg                                35


<210>  56
<211>  36
<212>  DNA
<213>  Artificial sequence

<220>
<223>  Artificial sequence

<400>  56
atatgcggcc gctaactgtt gatggttact cggaac                               36


<210>  57
<211>  42
<212>  DNA
<213>  Artificial sequence

<220>
<223>  Artificial sequence

<400>  57
tcgagatggt gaccggctac cggctgttcg aggagattct gg                        42


<210>  58
<211>  42
<212>  DNA
<213>  Artificial sequence

<220>
<223>  Artificial sequence

<400>  58
tcgaccagaa tctcctcgaa cagccggtag ccggtcacca tc                        42


<210>  59
<211>  42
<212>  DNA
<213>  Artificial sequence

<220>
<223>  Artificial sequence

<400>  59
gatccgtgac cggctaccgg ctgttcgagg agattctgta gc                        42


<210>  60
<211>  42
<212>  DNA
<213>  Artificial sequence

<220>
<223>  Artificial sequence

<400>  60
ggccgctaca gaatctcctc gaacagccgg tagccggtca cg                          42


<210>  61
<211>  38
<212>  DNA
<213>  Artificial sequence

<220>
<223>  Artificial sequence

<400>  61
atctcgagat gcggcccaag ctgtctgagg agcagcag                               38


<210>  62
<211>  35
<212>  DNA
<213>  Artificial sequence

<220>
<223>  Artificial sequence

<400>  62
aattggatcc ggagatctca ttgccaaaca cttcg                                  35


<210>  63
<211>  35
<212>  DNA
<213>  Artificial sequence

<220>
<223>  Artificial sequence

<400>  63
atatgtcgac cggcccaagc tgtctgagga gcagc                                  35


<210>  64
<211>  36
<212>  DNA
<213>  Artificial sequence

<220>
<223>  Artificial sequence

<400>  64
atatgcggcc gctcaggaga tctcattgcc aaacac                                 36


<210>  65
<211>  46
<212>  DNA
<213>  Artificial sequence

<220>
<223>  Artificial sequence

<400>  65
tcgacaacca cccgatgctc atgaaccttc ttaaagataa ttaagc                    46


<210>  66
<211>  46
<212>  DNA
<213>  Artificial sequence

<220>
<223>  Artificial sequence

<400>  66
ggccgcttaa ttatctttaa gaaggttcat gagcatcggg tggttg                    46


<210>  67
<211>  45
<212>  DNA
<213>  Artificial sequence

<220>
<223>  Artificial sequence

<400>  67
tcgagatgaa ccacccgatg ctcatgaacc ttcttaaaga taatg                     45


<210>  68
<211>  45
<212>  DNA
<213>  Artificial sequence

<220>
<223>  Artificial sequence

<400>  68
gatccattat ctttaagaag gttcatgagc atcgggtggt tcatc                     45


<210>  69
<211>  51
<212>  DNA
<213>  Artificial sequence

<220>
<223>  Artificial sequence

<400>  69
tcgagggagg tgggggtagt ggagggggag gtagcggtgg cggtggtagt g              51


<210>  70
<211>  51
<212>  DNA
<213>  Artificial sequence

<220>
<223>  Artificial sequence

<400>  70
tcgacactac caccgccacc gctacctccc cctccactac ccccacctcc c              51

<210>  71
<211>  51
<212>  DNA
<213>  Artificial sequence

<220>
<223>  Artificial sequence

<400>  71
gatccggtgg cggtggtagt gggggcggag gtagcggagg tgggggtagt a                51


<210>  72
<211>  51
<212>  DNA
<213>  Artificial sequence

<220>
<223>  Artificial sequence

<400>  72
gatctactac ccccacctcc gctacctccg cccccactac caccgccacc g                51


<210>  73
<211>  56
<212>  DNA
<213>  Artificial sequence

<220>
<223>  Artificial sequence

<400>  73
caacgaaatc ttcgagtgtg aagacgaatt ccagaatctc ctcgaacagc cggtag           56


<210>  74
<211>  56
<212>  DNA
<213>  Artificial sequence

<220>
<223>  Artificial sequence

<400>  74
ctaccggctg ttcgaggaga ttctggaatt cgtcttcaca ctcgaagatt tcgttg           56


<210>  75
<211>  56
<212>  DNA
<213>  Artificial sequence

<220>
<223>  Artificial sequence

<400>  75
gctgctcctc agacagcttg ggccggaatt cattatcttt aagaaggttc atgagc           56


<210>  76

<211>  56
<212>  DNA
<213>  Artificial sequence

<220>
<223>  Artificial sequence

<400>  76
gctcatgaac cttcttaaag ataatgaatt ccggcccaag ctgtctgagg agcagc          56


<210>  77
<211>  51
<212>  DNA
<213>  Artificial sequence

<220>
<223>  Artificial sequence

<400>  77
aattcggagg tggcggaagc ggtggcggcg ggagtggagg aggcggtagc g               51


<210>  78
<211>  51
<212>  DNA
<213>  Artificial sequence

<220>
<223>  Artificial sequence

<400>  78
aattcgctac cgcctcctcc actcccgccg ccaccgcttc cgccacctcc g               51


<210>  79
<211>  66
<212>  DNA
<213>  Artificial sequence

<220>
<223>  Artificial sequence

<400>  79
tcgaccatgt cgagatgcac ccactcctga tgggacttct catggaatcg cagtggggcg      60

cctagc                                                                 66


<210>  80
<211>  66
<212>  DNA
<213>  Artificial sequence

<220>
<223>  Artificial sequence

<400>  80
ggccgctagg cgccccactg cgattccatg agaagtccca tcaggagtgg gtgcatctcg      60

acatgg                                                                 66

<210>  81
<211>  66
<212>  DNA
<213>  Artificial sequence

<220>
<223>  Artificial sequence

<400>  81
tcgacgctgc ttccaaacat aaacaactgt cggagcttct acgaggaggc agcggctcta      60

gttagc                                                                  66


<210>  82
<211>  66
<212>  DNA
<213>  Artificial sequence

<220>
<223>  Artificial sequence

<400>  82
ggccgctaac tagagccgct gcctcctcgt agaagctccg acagttgttt atgtttggaa      60

gcagcg                                                                  66


<210>  83
<211>  66
<212>  DNA
<213>  Artificial sequence

<220>
<223>  Artificial sequence

<400>  83
tcgacggaca cgaaccactc acgcttctgg aacgactgct aatggacgac aagcaggcgg      60

tctagc                                                                  66


<210>  84
<211>  66
<212>  DNA
<213>  Artificial sequence

<220>
<223>  Artificial sequence

<400>  84
ggccgctaga ccgcctgctt gtcgtccatt agcagtcgtt ccagaagcgt gagtggttcg      60

tgtccg                                                                  66


<210>  85
<211>  69
<212>  DNA
<213>  Artificial sequence

<220>
<223>  Artificial sequence

<400>  85
tcgacagcaa ggtgtctcag aacccaattc ttaccagttt gttgcaaatc acagggaacg      60

gggggtagc                                                              69


<210>  86
<211>  69
<212>  DNA
<213>  Artificial sequence

<220>
<223>  Artificial sequence

<400>  86
ggccgctacc ccccgttccc tgtgatttgc aacaaactgg taagaattgg gttctgagac      60

accttgctg                                                              69


<210>  87
<211>  69
<212>  DNA
<213>  Artificial sequence

<220>
<223>  Artificial sequence

<400>  87
tcgacaaata ctctcaaacc agtcacaaac tagtgcagct tttgacaaca actgccgaac      60

agcagtagc                                                              69


<210>  88
<211>  69
<212>  DNA
<213>  Artificial sequence

<220>
<223>  Artificial sequence

<400>  88
ggccgctact gctgttcggc agttgttgtc aaaagctgca ctagtttgtg actggtttga      60

gagtatttg                                                              69


<210>  89
<211>  69
<212>  DNA
<213>  Artificial sequence

<220>
<223>  Artificial sequence

<400>  89
tcgacaaaga atcaaaagac catcagctcc tacgctatct tttagataaa gatgagaaag      60

atttatagc                                                              69

<210>  90
<211>  69
<212>  DNA
<213>  Artificial sequence

<220>
<223>  Artificial sequence

<400>  90
ggccgctata aatctttctc atctttatct aaaagatagc gtaggagctg atggtctttt      60

gattctttg                                                              69


<210>  91
<211>  51
<212>  DNA
<213>  Artificial sequence

<220>
<223>  Artificial sequence

<400>  91
tcgaccaggc ccagcagaag agcctccttc agcagctact gactgaatag c               51


<210>  92
<211>  51
<212>  DNA
<213>  Artificial sequence

<220>
<223>  Artificial sequence

<400>  92
ggccgctatt cagtcagtag ctgctgaagg aggctcttct gctgggcctg g               51


<210>  93
<211>  66
<212>  DNA
<213>  Artificial sequence

<220>
<223>  Artificial sequence

<400>  93
tcgacgaggc agaagagccg tctctactta agaagctctt actggcacca gccaacactc      60

agtagc                                                                 66


<210>  94
<211>  66
<212>  DNA
<213>  Artificial sequence

<220>
<223>  Artificial sequence

<400>  94
ggccgctact gagtgttggc tggtgccagt aagagcttct taagtagaga cggctcttct      60

gcctcg                                                                66

<210>  95
<211>  67
<212>  DNA
<213>  Artificial sequence

<220>
<223>  Artificial sequence

<400>  95
tcgacctacc atacgaaggc agtctacttc tcaagctact tagagcccca gttgaggagg      60

tgtaggc                                                               67

<210>  96
<211>  67
<212>  DNA
<213>  Artificial sequence

<220>
<223>  Artificial sequence

<400>  96
ggccgcctac acctcctcaa ctggggctct aagtagcttg agaagtagac tgccttcgta      60

tggtagg                                                               67

<210>  97
<211>  66
<212>  DNA
<213>  Artificial sequence

<220>
<223>  Artificial sequence

<400>  97
tcgaccacgt ttatcagcat cctctacttc tcagcctact tagctcagag cacgagtcag      60

gttagc                                                                66

<210>  98
<211>  66
<212>  DNA
<213>  Artificial sequence

<220>
<223>  Artificial sequence

<400>  98
ggccgctaac ctgactcgtg ctctgagcta agtaggctga gaagtagagg atgctgataa      60

acgtgg                                                                66

<210>  99
<211>  42
<212>  DNA
<213>  Artificial sequence

<220>
<223>  Artificial sequence

<400>  99
tcgaccataa aattctacac cggctcttac aggagggtta gc                          42


<210>  100
<211>  42
<212>  DNA
<213>  Artificial sequence

<220>
<223>  Artificial sequence

<400>  100
ggccgctaac cctcctgtaa gagccggtgt agaattttat gg                          42


<210>  101
<211>  516
<212>  DNA
<213>  Artificial sequence

<220>
<223>  Artificial sequence

<400>  101
atggtcttca cactcgaaga tttcgttggg gactggcgac agacagccgg ctacaacctg       60

gaccaagtcc ttgaacaggg aggtgtgtcc agtttgtttc agaatctcgg ggtgtccgta      120

actccgatcc aaaggattgt cctgagcggt gaaaatgggc tgaagatcga catccatgtc      180

atcatcccgt atgaaggtct gagcggcgac caaatgggcc agatcgaaaa aatttttaag      240

gtggtgtacc ctgtggatga tcatcacttt aaggtgatcc tgcactatgg cacactggta      300

atcgacgggg ttacgccgaa catgatcgac tatttcggac ggccgtatga aggcatcgcc      360

gtgttcgacg gcaaaaagat cactgtaaca gggaccctgt ggaacggcaa caaaattatc      420

gacgagcgcc tgatcaaccc cgacggctcc ctgctgttcc gagtaaccat caacggagtg      480

accggctggc ggctgtgcga acgcattctg gcgtaa                                516


<210>  102
<211>  477
<212>  DNA
<213>  Artificial sequence

<220>
<223>  Artificial sequence

<400>  102
atggtcttca cactcgaaga tttcgttggg gactgggaac agacagccgc ctacaacctg       60

gaccaagtcc ttgaacaggg aggtgtgtcc agtttgctgc agaatctcgc cgtgtccgta      120

actccgatcc aaaggattgt ccggagcggt gaaaatgccc tgaagatcga catccatgtc      180

atcatcccgt atgaaggtct gagcgccgac caaatggccc agatcgaaga ggtgtttaag 240

gtggtgtacc ctgtggatga tcatcacttt aaggtgatcc tgccctatgg cacactggta 300

atcgacgggg ttacgccgaa catgctgaac tatttcggac ggccgtatga aggcatcgcc 360

gtgttcgacg gcaaaaagat cactgtaaca gggaccctgt ggaacggcaa caaaattatc 420

gacgagcgcc tgatcacccc cgacggctcc atgctgttcc gagtaaccat caacagt 477

<210> 103
<211> 36
<212> DNA
<213> Artificial sequence

<220>
<223> Artificial sequence

<400> 103
atggtgaccg gctaccggct gttcgaggag attctg 36

<210> 104
<211> 171
<212> PRT
<213> Artificial sequence

<220>
<223> Artificial sequence

<400> 104

Met Val Phe Thr Leu Glu Asp Phe Val Gly Asp Trp Arg Gln Thr Ala
1               5                   10                  15

Gly Tyr Asn Leu Asp Gln Val Leu Glu Gln Gly Gly Val Ser Ser Leu
            20                  25                  30

Phe Gln Asn Leu Gly Val Ser Val Thr Pro Ile Gln Arg Ile Val Leu
        35                  40                  45

Ser Gly Glu Asn Gly Leu Lys Ile Asp Ile His Val Ile Ile Pro Tyr
    50                  55                  60

Glu Gly Leu Ser Gly Asp Gln Met Gly Gln Ile Glu Lys Ile Phe Lys
65                  70                  75                  80

Val Val Tyr Pro Val Asp Asp His His Phe Lys Val Ile Leu His Tyr
                85                  90                  95

Gly Thr Leu Val Ile Asp Gly Val Thr Pro Asn Met Ile Asp Tyr Phe
            100                 105                 110

Gly Arg Pro Tyr Glu Gly Ile Ala Val Phe Asp Gly Lys Lys Ile Thr
        115                 120                 125

Val Thr Gly Thr Leu Trp Asn Gly Asn Lys Ile Ile Asp Glu Arg Leu
    130                 135                 140

Ile Asn Pro Asp Gly Ser Leu Leu Phe Arg Val Thr Ile Asn Gly Val
145                 150                 155                 160

Thr Gly Trp Arg Leu Cys Glu Arg Ile Leu Ala
                165                 170

<210>  105
<211>  159
<212>  PRT
<213>  Artificial sequence

<220>
<223>  Artificial sequence

<400>  105

Met Val Phe Thr Leu Glu Asp Phe Val Gly Asp Trp Glu Gln Thr Ala
1               5                   10                  15

Ala Tyr Asn Leu Asp Gln Val Leu Glu Gln Gly Gly Val Ser Ser Leu
            20                  25                  30

Leu Gln Asn Leu Ala Val Ser Val Thr Pro Ile Gln Arg Ile Val Arg
        35                  40                  45

Ser Gly Glu Asn Ala Leu Lys Ile Asp Ile His Val Ile Ile Pro Tyr
    50                  55                  60

Glu Gly Leu Ser Ala Asp Gln Met Ala Gln Ile Glu Glu Val Phe Lys
65                  70                  75                  80

Val Val Tyr Pro Val Asp Asp His His Phe Lys Val Ile Leu Pro Tyr
                85                  90                  95

Gly Thr Leu Val Ile Asp Gly Val Thr Pro Asn Met Leu Asn Tyr Phe
            100                 105                 110

Gly Arg Pro Tyr Glu Gly Ile Ala Val Phe Asp Gly Lys Lys Ile Thr
        115                 120                 125

Val Thr Gly Thr Leu Trp Asn Gly Asn Lys Ile Ile Asp Glu Arg Leu
    130                 135                 140

Ile Thr Pro Asp Gly Ser Met Leu Phe Arg Val Thr Ile Asn Ser
145                 150                 155

<210>  106
<211>  11
<212>  PRT
<213>  Artificial sequence

<220>
<223>  Artificial sequence

<400>  106

Val Thr Gly Tyr Arg Leu Phe Glu Glu Ile Leu
1               5                   10


<210>  107
<211>  591
<212>  DNA
<213>  Oplophorus gracilirostris

<400>  107
atggcgtact ccactctgtt cataattgca ttgaccgccg ttgtcactca agcttcctca      60

actcaaaaat ctaacctaac ttttacgttg gcagatttcg ttggagactg gcaacagaca     120

gctggataca accaagatca agtgttagaa caaggaggat tgtctagtct gttccaagcc     180

ctgggagtgt cagtcacgcc catacagaaa gttgtactgt ctggggagaa tgggttaaaa     240

gctgatattc atgtcataat accttacgag ggactcagtg gttttcaaat gggtctaatt     300

gaaatgatct tcaaagttgt ttaccccgtg gatgatcatc atttcaagat tattctccat     360

tatggtacac tcgttattga cggtgtaaca cccaacatga ttgactactt tggaagacct     420

taccctggaa ttgctgtatt tgacggcaag cagatcacag ttactggaac tctgtggaac     480

ggcaacaaga tctatgatga gaggctaatc aaccctgatg gttcactcct cttcagagtt     540

actatcaatg gagtcacggg atggaggctt tgcgagaaca ttcttgccta a              591


<210>  108
<211>  196
<212>  PRT
<213>  Oplophorus gracilirostris

<400>  108

Met Ala Tyr Ser Thr Leu Phe Ile Ile Ala Leu Thr Ala Val Val Thr
1               5                   10                  15


Gln Ala Ser Ser Thr Gln Lys Ser Asn Leu Thr Phe Thr Leu Ala Asp
            20                  25                  30


Phe Val Gly Asp Trp Gln Gln Thr Ala Gly Tyr Asn Gln Asp Gln Val
        35                  40                  45


Leu Glu Gln Gly Gly Leu Ser Ser Leu Phe Gln Ala Leu Gly Val Ser
    50                  55                  60

Val Thr Pro Ile Gln Lys Val Val Leu Ser Gly Glu Asn Gly Leu Lys
65                  70                  75                  80

Ala Asp Ile His Val Ile Ile Pro Tyr Glu Gly Leu Ser Gly Phe Gln
                85                  90                  95

Met Gly Leu Ile Glu Met Ile Phe Lys Val Val Tyr Pro Val Asp Asp
            100                 105                 110

His His Phe Lys Ile Ile Leu His Tyr Gly Thr Leu Val Ile Asp Gly
        115                 120                 125

Val Thr Pro Asn Met Ile Asp Tyr Phe Gly Arg Pro Tyr Pro Gly Ile
    130                 135                 140

Ala Val Phe Asp Gly Lys Gln Ile Thr Val Thr Gly Thr Leu Trp Asn
145                 150                 155                 160

Gly Asn Lys Ile Tyr Asp Glu Arg Leu Ile Asn Pro Asp Gly Ser Leu
                165                 170                 175

Leu Phe Arg Val Thr Ile Asn Gly Val Thr Gly Trp Arg Leu Cys Glu
            180                 185                 190

Asn Ile Leu Ala
        195

<210>  109
<211>  19
<212>  PRT
<213>  Artificial sequence

<220>
<223>  Artificial sequence

<400>  109

His Val Glu Met His Pro Leu Leu Met Gly Leu Leu Met Glu Ser Gln
1               5                   10                  15

Trp Gly Ala

<210>  110
<211>  20
<212>  PRT
<213>  Artificial sequence

<220>
<223>  Artificial sequence

<400>  110

Asp Ala Ala Ser Lys His Lys Gln Leu Ser Glu Leu Leu Arg Gly Gly
1               5                   10                  15

Ser Gly Ser Ser
            20

<210>  111
<211>  19
<212>  PRT
<213>  Artificial sequence

<220>
<223>  Artificial sequence

<400>  111

Gly His Glu Pro Leu Thr Leu Leu Glu Arg Leu Leu Met Asp Asp Lys
1               5                   10                  15

Gln Ala Val

<210>  112
<211>  19
<212>  PRT
<213>  Artificial sequence

<220>
<223>  Artificial sequence

<400>  112

Lys Val Ser Gln Asn Pro Ile Leu Thr Ser Leu Leu Gln Ile Thr Gly
1               5                   10                  15

Asn Gly Gly

<210>  113
<211>  19
<212>  PRT
<213>  Artificial sequence

<220>
<223>  Artificial sequence

<400>  113

Tyr Ser Gln Thr Ser His Lys Leu Val Gln Leu Leu Thr Thr Thr Ala
1               5                   10                  15

Glu Gln Gln

<210>  114
<211>  19
<212>  PRT
<213>  Artificial sequence

<220>
<223>  Artificial sequence

<400>  114

Glu Ser Lys Asp His Gln Leu Leu Arg Tyr Leu Leu Asp Lys Asp Glu
1               5                   10                  15

Lys Asp Leu

<210>  115
<211>  14
<212>  PRT
<213>  Artificial sequence

<220>
<223>  Artificial sequence

<400>  115

Gln Ala Gln Gln Lys Ser Leu Leu Gln Gln Leu Leu Thr Glu
1               5                   10

<210>  116
<211>  19
<212>  PRT
<213>  Artificial sequence

<220>
<223>  Artificial sequence

<400>  116

Glu Ala Glu Glu Pro Ser Leu Leu Lys Lys Leu Leu Leu Ala Pro Ala
1               5                   10                  15

Asn Thr Gln

<210>  117
<211>  19
<212>  PRT
<213>  Artificial sequence

<220>
<223>  Artificial sequence

<400>  117

Leu Pro Tyr Glu Gly Ser Leu Leu Leu Lys Leu Leu Arg Ala Pro Val

1               5                   10                  15

Glu Glu Val

<210>  118
<211>  19
<212>  PRT
<213>  Artificial sequence

<220>
<223>  Artificial sequence

<400>  118

His Val Tyr Gln His Pro Leu Leu Leu Ser Leu Leu Ser Ser Glu His
1               5                   10                  15

Glu Ser Gly

<210>  119
<211>  11
<212>  PRT
<213>  Artificial sequence

<220>
<223>  Artificial sequence

<400>  119

His Lys Ile Leu His Arg Leu Leu Gln Glu Gly
1               5                   10

## Description

### TECHNICAL FIELD

The present invention relates to a split luciferase and to a method of detecting vitamin D receptor ligand using the same in high sensitivity.

### CROSS REFERENCE TO RELATED APPLICATIONS

This application claims priority to Japanese Patent Application No. 2017-210604, filed October 31, 2017, the entire disclosure of which is specifically incorporated herein by reference.

### BACKGROUND OF THE INVENTION

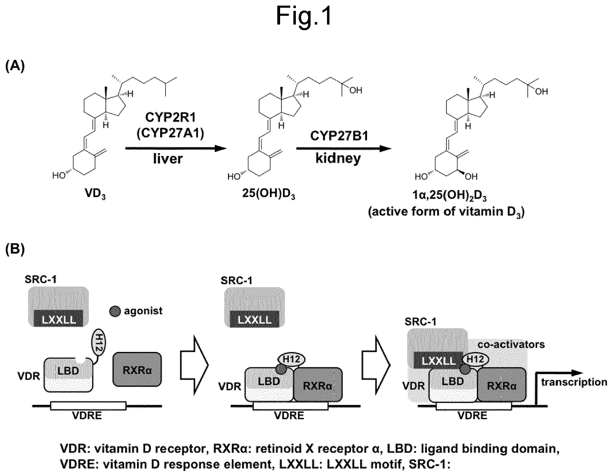

After vitamin D 3 (VD₃) is produced in the skin, it is transported to the liver by circulating vitamin D binding proteins (DBPs) and metabolized to 25 hydroxyvitamin D3 [25(OH)D₃]. It then binds to DBPs in the blood and is transported to the kidneys, where it is metabolized to 1α,25-dihydroxyvitamin D3 [1α,25(OH)₂)D₃], the most potent physiological activity (Fig. 1A). The 1α,25(OH)₂D₃, called active forms of vitamin D3, forms a heterodimer with the retinoid-X receptor (RXR) after specific binding to vitamin D receptor (VDR), which is present in the nucleus of target cell. It is then complexed with transcriptional coactivator, such as steroid receptor coactivating factor 1 (SRC-1) and vitamin D receptor interacting protein 205(DRIP-205), and binds to vitamin D responsive element (VDRE) on the DNAs to activate the various genes involved in the regulation of bone metabolism, cytodifferentiation and proliferation, and immune function (Fig. 1B).

Therefore, vitamin D deficiency in childhood may cause rickets and osteomalacia. Age-related vitamin D deficiencies are also said to be associated with osteoporosis, cancer, diabetes, arteriosclerosis, autoimmune disease, and cerebral disease such as Alzheimer's disease and Parkinson's disease. In recent years, vitamin D and its derivatives have been increasingly reported to improve proliferation inhibition of cancer cell, and memory and learning abilities, and are expected to be a therapeutic agent for cancer and dementia. However, despite the fact that more than 1,000 vitamin D derivatives have been synthesized and studied, only a few compound have reached pharmaceutical products. The main reasons for this are the low affinity of vitamin D derivatives to VDRs and the rapid metabolism of VDRs by various metabolic enzyme in the body to inactive ones. Therefore, developing a compound that has high affinity to VDRs and is less susceptible to inactivation by metabolic enzyme is likely to be a therapeutic agent of many of diseases discussed above. It is considered that the construction of a detecting system for searching and evaluating VDR-binding compound in a simple and short time out of a vast number of vitamin D derivatives plays a vital part in drug development.

In the past, competition assays using cattle thymus VDRs (non-patent document 1) and methods using coactivators such as SRC-1 (non-patent document 2) have been widely used as systems for screening VDR ligand and measuring its affinity. Meanwhile, as a system using cell, a system using a vitamin D responsive sequence (VDRE)-containing promotor luciferase reporter assay is known (non-patent document 3).

Patent document 1: JP-A-2016-163559

Non-patent document 1: D. Abe, T. Sakaki, T. Kusudo, A. Kittaka, N. Saito, Y. Suhara, T. Fujishima, H. Takayama, H. Hamamoto, M. Kamakura, M. Ohta, K. Inouye, Metabolism of 2 alpha-propoxy-1 alpha,25-dihydroxyvitamin D3 and 2 alpha-(3-hydroxypropoxy)-1 alpha,25-dihydroxyvitamin D3 by human CYP27A1 and CYP24A1, Drug metabolism and disposition: the biological fate of chemicals, 33 (2005) 778-784.

Non-patent document 2: K. Yasuda, S. Ikushiro, M. Kamakura, M. Takano, N. Saito, A. Kittaka, T.C. Chen, M. Ohta, T. Sakaki, Human cytochrome P450-dependent differential metabolism among three 2alpha-substituted-1alpha,25-dihydroxyvitamin D(3) analogs, The Journal of steroid biochemistry and molecular biology, 133 (2013) 84-92. Non-patent document 3: M. Nakabayashi, Y. Tsukahara, Y. Iwasaki-Miyamoto, M. Mihori-Shimazaki, S. Yamada, S. Inaba, M. Oda, M. Shimizu, M. Makishima, H. Tokiwa, T. Ikura, N. Ito, Crystal structures of hereditary vitamin D-resistant rickets-associated vitamin D receptor mutants R270L and W282R bound to 1,25-dihydroxyvitamin D3 and synthetic ligands, Journal of medicinal chemistry, 56 (2013) 6745-6760.

Non-patent document 4: H. Mano, M. Nishikawa, K. Yasuda, S. Ikushiro, N. Saito, M. Takano, A. Kittaka, T. Sakaki, Development of Novel Bioluminescent Sensor to Detect and Discriminate between Vitamin D Receptor Agonists and Antagonists in Living Cells, Bioconjugate chemistry, 26 (2015) 2038-2045.

Non-patent document 5: L.A. Zella, C.Y. Chang, D.P. McDonnell, J.W. Pike, The vitamin D receptor interacts preferentially with DRIP205-like LxxLL motifs, Archives of biochemistry and biophysics, 460 (2007) 206-212.

The entire description of patent document 1 and non-patent documents 1-5 are specifically incorporated herein by reference.

### Brief description of the invention

Although detection in high sensitivity is available by the methods described in non-patent documents 1 and 2 without use of cell, they are costly and also have the disadvantage of positively determining substance which is not transparent to cell. On the other hand, in the methods described in non-patent document 3, effects of VDR ligand including cell membrane permeability and cell toxicity can be evaluated, but there are problems that direct binding with VDRs is not detected, and it takes more than 12 hours before the assessment is completed.

In order to solve these problems, a new VDR ligand detection system using split luciferase was developed in 2015 (non-patent document 4, patent document 1). The detection system was a fused protein of the N-terminal (LucN) or C-terminal (LucC) regions of split firefly luciferase attached to the N-terminal and C-terminal ends of VDR ligand (LBD), named LucN-LBD-LucC and LucC-LBD-LucN biosensors. In this biosensor, when ligand binds to the LBD existing in biosensor, light emission of luciferase changes with structure change of the LBD. In particular, the biggest distinction is the ability to distinguish between agonist and antagonists in a single system, since binding of agonist decreases light emission of luciferase and binding of antagonists increases light emission.

The present inventors are aiming at visualization and spatio-temporal analysis of VDR agonist not only at cell level but also at the individual level, and LucN-LBD-LucC and LucC-LBD-LucN biosensors developed so far are unsuitable because, in addition to the low light emission, light emission decreases when agonist binds. That is, there is a need for an improvement to biosensor in which light emission increases when VDR agonist is detected.

The problem to be solved by the present invention is to provide a biosensor in which light emission increases when agonist binds, and the present invention aims to provide such a biosensor. In addition, the present invention provides a convenient method to screen a substance which binds strongly to vitamin D receptor.

Generally, in biosensor using the split type luciferase, when a linker sequence such as a GGGGS sequence or a repeating sequence thereof is inserted into the respective linking sites of LucN, LBD, and LucC, the sensitivities, reactivities, and the like may be improved. In addition to RXRs, VDR also binds to transcriptional coactivator such as SRC-1 and DRIP-205, as described above, which has been reported to occur via specific sequences (NHPMLMNLLKDN, LSETHPLLWTLLSSTEGDSM and LTEMHPILTSLLQNGVDHV as examples of amino acid sequence) called LXXLL motifs present in transcriptional coactivator (non-patent document 5). Therefore, the possibility of improving the performance of biosensor was considered by inserting LXXLL sequences into the respective connecting sites of LucN, LBD and LucC in addition to linker sequence.

Based on prepared LucN-LBD-LucC biosensor, we prepared a LucN-(GGGGS)×3-LBD-LucC biosensor with a three (GGGGS) repeating sequence, (GGGGS)×3, linker inserted between LucN and LBD, and investigated the response to agonist. However, similar to LucN-LBD-LucC biosensor, the luminescence was reduced in response to agonist.

Thus, as a further improvement, LucN-LXXLL-(GGGGS)×3-LBD-LucC biosensor with a LXXLL sequence inserted between LucN and (GGGGS)×3 was newly prepared and successfully resulted in obtaining a biosensor of the type in which light emission amount increased in response to agonist and light emission increased in response to agonist for which the present invention was aimed. These biosensors are called single molecular type because their light emission changes with structure changes within a molecule.

Also, focusing on interaction between LXXLL sequence and the LBD after agonist binding, biosensor consisting of two molecules of LucN-LXXLL and LBD-LucC was also developed, expecting that light emission amount increases when interaction between LXXLL sequence and LBD is detected. This type of biosensor is called double molecular type because its light emission is changed by interaction between two molecules. In the double molecular type, it has been confirmed that LucN and LucC are reconstituted and light emission is restored when an interaction occurs between LXXLL sequences and LBD in biosensor responsive to agonist.

Both single molecular type and double molecular type may be able to visualize vitamin D and its metabolite at cell and individual levels because of their increased light emission responsive to agonist. In addition, the newly developed preparation of recombinant animal (such as zebrafish and mouse) encoding the biosensor could visually analyze vitamin D distributions and mechanism during generation, development and growth of individuals that are currently difficult in analysis technology.

In addition, in the present invention, the use of 19 kDa luciferase or its variant from deep-sea shrimp (*Oplophorus gracilirostris*) as luciferase and the use of furimazine instead of natural coelenterazine as substrate, increased light intensity and made it available in higher sensitivity. The present invention has been accomplished based on these findings

### SUMMART OF THE INVENTION

The present invention is as follows:
[1] A fused protein of any one of (1) to (3) below:
   (1) a fused protein comprising in random order N terminal domain of luciferase (hereinafter referred to as LucN), at least one vitamin D binding domain of vitamin D receptor (hereinafter referred to as VDR) and C terminal domain of luciferase (hereinafter referred to as LucC), and further in random order comprising in random order one or both of LX¹X²LL sequences, wherein X¹ is M, W, T, S, E, V, R, Q, K, L or H and X² is N, T, S, G, E, R, Q, Y or K, and a linker sequence,
   (2) a fused protein comprising in random order LucN or LucC and LX¹X²LL sequence, wherein X¹ is M, W, T, S, E, V, R, Q, K, L or H and X² is N, T, S, G, E, R, Q, Y or K, wherein the fused protein may comprise further in random order a linker sequence,
   (3) a fused protein comprising in random order at least one vitamin D binding domain of VDR and LucC or LucN, wherein the fused protein may comprise further in random order a linker sequence.
[2] The fused protein according to [1], wherein the linker sequence is at least one selected from the group consisting of a GGGGS sequence and an EAAAK sequence, preferably 3 repeating sequences of GGGGS, (GGGGS)×3.
[3] The fused protein according to [1] or [2], wherein the fused protein (1) or (2) further comprises an insertion sequence A of 1 to 20 of amino acid number between LucN or LucC and LX¹X²LL sequence.
[4] The fused protein according to any one of [1] to [3], wherein the fused protein (1) or (2) further comprises an insertion sequence B of 1 to 20 of amino acid number between LX¹X²LL sequence and linker sequence.
[5] The fused protein according to any one of [1] to [4], wherein the luciferase is selected from the group consisting of Firefly luciferase; *Pyrophorus plagiophthalamus* luciferase; Emerald luciferase; *Renilla renifonnis* luciferase; *Cypridina noctiluca* luciferase; *Gaussia princeps* luciferase; *Oplophorus gracilirostris* luciferase; and variant of *Oplophorus gracilirostris* luciferase.
[6] The fused protein according to any one of [1] to [4], wherein the Firefly luciferase has an amino acid sequence denoted by SEQ ID NO: 36 of Sequence Listing, and the N terminal domain has an amino acid sequence of number 1 to 415 and the C terminal domain has an amino acid sequence of number 416 to 550.
[7] The fused protein according to any one of [1] to [4], wherein the *Renilla reniformis* luciferase has an amino acid sequence denoted by SEQ ID NO: 38 of Sequence Listing, and the N terminal domain has an amino acid sequence of number 1 to 229 and the C terminal domain has an amino acid sequence of number 230 to 311.
[8] The fused protein of any one of [1]-[4], wherein the Emerald luciferase has an amino acid sequence denoted by SEQ ID NO: 40 of Sequence Listing, and the N terminal domain has an amino acid sequence of number 1 to n and the C terminal domain has an amino acid sequence of number n+1 to 542, and n is an integer of any of 388 to 422.
[9] The fused protein according to any one of [1] to [4], wherein the variant of *Oplophorus gracilirostris* luciferase (hereinafter referred to as NLuc) has an amino acid sequence denoted by SEQ ID NO: 104 of Sequence Listing, and the N terminal domain of NLuc has amino acid sequence denoted by SEQ ID NO: 105 of Sequence Listing and the C terminal domain of NLuc has an amino acid sequence denoted by SEQ ID NO: 106 of Sequence Listing.
[10] The fused protein according to any one of [1] to [9], wherein the VDR is a VDR of human, mouse, rat, monkey, dog, or cattle.
[11] The fused protein according to claim 10, wherein the human VDR has an amino acid sequence denoted SEQ ID NO: 42 of Sequence Listing of which vitamin D binding domain has an amino acid sequence of number 121 to 427,
   the mouse VDR has an amino acid sequence denoted by SEQ ID NO: 44 of Sequence Listing, vitamin D binding domain of which has an amino acid sequence of numbers 121 to 422,
   the Rat VDR has an amino acid sequence denoted SEQ ID NO: 46 of Sequence Listing, vitamin D binding domain of which has an amino acid sequence of numbers 121 to 423, the Monkey VDR has an amino acid sequence denoted SEQ ID NO: 47 of Sequence Listing, vitamin D binding domain of which has an amino acid sequence of numbers 121 to 434,
   the Dog VDR has an amino acid sequence denoted SEQ ID NO: 48 of Sequence Listing, vitamin D binding domain of which has an amino acid sequence of numbers 121 to 427, the cattle VDR has an amino acid sequence denoted by SEQ ID NO: 49 of Sequence Listing, vitamin D binding domain of which has an amino acid sequence of numbers 121 to 426.
[12] The fused protein according to any one of [1] to [11], wherein the amino acid sequence of vitamin D binding domain has a mutation.
[13] The fused protein according to any one of [1] to [12], which has two vitamin D binding domains.
[14] The fused protein according to any one of [1] to [13], which further comprises two amino acids of any of VD, GS, VE, RS, or EF between any of LucN, LucC, LX¹X²LL sequence, linker sequence, insertion sequence A and insertion sequence B.
[15] A DNA encoding amino acid sequence of the fused protein according to any one of [1] to [14].
[16] A plasmid comprising the DNA according to [15] in a vector.
[17] A transformed cell which is a cell into which the plasmid according to [16] has been introduced.
[18] The transformed cell according to [17], wherein said cell is a cell derived from a mammal or an insect.
[19] A screening method of a substance that shows binding property to vitamin D receptor (hereinafter referred to as VDR) comprising
   cultivating the transformed cell according to [17] or [18] in the presence of a substrate of luciferase of which DNA is contained in the transformed cell, Mg²⁺, ATP and oxygen, and candidate substance, wherein a fused protein of the luciferase is fused protein (1) or fused protein (2) and fused protein (3), wherein when the fused protein (2) comprises LucN, the fused protein (3) comprises LucC and when the fused protein (2) comprises LucC, the fused protein (3) comprises LucN;
   measuring light emission in the cultivation and/or after the cultivation; and assessing the binding of candidate substance to the VDR based on the light emission and selecting a substance exhibiting binding property to the VDR.
[20] The sequence method according to [19], wherein a DNA encoding amino acid sequence of vitamin D binding domain, which is contained in the plasmid introduced into the transformed cell has a mutation in the amino acid sequence of vitamin D binding domain, and a candidate substance that specifically binds to a vitamin D binding domain having a mutation which is involved in disease is screened from the relation between the mutation of the amino acid sequence and the disease.
[21] A screening method of substance that shows binding property to vitamin D receptor (hereinafter referred to as VDR) comprising
   subjecting the fused protein according to any one of [1] to [14] to interaction with a coexistent substrate of luciferase, Mg²⁺, ATP and oxygen, and candidate substance, wherein the fused protein is fused protein (1) or fused protein (2) and fused protein (3), wherein when the fused protein (2) comprises LucN, the fused protein (3) comprises LucC and when the fused protein (2) comprises LucC, the fused protein (3) comprises LucN,
   measuring light emission during the interaction and/or after the interaction, and assessing the binding of candidate substance to the VDR based on the light emission and selecting a substance exhibiting binding property to the VDR.
[22] Screening method according to [21], wherein the amino acid sequence of the vitamin D binding domain contained in the fused protein has a mutation, and a candidate substance that specifically binds to the vitamin D binding domain having a mutation involved in disease is screened from the relation between the mutation of the amino acid sequence and the disease.
[23] The screening method according to any one of [19] to [22], wherein candidate substance is a compound having steroid nucleus, fatty acids, fat-soluble vitamins, or polyphenols.

### [Effect of the Invention]

The present invention can provide a biosensor in which light emission increases when agonist binds.

### BRIEF DESCRIPTION OF THE DRAWINGS

Figure 1 depicts (A) Metabolism of vitamin D 3 (VD₃) to 1α,25-dihydroxyvitamin D3 [1α,5(OH)2D₃], and (B) vitamin D dependent signaling.
Figure 2 depicts structure of single molecular type and double molecular type biosensor
Figure 3 depicts principle of variation in light emission of single molecular type biosensor.
Figure 4 depicts principle of variation in light emission of double molecular type biosensor.
Figure 5A depicts LucN-(GGGGS)×3-LBD-LucC biosensor 1α,25(OH)₂D₃ Responsiveness.
Figure 5B depicts 1α,25(OH)₂D₃ responsiveness of LucN-NHPMLMNLLKDN-(GGGGS)×3-LBD-LucC biosensor.
Figure 6 depicts concentration-dependence of LucN-NHPMLMNLLKDN-(GGGGS)×3-LBD-LucC biosensor on 25(OH)D₃ and 1α,25(OH)₂D₃.
Figure 7A depicts optimization of promotor and LXXLL Sequences to express biosensor protein.
Figure 7B depicts optimization of promotor and LXXLL Sequences to express biosensor protein.
Figure 8 depicts comparisons of LucN-NHPMLMNLLKDN-(GGGGS)×3-LBD-LucC and LucN-VDNHPMLMNLLKDN-(GGGGS)×3-LBD-LucC biosensors.
Figure 9 depicts responsiveness of double molecular type biosensor to 1α,25(OH)₂D₃.
Figure 10A depicts concentration-dependency of single molecular type biosensor on 1α,25(OH)₂D₃ concentration.
Figure 10B depicts concentration-dependency of double molecular type biosensor on 1α,25(OH)₂D₃ concentration
Figure 11 depicts concentration-dependency comparisons of LucN-VDNHPMLMNLLKDN-(GGGGS)×3-LBD-LucC and LucN-VDNHPMLMNLLKDN-(GGGGS)×3-LBD[Δ165-215 aa]-LucC biosensor on 1α,25(OH)₂D₃
Figure 12A depicts comparisons of responsiveness of HAT-LucN-VDNHPMLMNLLKDN-(GGGGS)×3-LBD-LucC and 6xHN-LucN-VDNHPMLMNLLKDN-(GGGGS)×3-LBD-LucC biosensor to 1α,25(OH)₂D₃.
Figure 12B depicts comparisons of expression levels of HAT-LucN-VDNHPMLMNLLKDN-(GGGGS)×3-LBD-LucC and 6xHN-LucN-VDNHPMLMNLLKDN-(GGGGS)×3-LBD-LucC biosensors.
Figure 13A depicts optimization of light emission measuring time for 6xHN-LucN-VDNHPMLMNLLKDN-(GGGGS)×3-LBD-LucC biosensor.
Figure 13B depicts optimization of light emission measuring time for 6xHN-LucN-VDNHPMLMNLLKDN-(GGGGS)×3-LBD-LucC biosensor.
Figure 14 depicts concentration-dependency comparisons of 6xHN-LucN-VDNHPMLMNLLKDN-(GGGGS)×3-LBD-LucC biosensor on 25(OH)D₃ and 1α,25(OH)₂D₃.
Figure 15 depicts responsiveness of 6xHN-LucN-VDNHPMLMNLLKDN-(GGGGS)×3-LBD(R274L)-LucC biosensor to 1α,25(OH)₂D₃.
Figure 16 depicts variation in light emission after adding 1,25D3 (1α,25(OH)₂D₃) and NS-4 to double molecular type biosensor

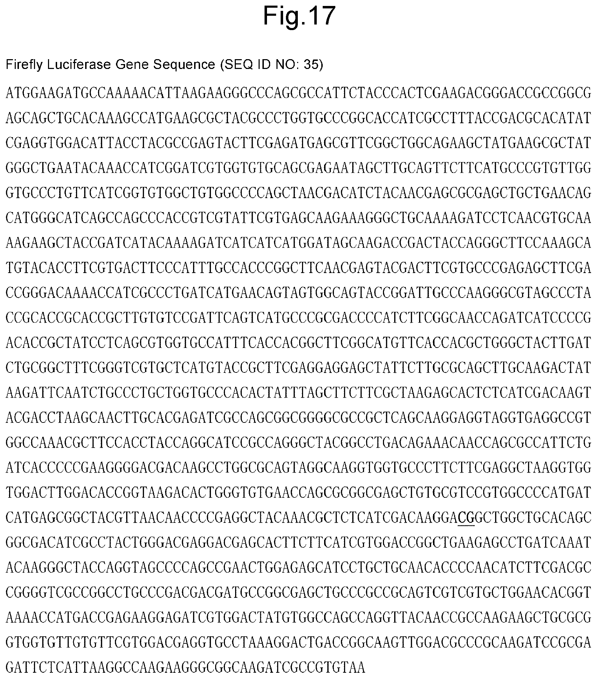

Figure 17 depicts firefly luciferase genes sequence (SEQ ID NO: 35), cleavage site between N terminal domain and C terminal domain in the underlined region.

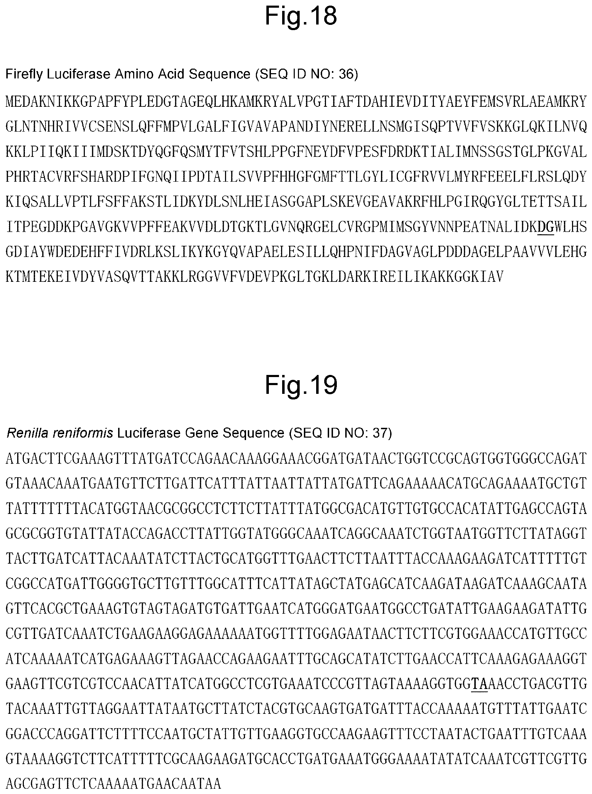

Figure 18 depicts cleavage site of the firefly luciferase amino acid sequence (SEQ ID NO: 36), underlined with N terminal domain and C terminal domain.
Figure 19 depicts *Renilla reniformis* luciferase (SEQ ID NO: 37) gene sequence, underlined cleavage site between N terminal domain and C terminal domain.

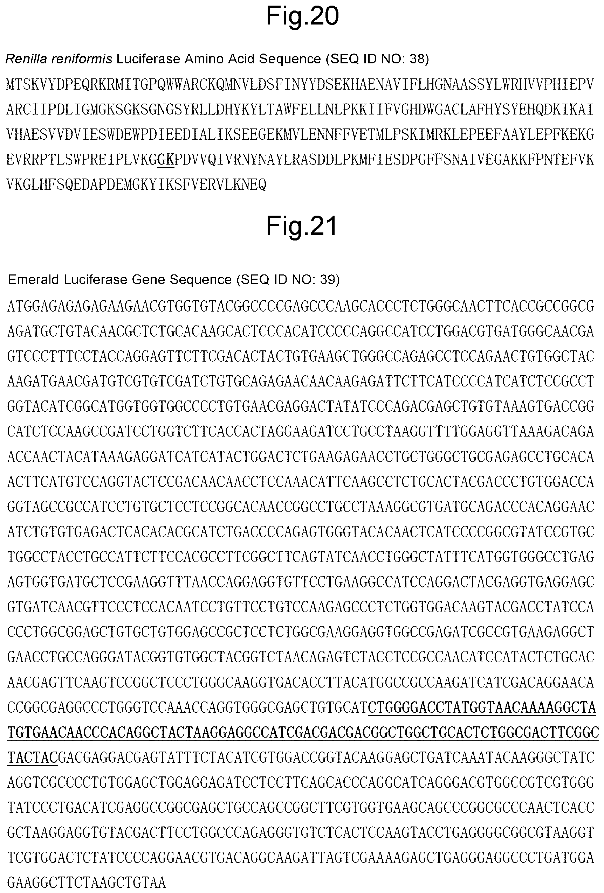

Figure 20 depicts *Renilla reniformis* luciferase amino acid sequence (SEQ ID NO: 38), underlined with N terminal domain and C terminal domain (cleavage site).
Figure 21 depicts Emerald luciferase (SEQ ID NO: 39), cleavage candidate site of N terminal domain and C terminal domain (n=388×3 (=1164) to 422×3 (=1266)) underlined.
Figure 22 depicts Emerald luciferase amino acid sequence (SEQ ID NO: 40), underlined by cleavage candidate site of N terminal domain and C terminal domain (n=388 to 422).
Figure 23 depicts human vitamin D receptor base sequence (SEQ ID NO: 41), underlined: vitamin D binding domain.
Figure 24 depicts human vitamin D receptor amino acid sequence (SEQ ID NO: 42), underlined: vitamin D binding domain.
Figure 25 depicts mouse vitamin D receptor base sequence (SEQ ID NO: 43).
Figure 26 depicts mouse vitamin D receptor amino acid sequence (SEQ ID NO: 44), underlined: vitamin D binding domain.
Figure 27 depicts rat vitamin D receptor base sequence (SEQ ID NO: 45).
Figure 28 depicts rat vitamin D receptor amino acid sequence (SEQ ID NO: 46), underlined: vitamin D binding domain.
Figure 29 depicts monkey vitamin D receptor amino acid sequence (SEQ ID NO: 47), underlined: vitamin D binding domain.
Figure 30 depicts dog vitamin D receptor amino acid sequence (SEQ ID NO: 48), underlined: vitamin D binding domain.
Figure 31 depicts cattle vitamin D receptor amino acid sequence (SEQ ID NO: 49), underlined: vitamin D binding domain.
Figure 32-1 depicts structure of NanoLuciferase type biosensor.
Figure 32-2 depicts structure of NanoLuciferase type biosensor.
Figure 33 depicts light emission principle of single molecular type NanoLuc-type biosensor.
Figure 34 depicts Light emission principle of double molecular type NanoLuc-type biosensor.
Figure 35 depicts selection results of double molecular type NanoLuciferase biosensor.
Figure 36 depicts (GGGGS)×3 performance comparison results of biosensor with x3 insertions.
Figure 37 depicts affinity of LXXLL sequence comparison results.
Figure 38 depicts optimization of LBDs and comparison results of R274L type biosensor.
Figure 39 depicts comparison results of single molecular type NanoLuciferase-type biosensor.
Figure 40 depicts comparison results of double molecular type and single molecular type NanoLuciferase-type biosensor in *in vitro* system.
Figure 41 depicts 25(OH)D₃ concentration dependency and time course test results.
Figure 42 depicts a test of 25(OH)D₃ detection limit of double molecular type N29+ N31 biosensor.
Figure 43 depicts affinity test results of calcitroic acid.
Figure 44 depicts normal phase HPLC-charts of rat blood plasma extract and the results of the fractions collected.
Figure 45 depicts calculation results of 25(OH)D₃ and 1α,25(OH)₂D₃ in blood plasma.

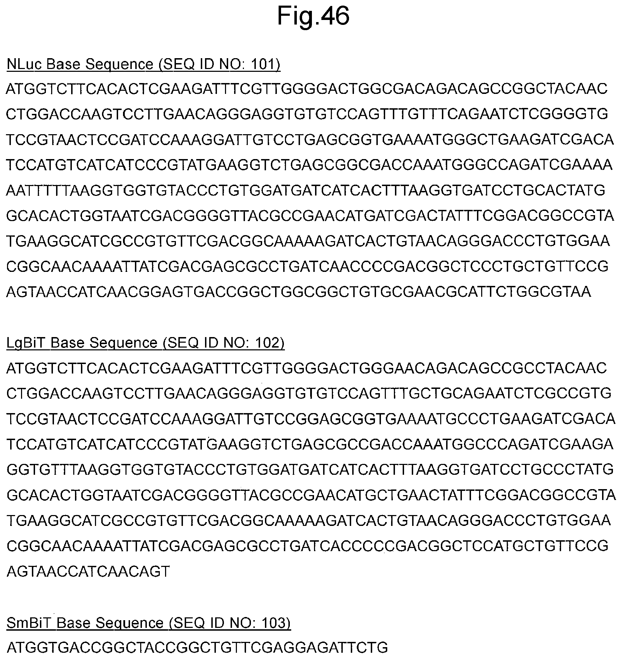

Figure 46 depicts NLuc base sequence (SEQ ID NO: 101), LgBiT base sequence (SEQ ID NO: 102), and SmBiT base sequence (SEQ ID NO: 103)
Figure 47 depicts NLuc amino sequence (SEQ ID NO: 104), LgBiT amino sequence (SEQ ID NO: 105), and SmBiT amino sequence (SEQ ID NO: 106)
Figure 48 depicts *Oplophorus gracilirostris* (deep-sea shrimp) luciferase gene sequence (SEQ ID NO: 107), and amino acid sequence (SEQ ID NO: 108)

### EMBODIMENTS OF THE INVENTION

### <Fusion protein>

The present invention relates to a fused protein of any one of (1) to (3) below:
(1) a fused protein comprising in random order N terminal domain of luciferase (hereinafter referred to as LucN), at least one vitamin D binding domain of vitamin D receptor (hereinafter referred to as VDR) and C terminal domain of luciferase (hereinafter referred to as LucC), and further comprising in random order one or both of LX¹X²LL sequences, wherein X¹ is M, W, T, S, E, V, R, Q, K, L or H and X² is N, T, S, G, E, R, Q, Y or K, and a linker sequence,
(2) a fused protein comprising in random order LucN or LucC and LX¹X²LL sequence, wherein X¹ is M, W, T, S, E, V, R, Q, K, L or H and X² is N, T, S, G, E, R, Q, Y or K, wherein the fused protein may comprise further in random order a linker sequence,
(3) a fused protein comprising in random order at least one vitamin D binding domain of VDR and LucC or LucN, wherein the fused protein may comprise further in random order a linker sequence.

Luciferase is an enzyme oxidizing substance (generically referred to as luciferin) as substrate, and releases a part of the energy generated in the oxidation reaction as light emission, and various enzymes are known depending on its origin. In the present invention, various types of luciferase can be used without any particular restriction as long as it is an enzyme that oxidizes substance to be substrate to generate luciferin. Luciferase may include, for example, a luciferase selected from the group consisting of firefly luciferase, a *Pyrophorus plagiophthalamus* luciferase, an Emerald luciferase, a *Renilla reniformis* luciferase, a *Cypridina noctiluca* luciferase, and *Gaussia princess* luciferase, prawns *Oplophorus gracilirostris* (deep sea prawns) luciferase and a variant of *Oplophorus gracilirostris* luciferase. A substrate of firefly luciferase and *Pyrophorus plagiophthalamus* luciferase is firefly luciferin, and a substrate of *Renilla reniformis* luciferase, *Cypridina noctiluca luciferase, Gaussia* luciferase and *Oplophorus gracilirostris* luciferase is coelenterazine. Substrate of variant of *Oplophorus gracilirostris* luciferase is furimazine.

In a fused protein of the present invention, luciferase is divided into N terminal domain LucN and C terminal domain LucC. In fused protein (1), LX¹X²LL sequences, linker sequence, and at least one vitamin D binding domain of VDRs are included between LucN and LucC. Fused protein (2) is a fused protein comprising at least LucN or LucC and a LX¹X²LL sequence. Fused protein (3) is a fused protein comprising at least one vitamin D binding domain of VDR and at least LucC or LucN.

The site of cleavage of luciferase into N terminal domain and C terminal domain is determined by considering that VDR agonist binds to VDR vitamin D binding domain as a binding substance, followed by interaction with LX¹X²LL sequence, allowing luciferase N terminal domain and C terminal domain to be reconstituted to indicate luciferase enzyme activity.

If luciferase is firefly luciferase, the amino acid sequence is denoted by SEQ ID NO: 36 of Sequence Listing (base sequence is denoted by SEQ ID NO: 35 of Sequence Listing), N terminal domain has amino acid sequence numbers 1 through 415, and C terminal domain has amino acid sequence numbers 416 through 550. When N terminal domain has an amino acid sequence of 1 to 415 and C terminal domain has an amino acid sequence of 416 to 550, then a binding substance binds to vitamin D binding domain of VDR, followed by interaction with LX¹X²LL sequence, luciferase N terminal domain and C terminal domain are reconstituted to indicate luciferase enzyme activity. However, unless cleavage site of N terminal domain and C terminal domain is between 415 and 416 of amino acid sequence but if cleavage site is between 415 and 500, the same function can be achieved. For example, in the following reference document A, cleavage site of the N-terminal part and the C-terminal part of luciferase is set to various positions, and light emission capability is verified. It has been shown that light emission capability is maintained by cleavage at sites other than 415 and 416. In reference document A, activity is detected not only between 415 and 416 but also between 420 and 421, 437 and 438, 445 and 446, 455 and 456, 475 and 476, and 500 and 501.

### Reference document A: Firefly Luciferase Enzyme Fragment Complementation for Imaging in Cells and Living Animals.Anal.Chem.2005, 77, 1295-1302

If luciferase is *Renilla reniformis* luciferase, the amino acid sequence is denoted by SEQ ID NO: 38 of Sequence Listing (base sequence is denoted by SEQ ID NO: 37 of Sequence Listing), N terminal domain has amino acid sequence numbers 1 through 229, and C terminal domain has amino acid sequence numbers 230 through 311. However, in the case of *Renilla reniformis* luciferase, as in the case of firefly luciferase, cleavage site between N terminal domain and C terminal domain is not limited to between 229 and 230, and the cleavage can be at other sites.

If luciferase is Emerald luciferase, then amino acid sequence is denoted by SEQ ID NO: 40 of Sequence Listing (base sequence is denoted by SEQ ID NO: 39 of Sequence Listing), N terminal domain has amino acid sequence numbers 1 to n, C terminal domain has amino acid sequence numbers n+1 to 542, and n is any integer from 388 to 422. The n that becomes segmentation site is any of 388 to 422. In fusion proteins of Emerald luciferase in which N terminal domain and C terminal domain are cleaved at either of these sites, when the binding substance binds to vitamin D binding domain of VDR, followed by interaction with LX¹X²LL sequence, N terminal domain and C terminal domain of luciferase are reconstructed to indicate enzyme activity of luciferase.

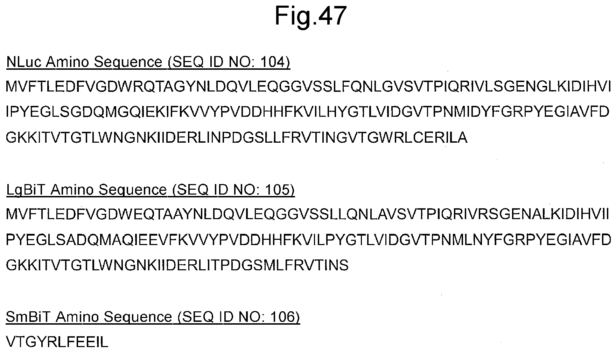

When the luciferase is a variant (NLuc) of *Oplophorus gracilirostris* luciferase, NLuc has the amino acid sequence shown in SEQ ID NO: 104 of the Sequence Listing, the N-terminal domain of NLuc has the amino acid sequence shown in SEQ ID NO: 105 of the Sequence Listing, and the C-terminal domain of NLuc has the amino acid sequence shown in SEQ ID NO: 106 of the Sequence Listing. Each sequence is also shown in Fig. 47. N terminal domain of NLuc with amino acid sequence indicated by SEQ ID NO: 105 of Sequence Listing and C terminal domain of NLuc with amino acid sequence indicated by SEQ ID NO: 106 of Sequence Listing are commercially available from Promega as LgBiT and SmBiT, respectively. LgBiT is a large fragment of NLuc, Large BiT (LgBiT) [18 kDa], and SmBiT is a small peptide fragment of NLuc (11 amino acids), Small BiT (SmBiT). LgBiT and SmBiT are expressed as fused protein with target proteins, respectively, and when interaction occurs, LgBiT and SmBiT are reconstituted and light emission is restored. NLuc is about 80-240 times higher in light intensity than firefly luciferase (FLuc). The following papers can be referred to for NLuc. M.P. Hall, J. Unch, B.F. Binkowski, M.P. Valley, B.L. Butler, M.G. Wood, P. Otto, K. Zimmerman, G. Vidugiris, T. Machleidt, M.B. Robers, H.A. Benink, C.T. Eggers, M.R. Slater, P.L. Meisenheimer, D.H. Klaubert, F. Fan, L.P. Encell, K.V. Wood, Engineered luciferase reporter from a deep sea shrimp utilizing a novel imidazopyrazinone substrate, ACS Chem Biol, 7 (2012) 1848-1857.

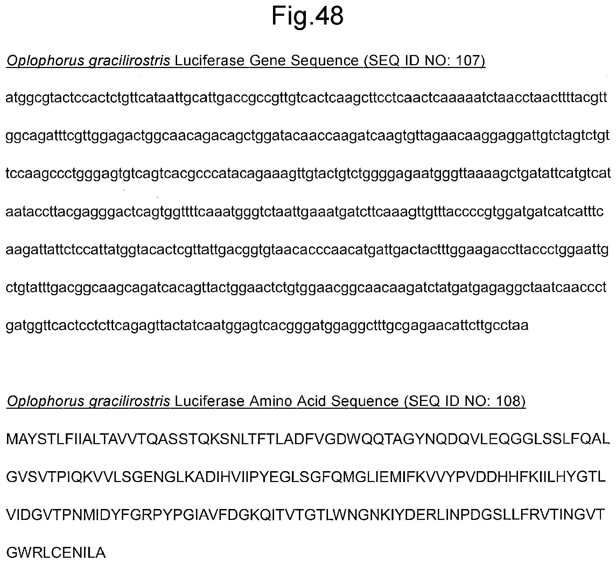

If luciferase is *Oplophorus gracilirostris* luciferase (wild type), it has amino acid sequence indicated by SEQ ID NO: 108 of Sequence Listing (also shown in Fig. 48), and N terminal domain and C terminal domain can be determined as appropriate by referring to NLuc case. For *Oplophorus gracilirostris* luciferase (wild type), see S. Inouye, K. Watanabe, H. Nakamura, O. Shimomura, Secretional luciferase of the luminous shrimp Oplophorus gracilirostris: cDNA cloning of a novel imidazopyrazinone luciferase (1), FEBS Lett, 481 (2000) 19-25.

Fused protein (1) is a fused protein comprising LucN, at least one VDR (which may hereinafter be referred to simply as VDR) and LucC in random order, and further comprising one or both of a LX¹X²LL sequence (which may hereinafter be referred to as LXXLL sequence) and a linker sequence in random order. The order of LucN, VDR, and LucC is preferably N-terminal to LucN-VDR-LucC, but may be LucC-VDR-LucN, VDR-LucC-LucN, and VDR-LucN-LucC. Any one or both of LXXLL sequence and the linker sequence may be inserted between any of the above LucN, VDR, and LucC, or added to the N-terminal and/or C-terminal sides. Examples of LucN-VDR-LucC include preferably LucN-LXXLL-VDR-LucC, LucN-LXXLL-VDR-LucC, and LucN-LXXLL-linker VDR-LucC, and examples of VDR-LucC-LucN include preferably VDR-LucC-LucN-LXXLL, VDR-LucC-linker LucN-LXXLL and the like.

Fused protein (2) is a fused protein which comprises a LucN or LucC and a LXXLL sequence in random order and may further comprise a linker sequence in random order. Preferably, from the N-terminal end, it can be LucN-LXXLL, LucN-linker-LXXLL, LXXLL-LucN, LucC-LXXLL, LXXLL-LucC or the like.

Fused protein (3) is a fused protein comprising at least one VDR and LucC or LucN in in random order, wherein fused protein may further comprise linker sequence in random order. Preferably, from the N-terminal side, it can be VDR-LucC, LucN-VDR, LucC-VDR, LucN-VDR, VDR-linker-LucC, and the like.

LX¹X²LL sequences that fused protein (1) and (2) can have are specific sequences called LXXLL motifs present in transcriptional coactivator. LXXLL motifs are ligand binding domain-binding sequences found in coactivators that bind to ligand bound receptors and promote transcription activity. X¹ in the sequence is M, W, T, S, E, V, R, Q, K, L, or H, and X² is N, T, S, G, E, R, Q, Y, or K. Examples of amino acid sequence containing LXXLL motifs have been reported, for example, NHPMLMNLLKDN, LSETHPLLWTLLSSTEGDSM and LTEMHPILTSLLQNGVDHV (non-patent document 5). In addition, examples of amino acid sequence containing LXXLL motifs can also be exemplified by LXXLL motifs described in reference document B-D below. LXXLL motifs in which X¹ in the sequence is S, E, V, R, Q, K, L or H, or X² is G, E, R, Q, Y or K are LXXLL motifs are described in Reference documents B-D below. LXXLL sequence is a sequence derived from a transcription activity conversion factor or an artificially synthesized sequence, and affinity to LBD differs for each sequence, so that it can be appropriately selected in view of light intensity obtained by a fused protein including each LXXLL sequence.

Reference document B: C. Rachez, M. Gamble, C.P. Chang, G.B. Atkins, M.A. Lazar, L.P. Freedman, The DRIP complex and SRC-1/p160 coactivators share similar nuclear receptor binding determinants but constitute functionally distinct complexes, Mol Cell Biol, 20 (2000) 2718-2726.

Reference document C: C. Chang, J.D. Norris, H. Gron, L.A. Paige, P.T. Hamilton, D.J. Kenan, D. Fowlkes, D.P. McDonnell, Dissection of the LXXLL nuclear receptor-coactivator interaction motif using combinatorial peptide libraries: discovery of peptide antagonists of estrogen receptors alpha and beta, Mol Cell Biol, 19 (1999) 8226-8239. Reference document D: B. He, E.M. Wilson, Electrostatic modulation in steroid receptor recruitment of LXXLL and FXXLF motifs, Mol Cell Biol, 23 (2003) 2135-2150.

Linker sequence which can be used in fused protein (1) to (3) can be exemplified by, for example, a GGGGS sequence and an EAAAK sequence. GGGGS sequence is known as linker sequence, which is high in flexibility and softness, and EAAAK sequence is known to be low in flexibility and softness (Reference document E: G. Li, Z. Huang, C. Zhang, B. J. Dong, R. H. Guo, H. W. Yue, L. T. Yan, X. H. Xing, Construction of a linker library with widely controllable flexibility for fusion protein design, Applied microbiology and biotechnology, 100 (2016) 215-225.). When used as a linker sequence in the present invention, GGGGS and EAAAK sequences can be repeating sequence 2 to 6 times each or a combination of GGGGS and EAAAK (in any order). In the present invention, it is preferably (GGGGS)×3.

The VDR of fused protein (1) and (3) resides in the nucleus of target cell, binds specifically to active form of vitamin D3 (1α,25(OH)₂D₃), induces gene expression of vitamin D dependent proteins, and plays key roles in the regulation of bone metabolism, cytodifferentiation and proliferation, and immune function. VDR is inherent in various mammals, for example, amino acid sequences and base sequences of VDR in human, in mouse, in rat, in monkey, and in rabbits are known.

Human VDR has an amino acid sequence denoted by SEQ ID NO: 42 of Sequence Listing, vitamin D binding domain of which has amino acid sequence of numbers 121 to 427.

Mouse VDR has an amino acid sequence denoted by SEQ ID NO: 44 of Sequence Listing, vitamin D binding domain of which has an amino acid sequence of numbers 121 to 422.

Rat VDR has an amino acid sequence denoted by SEQ ID NO: 46 of Sequence Listing, vitamin D binding domain of which has an amino acid sequence of numbers 121 to 423.

Monkey VDR has an amino acid sequence denoted by SEQ ID NO: 47 of Sequence Listing, vitamin D binding domain of which has an amino acid sequence of numbers 121 to 434.

Dog VDR has an amino acid sequence denoted by SEQ ID NO: 48 of Sequence Listing, vitamin D binding domain of which has amino acid sequence of numbers 121 to 427.

Cattle VDR has an amino acid sequence denoted by SEQ ID NO: 49 of Sequence Listing, vitamin D binding domain of which has amino acid sequence of numbers 121 to 426.

Amino acid sequence of vitamin D binding domain may also have a mutation. For example, it has been reported that the mutation found in rickets patient is present in vitamin D binding region and reduces affinity of active form of vitamin D and receptors. Previous reports have reported that mutation of R274L present in vitamin D binding region is about 1/1000 of affinity to vitamin D receptor of healthy subject (non-patent document 5).

Mutation found in rickets patient is present in DNA binding region or vitamin D binding region, but because this study focuses on vitamin D binding region, mutation in vitamin D binding region is presented below. Vitamin D binding domain of human is shown in SEQ ID NO: 42, whose vitamin D binding domain has amino acid sequence of numbers 121 to 427.

T146I, R158C, C190W, L227P, L233S, ΔK246, F251C, Q259P, Q259E, L263R, I268T, R274L, R274H, W286R, H305Q, I314S, G319V, E329K, V346M, R391C, R391S, E420K, E420A

These mutations are mutations seen in rickets patient, but they are divided into three categories: mutations, which reduce affinity with active form of vitamin D, mutations, which have normal affinity with active form of vitamin D but inhibit interaction between vitamin D receptor and cofactors (RXR protein and other transfer factors), and mutations, which have unknown mechanism. For mutations whose mechanism is unknown, affinity with active form of vitamin D can be assessed by using biosensor with each mutation.

Reference documents 1-7, written for mutation found in rickets patient, are shown below. Reference document 1: The Unique Tryptophan Residue of the Vitamin D Receptor Is Critical for Ligand Binding and Transcriptional Activation. JOURNAL OF BONE AND MINERAL RESEARCH Volume 16, Number 1, 2001 39-45

Reference document 2: Novel Compound Heterozygous Mutations in the Vitamin D Receptor Gene in a Korean Girl with Hereditary Vitamin D Resistant Rickets. J Korean Med Sci 2011; 26: 1111-1114

Reference document 3: Crystal Structures of Hereditary Vitamin D-Resistant Rickets-Associated Vitamin D Receptor Mutants R270L and W282R Bound to 1,25-Dihydroxyvitamin D3 and Synthetic Ligands. J. Med.Chem.2013, 56, 6745-6760 Reference document 4: Mutations in the vitamin D receptor and hereditary vitamin D-resistant rickets. BoneKEy Reports 3, Article number: 510.(2014) 1-11

Reference document 5: Crystal structure of hereditary vitamin D-resistant rickets-Associated mutant H305Q of vitamin D nuclear receptor bound to its natural ligand. Journal of Steroid Biochemistry & Molecular Biology 121 (2010) 84-87

Reference document 6: Mutations in the Vitamin D Receptor Gene in Four Patients with Hereditary 1,25-Dihydroxyvitamin D-Resistant Rickets. Arq Bras Endocrinol Metab 2008;52/8 1244-1251

Reference document 7: The Vitamin D Receptor and the Syndrome of Hereditary 1,25-Dihydroxyvitamin D-Resistant Rickets. Endocrine Reviews 20(2): 156-188 1999

In addition, for mutation in vitamin D binding region, mutation in vitamin D binding region of other animals as compared to that of human above is shown in the following table. In the table, hatched lines for C190W mean that C(Cys) is not stored.

**[Table 1]**

| | Mutation position of the amino acid in LBD | | | | | | |
|---|---|---|---|---|---|---|---|
| Human | R158C | C190W | L227P | L233S | ΔK246 | F251C | Q259P |
| Mouse | 158 | | 227 | 233 | 241 | 246 | 254 |
| Rat | 158 | | 227 | 233 | 242 | 247 | 255 |
| Chicken | 181 | | 251 | 257 | 270 | 275 | 283 |
| Dog | 158 | | 227 | 233 | 246 | 251 | 259 |
| Zebrafish | 190 | | 255 | 261 | 274 | 279 | 287 |
| Chimpanzee | 158 | 190 | | | | | 228 |
| Cattle | 158 | 190 | | 233 | 244 | 249 | 257 |
| Frog | 158 | | 227 | 233 | 239 | 244 | 252 |
| Monkey | 165 | | 234 | 240 | 253 | 258 | 266 |
| | | | | | | | |

| | Mutation position of the amino acid in LBD | | | | | | |
|---|---|---|---|---|---|---|---|
| Human | Q259E | L263R | R274L | R274H | W286R | H305Q | I314S |
| Mouse | 254 | 258 | 269 | 269 | 281 | 300 | 309 |
| Rat | 255 | 259 | 270 | 270 | 282 | 301 | 310 |
| Chicken | 283 | 287 | 298 | 298 | 310 | 329 | |
| Dog | 259 | 263 | 274 | 274 | 286 | 305 | 314 |
| Zebrafish | 287 | 291 | 302 | 302 | 314 | 333 | |
| Chimpanzee | 228 | 232 | 243 | 243 | 255 | 274 | 283 |
| Cattle | 257 | 261 | 272 | 272 | 284 | 304 | 313 |
| Frog | 252 | 256 | 267 | 267 | 279 | 298 | |
| Monkey | 266 | 270 | 281 | 281 | 293 | 312 | 321 |
| | | | | | | | |

| | Mutation position of the amino acid in LBD | | | | | | |
|---|---|---|---|---|---|---|---|
| Human | G319V | E329K | V346M | R391C | E420K | E420A | |
| Mouse | 314 | 324 | 331 | 386 | 415 | 415 | |
| Rat | 315 | 325 | 332 | 387 | 416 | 416 | |
| Chicken | 343 | 353 | 360 | 415 | 444 | 444 | |
| Dog | 319 | 329 | 346 | 391 | 420 | 420 | |
| Zebrafish | 347 | 357 | 364 | 417 | 446 | 446 | |
| Chimpanzee | 288 | 298 | 305 | | 389 | 389 | |
| Cattle | 318 | 328 | 335 | 390 | 419 | 419 | |
| Frog | 312 | 322 | 329 | 384 | 413 | 413 | |
| Monkey | 326 | 336 | 343 | 398 | 427 | 427 | |

Fusion protein (1) of the present invention can be a fusion protein with one LBD represented by LucN for the N-end domain of luciferase, LucC for the C-end domain of luciferase, LBD for the vitamin D binding domain of VDR, LXXLL for LX¹X²LL sequence, GGGGS for GGGGS sequence (where GGGGS sequence is two to six consecutive: (GGGGS)×N (N=2-6)), simply denoted as GGGGS below), and may be the fusion protein with one LBD represented by, for example, LucN-LBD-LucC, LucN-GGGGS-LBD-LucC, LucN-LXXLL-LBD-LucC, LucN-LXXLL-GGGGS-LBD-LucC, LucC-LBD-LucN, LucC-GGGGS-LBD-LucN, LucC-LXXLL-LBD-LucN, LucC-LXXLL-GGGGS-LBD-LucN, LBD-LucC-LucN-LXXLL, LBD-LucC-GGGGS-LucN-LXXLL, or LucN-LXXLL-LBD-LucC.

Fused protein (2) of the present invention may be, for example, a fused protein represented by LucC-LXXLL, LucN-LXXLL, LXXLL-LucN, LXXLL-LucC, LucC-GGGGS-LXXLL, LucN-GGGGS-LXXLL, LXXLL-GGGGS-LucN, or LXXLL-GGGGS-LucC.

Fused protein (3) may be a fused protein having one LBD represented by LBD-LucN, LBD-LucC, LucC-LBD, LucN-LBD, LBD-GGGGS-LucN, LBD-GGGGS-LucC, LucC-GGGGS-LBD, or LucN-GGGGS-LBD.

In addition, fused protein (1) and (3) of the present invention may have two vitamin D binding domains. Fused protein (1) of the present invention may also be a structure with two LBDs between LucN-LXXLL-GGGGS- and LucC or between LucC-LXXLL-GGGGS and LucN, for example, LucN-LXXLL-GGGGS-LBD-LBD-LucC, LucC-LXXLL-GGGGS-LBD-LBD-LucN. In addition, it maybe a structure in which LBDs are present at either end, e.g., LucN-LXXLL-GGGGS-LBD-LucC-LBD, LucC-LXXLL-GGGGS-LBD-LucN-LBD, LBD-LucC-LXXLL-GGGGS-LBD-LucN, and LBD-LucN-LXXLL-GGGGS-LBD-LucC. Fused protein (3) of the present invention may be, for example, LBD-LBD-LucC, LBD-LBD-LucN, LBD-LucC-LBD, and LBD-LucN-LBD. Further, when two or more LBDs are linked, LBDs of the same type of sequence can be linked, and LBDs of different types of sequence can be linked. Linkage of LBDs of the same or different type of sequence may result in a higher reduction in light emission, no change in light emission, or conversely, an increase in light emission after active form of vitamin D3 binding.

In the examples of fused protein (1) to (3) above, the order of the respective sequences is not intended to be limited thereto. In addition, there may be direct linkage between amino acid sequences or there may be intervention of any of 1-5 amino acids, e.g., VD, GS, VE, RS, EF, etc. VDs, GSs, VEs, RSs, EFs, etc. which are sequences included due to the sequence of restriction enzyme or linker used in cloning.

When fused protein (1) and (2) of the present invention have a LX¹X²LL sequence, LX¹X²LL sequence may further have an insertion sequence A on its N-terminal side and an insertion sequence B on its C-terminal side. Insertion sequence A may have an amino acid number of 1 to 20, preferably 3 to 10, more preferably 3 to 8. Insertion sequence B may have an amino acid number of 1 to 20, preferably 3 to 10, more preferably 3 to 7. Sequences having these two insertion sequences, preferred sequences and more preferred sequences, respectively, can be written as follows:
(A) ₁₋₂₀ₐₐ-LX¹X² LL(B)₁₋₂₀ₐₐ
(A) ₃₋₀₀ₐₐ-LX¹X²LL(B)₃₋₁₀ₐₐ
(A) ₃₋₈ₐₐ-LX¹X²LL(B)₃₋₇ₐₐ
(A) indicates insertion sequence A, and 1-20aa indicates that amino acid number of insertion sequence A is 1-20. (B) indicates insertion sequence B, and 1-20 aa indicates that amino acid number of insertion sequence B is 1-20.

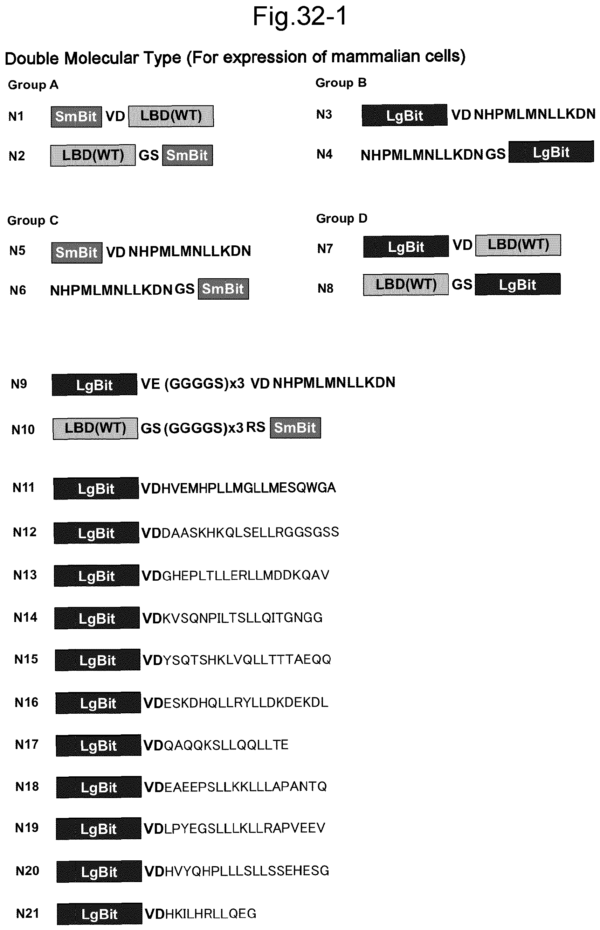

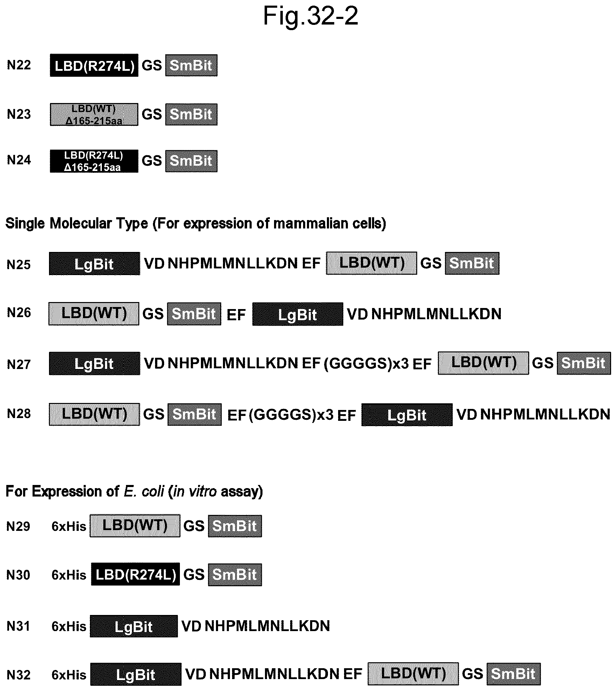

The amino acids in insertion sequences A and B are optional, but insertion sequence A can be, for example, "NHPM" in "NHPMLMNLLKDN" (SEQ ID NO: 50), exemplified above in non-patent document 5, "LSETHPL" in "LSETHPLLWTLLSSTEGDSM" (SEQ ID NO: 51), and "LTEMHPI" in "LTEMHPILTSLLQNGVDHV" (SEQ ID NO: 52). Further, insertion sequence A can be exemplified by sequences located between LX¹X²LL sequences and LgBit contained in N11-N21, N25-N28, N31, and N32 shown in Fig. 32. LX¹X²LL sequences having these insertion sequence A are described in Reference documents B-D. However, the two amino acids VD immediately flanking LgBit are amino acids other than insertion sequence A and are sequences contained due to restriction enzyme or linker used to clone LgBiT and LXXLL.

Amino acids in the insertion sequence are optional, but insertion sequence B can be, for example, "KDN" in "NHPMLMNLLKDN" exemplified in non-patent document 5 above, "SSTEGDSM" in "LSETHPLLWTLLSSTEGDSM" and "QNGVDHV" in "LTEMHPILTSLLQNGVDHV". Further, insertion sequence B can exemplified by a sequence located on the side (C-terminal side) distant from LgBit of LX¹X²LL sequence included in, for example, N11 to N21 shown in Fig. 32. For example, in N11, it is "MESQWGA". LX¹X²LL sequences having these insertion sequence B are described in Reference documents B-D.

LX¹X²LL sequences with insertion sequences A and B (LX¹X²LL sequence-related sequences) described in N11-N21 are shown below. HVEMHPLLMGLLMESQWGA (N11, SEQ ID NO: 109), DAASKHKQLSELLRGGSGSS (N12, SEQ ID NO: 110), GHEPLTLLERLLMDDKQAV (N13, SEQ ID NO: 111), KVSQNPILTSLLQITGNGG (N14, SEQ ID NO: 112), YSQTSHKLVQLLTTTAEQQ (N15, SEQ ID NO: 113), ESKDHQLLRYLLDKDEKDL (N16, SEQ ID NO: 114), QAQQKSLLQQLLTE (N17, SEQ ID NO: 115), EAEEPSLLKKLLLAPANTQ (N18, SEQ ID NO: 116), LPYEGSLLLKLLRAPVEEV (N19, SEQ ID NO: 117), HVYQHPLLLSLLSSEHESG (N20, SEQ ID NO: 118), HKILHRLLQEG (N21, SEQ ID NO: 119)

When LX¹X²LL sequence having insertion sequences A and B is denoted (A) LXXLL (B), fused protein (1) is denoted by LucN--(A) LXXLL (B)--LBD-LucC, LucN (A) LXXLL (B)--GGGGS-LBD-LucC, LucC (A)--LXXLL (B)--LBD-LucN, LucC (A) LXXLL (B)--GGGGS-LBD-LucN, LBD-LucC-LucN (A) LXXLL (B)--LBD-LucC-GGGGS-LucN (A)--LXXLL (B), or LXXLL (B)--LucN--LBD-LucC, for example. Fused protein (2) is represented, for example, by LucC-(A) LXXLL (B), LucN-(A) LXXLL (B), (A) LXXLL (B)-LucN, (A) LXXLL (B)-LucC, LucC-GGGGS (A) LXXLL (B), (LucN-GGGGS-(A) LXXLL (B), (A) LXXLL (B)-GGGGS-LucN, (A) LXXLL (B)-GGGGS-LucC.

In addition, any fused proteins (1)-(3) of the present invention may have n (e.g., n=3-10) His tag on the N-terminal side.

The present invention encompasses DNAs encoding amino acid sequence of any one of fused protein (1) to (3) of the above the present invention. DNAs encoding amino acid sequence of fused protein (1) to (3) of the present invention can be prepared by joining by any of DNA encoding amino acid sequence of N terminal domain of luciferase, DNA encoding LX¹X²LL sequence, DNA encoding linker sequence, DNA encoding amino acid sequence of vitamin D binding domain, and DNA encoding amino acid sequence of C terminal domain of luciferase, or insertion sequence A and B, as required, in a conventional manner.

The present invention encompasses a plasmid which contains in any of vector DNAs encoding amino acid sequence of any one of fused proteins (1) to (3) of the above the present invention. Preferably, a plasmid containing DNA encoding amino acid sequence of fused protein (1) of the present invention and a plasmid containing DNA encoding amino acid sequences of fused protein (2) and (3) of the present invention are included. Vectors constituting the present plasmid are optional, and can be appropriately selected according to a cell to be transformed by introducing the present plasmid. It is preferable that plasmid contains promotor, for example, SV40, CMV, CAG promotor, or the like, because it is preferable that fused protein of the present invention be expressed constitutively and highly in a cell.

Fused proteins (1) to (3) of the present invention can be prepared by expressing in a cell (transformant) introduced with a plasmid which contains the DNA encoding amino acid sequence of fused protein (1) to (3) of the present invention. The introduction of DNAs into a cell (transformation) and the expression of proteins in the transformants can be carried out as appropriate by conventional methods. Fused protein of the present invention can also be synthesized by *in vitro* synthetic methods using DNAs encoding amino acid sequence of any of fused proteins (1) to (3) of the present invention. Synthesis of proteins in *in vitro* can be carried out using commercially available kit, and commercially available kit can include, for example, TNT SP6 High-Yield Wheat GermProtein Expression System (Promega). However, it is not intended to be limited to this kit, and for example, TNT SP6 Coupled Wheat Germ Extract System (Promega) or TNT SP6 Coupled Reticulocyte Lysate System (Promega) can be used as well.

A cell into which the plasmid of the present invention has been introduced can be used regardless of whether it is adhesive or adherent, as long as it can contain plasmid DNAs in cell. Specifically, any cells (NIH3T3 cell, a PC 12 cell, a HEK293 cell, a COS-7 cell, a CHO cell, a HeLa cell, a Sf-9 cell, a S2 insect) derived from mammals and insects can be used. In particular, a cell derived from mammals is preferable from the viewpoint that it is used for screening a substance which is permeable to cell membrane and has a binding property to VDR in mammals. Similarly, when used to screen a VDR-binding substance in another animal, it is preferable to use a cell in which plasmid has been introduced into a cell from the animal.

### <Screening method (intracellular)>

The present invention encompasses screening method in cell of substance that exhibits binding to VDR. Screening method of the present invention comprises: cultivation of a cell into which a plasmid of the present invention has been introduced in the presence of a substrate of a luciferase of which DNA is contained in the transformed cell, Mg²⁺, ATP, oxygen and a candidate substance; measuring light emission in cultivation and/or after cultivation; assessing the binding of said candidate substance to VDR based on light emission; and selecting a substance exhibiting binding property to VDR.

The plasmid of the present invention is a plasmid that contains DNA encoding amino acid sequence of any of fused proteins (1) to (3) of the present invention, more specifically, the fused protein is fused protein (1) or fused protein (2) and fused protein (3). However, if fused protein (2) contains LucN, then fused protein (3) contains LucC, and if fused protein (2) contains LucC, then fused protein (3) contains LucN. A cell into which a plasmid containing DNAs encoding amino acid sequence of these fused proteins have been introduced is used.

The cell into which the plasmid of the present invention has been introduced is cultivated in the coexistence of a substrate of luciferase of which DNA is contained in the transformed cell, Mg²⁺, ATP and oxygen, and a candidate substance. Substrate of luciferase of which DNA is contained in the transformed cell can be appropriately selected according to the type of luciferase. The substrate is firefly luciferin when luciferase is firefly luciferase *Pyrophorus plagiophthalamus* luciferase or Emerald luciferase. The substrate is coelenterazine when luciferase is *Renilla reniformis* luciferase, *Cypridina noctiluca* luciferase, or *Gaussia* luciferase.

The candidate substance is not limited and there is no limitation as long as it is a compound of which binding property to VDR is to be assessed. For example, a compound having steroid nucleus can be exemplified. In addition to compound with steroid nucleus, fatty acids, fat-soluble vitamins, and polyphenols can be also candidate substances. However, it is not intended to be limited to these substances.

Cultivation of cell can be appropriately determined according to the type of cell. When using mammal cell, cultivation can be achieved by seeding cells on petri dishes, etc. and incubating at 37°C in a commercial basal medium (MEM, DMEM, etc.) containing 5-10% serum-free medium or fetal bovine serum in the presence of 5%CO₂, according to conventional methods.

Light emission is measured during cultivation and/or after cultivation of cell. Based on the measured emission, the binding property of candidate substance to VDR is evaluated and a substance exhibiting binding property to VDR is selected. Light emission can be measured, for example, using a microplate reader. However, it is not intended to be limited to this, and a conventional light emission measurement method and device can be used as appropriate.

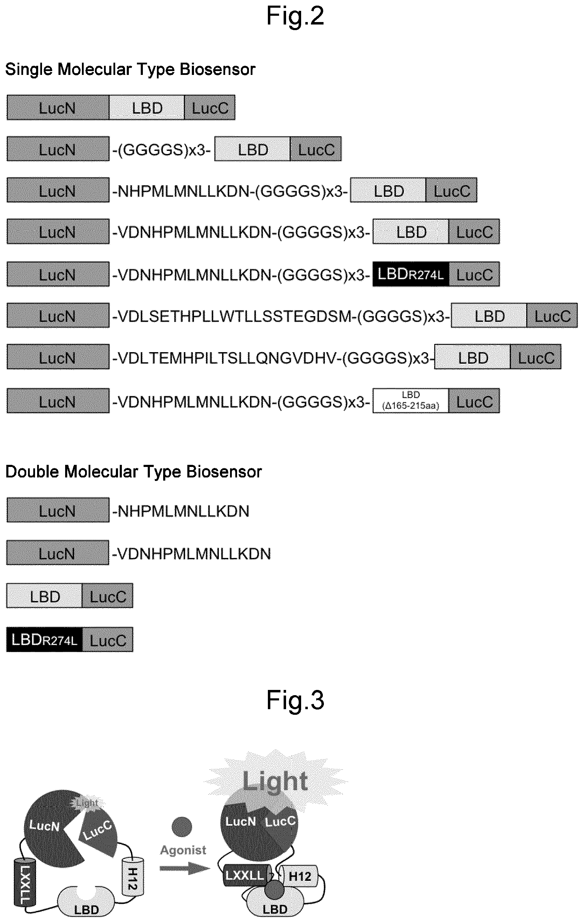

When the plasmid contains DNA encoding amino acid sequence of fused protein (1) of the present invention, the cell to which the plasmid of the present invention has been introduced expresses fused protein (1) of the present invention in the cell and this fused protein exhibits a luciferase activity. Because N terminal domain gets closer to C terminal domain in the presence of substance (agonist) with binding property to VDR, as shown in Fig. 3, and exhibits light emission in the presence of substrate of luciferase, Mg²⁺, ATP and oxygen.

When the plasmid contains DNAs encoding amino acid sequence of fused proteins (2) and (3) of the present invention, then fused proteins (2) and (3) of the present invention are expressed (when fused protein (2) contains LucN, fused protein (3) contains LucC, and when fused protein (2) contains LucC, fused cell (3) contains LucN), in these two fused proteins, N terminal domain gets closer to C terminal domain in the presence of substance (agonist) with binding property to VDR, exhibiting a luciferase activity and exhibiting light emission in the presence of substrate of luciferase, Mg²⁺, ATP and oxygen.

When agonist (active form of vitamin D or its derivatives) is administered to the cell expressing fused protein of the present invention, agonist is considered to bind to vitamin D binding region because light emission occurs. Thus, if there is a substance that results in light emission as a result of the screening, such a compound will be a candidate for agonist. In Examples, results for agonist with steroid nucleus are shown. However, even in a compound that does not have steroid nucleus, there may be a substance exhibiting binding property.

Looking at structure shown below, examples of compound with steroid nucleus are shown. Among these, as shown in Examples, VD3 and VD2 do not cause an increase in light emission, but only one OH group [25(OH)D3, 25(OH)D2] or two OH groups [1α, 25(OH)2D3, 1α,25(OH)2D2] cause a significant increase in light emission. Thus, the screening method of the present invention is likely to find a new VDR ligand because there is a case in which the structure is similar but light emission is not increased (not bound to vitamin D binding region), but conversely there is a case in which it is possible to bind even if the structure is quite different.

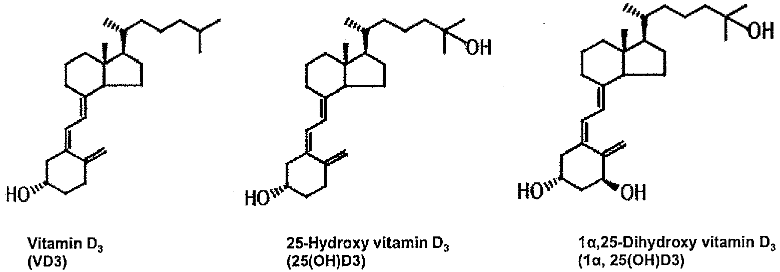

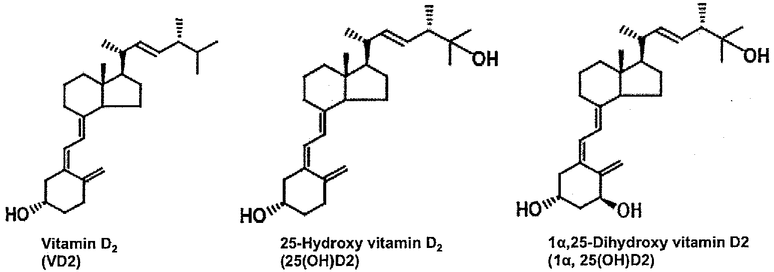

### <Screening method (in vitro)>

The present invention is a screening method of a substance that exhibits binding property to VDR, including *in vitro* screening method that does not use cell to screen. *In vitro* screening method of the present invention comprises subjecting fusion protein (1) or (2) and (3) of the present invention to interaction in the presence of a luciferase substrate, Mg²⁺, ATP and oxygen, and a candidate agent, measuring light emission during and/or after interaction, assessing the binding property of the candidate agent to VDR based on light emission, and selecting a substance that exhibits binding property to VDR.

*In vitro* screening method of the present invention can be carried out in the same manner as the intracellular screening methods of the present invention, except that the fusion protein of the present invention is used instead of cultivating the cells into which the plasmid of the present invention has been introduced, and the fusion protein is subjected to interaction. It should be noted that if fused protein (2) contains LucN, then fused protein (3) contains LucC, and if fused protein (2) contains LucC, then fused protein (3) contains LucN as well. The conditions under which the fused protein subjected to interaction can be appropriately selected from the conditions suitable for luciferase activity, depending on the type of luciferase contained in the fused protein. *In vitro* screening method with fused protein requires less time for assays because it does not require cultivation of cell.

Light emission is measured during and/or after the interaction of fused protein. Based on the measured light emission, binding of candidate substance to VDR is evaluated and a substance exhibiting binding to VDR is selected. Light emission can be measured, for example, using a microplate reader. However, it is not intended to be limited to this, and a conventional light emission measurement method and device can be used as appropriate.

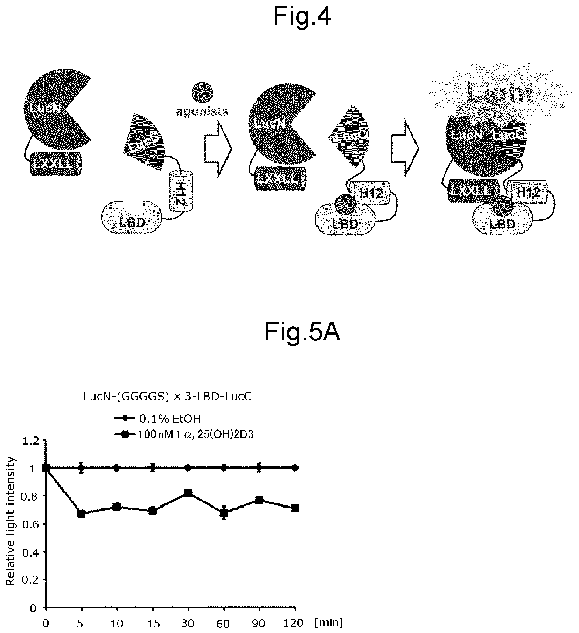

The fused protein of the present invention exhibits a luciferase activity in the presence of a substance (agonist) having binding properties to VDR, with close distances between N terminal domain and C terminal domain, and exhibits a light emission in the presence of substrate of luciferase, Mg²⁺, ATP and oxygen (reference Figs. 3 and 4). These mechanisms are substantially the same as with the case using cell described above.

In the intercellular screening method of the present invention, the DNA encoding amino acid sequence of vitamin D binding domain contained in plasmid introduced into transformed cell may be a DNA in which amino acid sequence of vitamin D binding domain has a mutation. In *in vitro* screening method of the present invention, an amino acid sequence of vitamin D binding domain contained in fused protein may have a mutation. A candidate substance which specifically binds to vitamin D binding domain having a mutation involved in disease can also be screened by expressing a fused protein having vitamin D binding domain having the mutation and measuring light emission based on the expression, from the relation between the mutation of amino acid sequence and disease.

For example, it has been reported that a mutation, found in rickets patient, is present in vitamin D binding region and reduces affinity of active form of vitamin D and receptors. Previous reports have shown that mutation of R274L present in vitamin D binding region is about 1/1000 of affinity of healthy subject to vitamin D receptor. If a compound exhibiting light emission is hit by the screening of the present invention using the biosensor with the mutation (LucC-LXXLL-GGGGS-LBD-LucN R274L), it can be interpreted that there is a compound binding to the receptor even if it has a mutation. It can be used as a compound screening to develop a therapeutic agent that works on rickets patient.

### EXAMPLES

The present invention is explained in more detail below on the basis of examples. However, the examples are illustrative of the present invention, and the present invention is not intended to be limited to the examples.

### EXAMPLE 1: Cloning of human VDR (hVDR) Genes

tRNA was extracted from THP-1 cell derived from human acute monocytic leukemias, and human VDR was amplified by PCR using, as template, cDNA synthesized by reverse transcription and primer 1 and 2 (SEQ ID NO: 1 and 2). PCR was performed under the following conditions: Reaction 1 (94°C, 2 minutes later, 98°C, 10 seconds, 56°C, 30 seconds, 68°C, 1 minute, 30 cycles; template DNA 10 ng, MgSO₄ 1.5 mM, dNTPs 0.2 mM, KOD-plus neo-DNA polymerase (TOYOBO) 0.2 U, 10 x PCR Buffer for KOD plus neo), primer 0.3 µM, final volume 10µL. Electrophoresis of 1 µl of PCR products with 1% agarose gel resulted in a specific amplification at the site of interest (approximately 1.3 kb) (hereinafter electrophoresis at 1% agarose gel is simply abbreviated as electrophoresis). After confirming the amplification of the fragments of interest by electrophoresis, the PCR product was cloned into pCR Blunt II-TOPO vectors according to the instructions in Zero blunt TOPO PCR Cloning Kit (Invitrogen), *E. coli* HST08 strain was transformed with calcium chloride method, and applied to LB agar medium (10 g of polypeptone, 5 g of yeast extract, 5 g of NaCl and 15 g of agar dissolved in 1 L of distilled water) containing 30µg/mL of kanamycin. Several of the obtained *E.coli* colony were selected, inoculated into 2 mL of LB liquid culture medium (containing 30 µg/mL of kanamycin), and shaking culture was performed at 37°C and 200 rpm for 16 hours. The plasmid was then extracted using QIAprep Spin Miniprep Kit (QIAGEN) and plasmid concentrations were measured using a 260 nm absorbance to yield template DNAs. Cycle sequencing reactions were performed using BigDye Terminator v3.1 Cycle Sequencing Kit (Applied Biosystems). Sequence analysis confirmed that the gene was a gene encoding hVDR. This plasmid was named pCR-Blunt II-TOPO-hVDR.

SEQ ID NO: 1:
   5'-AATTCTCGAGATGGAGGCAATGGCGGCCAGCACTTC-3'
SEQ ID NO: 2:
   5'-ATATGCGGCCGCTCAGGAGATCTCATTGCCAAACAC-3'

### EXAMPLE 2: Preparation of LucN-LBD-LucC genes

The LBDs (ligand binding region 121-427 aa) of hVDR were amplified by PCR using primer 3 and 4 (SEQ ID NO: 3 and 4) with pCR-Blunt II-TOPO-hVDR prepared in Example 1 as template. Also, by using pGL4. 31 (Promega) vectors encoding Luc2 (firefly luciferase) as template, and primer 5 and 6 (SEQ ID NO: 5 and 6), primer 7 and 8 (SEQ ID NO: 7 and 8), LucN(Luc2 1-1246 bp), and LucC(Luc2 1247-1650 bp) were amplified by PCR, respectively. PCRs were performed under the condition: Reaction 2 (94 °C, 2 minutes later, 98 °C, 10 s, 56 °C, 30 s, 68 °C, 30 s, 30 cycles; template DNA 10 ng, MgSO₄ 1.5 mM, dNTPs 0.2 mM, KOD-plus neo-DNA polymerase (TOYOBO) 0.2 U, 10 x PCR Buffer for KOD plus neo), primer 0.3 µM each and 10µL of the final solution. After amplification was confirmed by electrophoresis of using 1 µl of each PCR product (LucN, LBD, LucC), 20 ng each of PCR products was mixed with primer 5 and 8 (SEQ ID NO: 5 and 8), and LucN, LBD, and LucC were ligated by overlap-PCR. The PCR was performed under the condition: Reaction 3 (94°C, for 2 min, 98°C, for 10 sec, 56°C, for 30 sec, 68°C, for 1 min 30 sec, 30 cycles; PCR products 20 ng each, MgSO₄ 1.5 mM, dNTPs 0.2 mM, KOD-plus neo-DNA polymerase (TOYOBO) 0.2 U, 10 x PCR Buffer for KOD plus neo), primer 0.3 µM each, and a final volume of 10µL. Electrophoresis of PCR products revealed a specific amplification at the site of interest (approximately 2.2 kb). The PCR products cloned into pCR-Blunt II-TOPO vectors by the methods described above and the plasmid DNAs was named pCR-Blunt II-TOPO-LucN-LBD-LucC after sequence analysis. 
SEQ ID NO: 3:
5'-
TACAAACGCTCTCATCGACAAGGACCGGCCCAAGCTGTCTGAGGAGCAGC-3'
 
SEQ ID NO: 4:
5'-CGATGTCGCCGCTGTGCAGCCAGCCGGAGATCTCATTGCCAAACACTTCG-
3'
 SEQ ID NO: 5:
5'-AATTCTCGAGATGGAAGATGCCAAAAACATTAAG-3' 
SEQ ID NO: 6:
5'-GCTGCTCCTCAGACAGCTTGGGCCGGTCCTTGTCGATGAGAGCGTTTGTA-
3'
 
SEQ ID NO: 7:
5'-CGAAGTGTTTGGCAATGAGATCTCCGGCTGGCTGCACAGCGGCGACATCG-
3'
 SEQ ID NO: 8:
5'-ATATGCGGCCGCTTACACGGCGATCTTGCCGCCCTTCTTG-3'

### EXAMPLE 3: Preparation of LucN-(GGGGS)×3-LBD-LucC genes

A linear DNA fragment in which (GGGGS)×3 linker sequence was added between LucN and LBD was obtained by inverse PCR using primer 9 and 10 (SEQ ID NO: 9 and 10) with pCR-Blunt II-TOPO-LucN-LBD-LucC prepared in Example 2 as template.

For Inverse PCR, KOD-plus mutagenesis kit of TOYOBO Co. was used. The Inverse PCR was performed under the conditions: Reaction 5 (94°C, 2 min, 98°C, 10 sec, 68°C, 6 min, 12 cycles; template DNA 10 ng, dNTPs 0.2 mM, KOD-plus DNA polymerase,(TOYOBO) 0.2 U, 10 x Buffer for iPCR, primer 0.3µM each, and final volume 10µL. After confirmation of amplification product of about 6 kbp with 1 µL of PCR products by agarose gel electrophoresis, 10 µL of PCR products was added with 5U of restriction enzyme *Dpn*I, and the methylated template DNAs were digested by reacting at 37 °C for 2 hours. 1 µL of *DpnI* treated solution, 5µL of T4 Polynucleotide Kinase 2, 5µL of Ligation high 2, and 3.5 µL of sterile distilled water were mixed, and total volume was adjusted to 7.5 µL, followed by ligation reaction at 16°C for 1 hour. *E.coli* HST08 strain was transformed with calcium chloride method and applied to an LB agar medium containing 30 µg/mL of kanamycin (10 g of polypeptone, 5 g of yeast extract, 5 g of NaCl and 15 g of agar dissolved in 1 L of distilled water). Several of the obtained *E.coli* colony were selected, inoculated into 2 mL of LB liquid culture medium (containing 30 µg/mL of kanamycin), and shaking culture was performed at 37°C and 200 rpm for 16 hours. The plasmid was then extracted using QIAprep Spin Miniprep Kit (QIAGEN) and plasmid concentrations were measured using a 260 nm absorbance. The resulting plasmid DNAs were named pCR-Blunt II-TOPO-LucN-(GGGGS)×3-LBD-LucC after sequencing analysis. 
SEQ ID NO: 9:
5'-
GGAGGTAGCGGTGGCGGTGGTAGTCTGCCCCACCTGGCTGACCTGGTCAG-3'
 SEQ ID NO: 10:
5'-CCCTCCACTACCCCCACCTCCGTCCTTGTCGATGAGAGCGTTTGTAGCC-3'

### EXAMPLE 4: Preparation of LucN-NHPMLMNLLKDN-(GGGGS)×3-LBD-LucC, LucN-VDLSETHPLLWTLLSSTEGDSM-(GGGGS)×3-LBD-LucC, LucN-VDLTEMHPILTSLLQNGVDHV-(GGGGS)×3-LBD-LucC genes

Using pCR-Blunt II-TOPO-LucN-(GGGGS)×3-LBD-LucC prepared in Example 3 as template, linear DNA fragments in which LXXLL sequence (amino acid sequence NHPMLMNLLKDN, VDLSETHPLLWTLLSSTEGDSM and VDLTEMHPILTSLLQNGVDHV) was added respectively to between LucN and (GGGGS)×3 linker sequence were obtained by inverse PCR using respectively primer 11 and 12 (SEQ ID NO: 11 and 12), primer 13 and 14 (SEQ ID NO: 13 and 14), primer 15 and 16 (SEQ ID NO: 15 and 16). Inverse PCR was performed under the conditions: Reaction 5 (94°C, 2 min, 98°C 10 sec, 68°C, 6 min, 12 cycles; template DNA 10 ng, dNTPs 0.2 mM, KOD-plus DNA polymerase,(TOYOBO) 0.2 U, 10 x Buffer for iPCR, primer 0.3 µM each, and final volume 10µL. After confirmation of amplification product of about 6 kbp with 1 µL of PCR products by agarose gel electrophoresis, 5 U of restriction enzyme *DpnI* was added to 10 µL of PCR products, and the methylated template DNAs were digested by reacting at 37°C for 2 hours. 1µL of *DpnI* treated solution, 5 µL of T4 Polynucleotide Kinase 2.5 µL of Ligation high 2, and 3.5 µL of sterile distilled water were mixed, and Total volume was adjusted to 7.5 µL, followed by ligation reaction at 16°C for 1 hour. After sequence analysis, the obtained plasmid DNAs were respectively named pCR-Blunt II-TOPO-LucN-NHPMLMNLLKDN-(GGGGS)×3-LBD-LucC, pCR-Blunt II-TOPO-LucN-VDLSETHPLLWTLLSSTEGDSM-(GGGGS)×3-LBD-LucC, pCR-Blunt 11-TOPO-LucN-VDLTEMHPILTSLLQNGVDHV-(GGGGS)×3-LBD-LucC.
SEQ ID NO: 11:
   5'-CATGAGCATCGGGTGGTTGTCCTTGTCGATGAGAGCGTTTGTAGC-3'
SEQ ID NO: 12:
   5'-AACCTTCTTAAAGATAATGGAGGTGGGGGTAGTGGAGGGGGAGG-3'

SEQ ID NO: 13:
5'-
ACAAGCCTTCTTCAGAATGGAGTTGACCATGTGGAGGTGGGGGTAGTGGA
GGGGGAGG-3'

SEQ ID NO: 14:
5'-
GAGGATCGGGTGCATCTCTGTGAGGTCGACGTCCTTGTCGATGAGAGCGTTT
GTAG-3'

SEQ ID NO: 15:
5'-
TGGACACTTCTTAGCAGCGAAGGGGACAGCATGGGAGGTGGGGGTAGTGGA
GGGGGAGG-3'

SEQ ID NO: 16:

5'-
AAGAAGCGGGTGTGTCTCGCTGAGGTCGACGTCCTTGTCGATGAGAGCGTTT
GTAG-3'

### EXAMPLE 5: Preparation of vectors for expressing LucN-LBD-LucC, LucN-(GGGGS)×3-LBD-LucC, LucN-NHPMLMNLLKDN-(GGGGS)×3-LBD-LucC, LucN-VDLSETHPLLWTLLSSTEGDSM-(GGGGS)×3-LBD-LucC, LucN-VDLTEMHPILTSLLQNGVDHV-(GGGGS)×3-LBD-LucC biosensor in mammal cells

Approximately 1µg each of pCR-Blunt II-TOPO-LucN-LBD-LucC, pCR-Blunt II-TOPO-LucN-(GGGGS)x3-LBD-LucC, pCR-Blunt II-TOPO-LucN-NHPMLMNLLKDN-(GGGGS)x3-LBD-LucC, pCR-Blunt II-TOPO-LucN-VDLSETHPLLWTLLSSTEGDSM-(GGGGS)×3-LBD-LucC and pCR-Blunt II-TOPO-LucN-VDLTEMHPILTSLLQNGVDHV-(GGGGS)×3-LBD-LucC plasmid DNAs prepared in Examples 2, 3 and 4 were treated with 3 U each of restriction enzyme *Xho*I, *Sph*I and *Not*I (37°C, 1 hour) and electrophoresis was performed. DNA fragment of interest was extracted from agarose gel and purified using QIAquick Gel Extraction Kit (QIAGEN to yield insert fragments (LucN-LBD-LucC, LucN-(GGGGS)×3-LBD-LucC, LucN-NHPMLMNLLKDN-(GGGGS)×3-LBD-LucC, LucN-VDLSETHPLLWTLLSSTEGDSM-(GGGGS)×3-LBD-LucC and LucN-VDLTEMHPILTSLLQNGVDHV-(GGGGS)×3-LBD-LucC). Approximately 1 µg of pEBMulti-neo vector (Wako) and pIRES2-AcGFP vector for expression in mammal cell were treated with 3 U each of restriction enzyme *Xho*I and *Not*I (37°C, 1 hour) followed by dephosphorylation of the cleaved ends with Calf Intestinal Alkaline Phosphatase (TaKaRa) and purified by ethanol precipitation to yield vector fragments. The insert and vector fragments were mixed in molar ratios of about 3:1 to 10:1, and an equal volume of Ligation High Ver. 2 (TOYOBO) was added to the mixture, followed by reaction at 16°C for 1 hour. *E.coli* HST08 strain was then transformed with calcium chloride method and applied to an LB agar medium containing 30 µg/mL kanamycin. Several of the obtained *E.coli* colony were selected, inoculated into 2 mL of LB liquid culture medium (containing 30 µg/mL of kanamycin), and shaking culture was performed at 37°C and 200 rpm for 16 hours. The plasmid was then extracted using QIAprep Spin Miniprep Kit (QIAGEN), a portion of which was treated with 1 U of restriction enzyme *Xho*I and *Not*I, and the introduction of insert was confirmed by electrophoresis. The resulting plasmid DNAs were each named pEBMulti-neo-LucN-LBD-LucC, pIRES2-LucN-LBD-LucC, pEBMulti-neo-LucN-(GGGGS)×3-LBD-LucC, pEBMulti-neo-LucN-NHPMLMNLLKDN-(GGGGS)×3-LBD-LucC, pIRES2-LucN-NHPMLMNLLKDN-(GGGGS)×3-LBD-LucC, pEBMulti-neo-LucN-VDLSETHPLLWTLLSSTEGDSM-(GGGGS)×3-LBD-LucC, pIRES2-LucN-VDLSETHPLLWTLLSSTEGDSM-(GGGGS)×3-LBD-LucC, pEBMulti-neo-LucN-VDLTEMHPILTSLLQNGVDHV-(GGGGS)×3-LBD-LucC, pIRES2-LucN-VDLTEMHPILTSLLQNGVDHV-(GGGGS)×3-LBD-LucC.

### EXAMPLE 6: Preparation of vectors for expressing LucN-VDNHPMLMNLLKDN-(GGGGS)×3-LBD-LucC and LucN-VDNHPMLMNLLKDN-(GGGGS)×3-LBD(R274L)-LucC biosensor in mammal cell

pCR-Blunt II-TOPO-LucN-NHPMLMNLLKDN-(GGGGS)×3-LBD-LucC plasmid DNAs prepared in Example 4 were used as template and VDNHPMLMNLLKDN-(GGGGS)×3-LBD-LucC fragments were amplified by PCR using primer 17 and 18 (SEQ ID NO: 17 and 18). PCR was performed under the condition: Reaction 6 (94°C, 2 min, 98°C, 10 sec, 56°C, 30 sec, 68°C, 1 min, 30 cycles; template DNA 10 ng, MgSO₄ 1.5 mM, dNTPs 0.2 mM, KOD-plus neo-DNA polymerase (TOYOBO) 0.2 U, 10 x PCR Buffer for KOD plus neo), primer 0.3µM, final volume 10 µL. The PCR products were subjected to electrophoresis and DNA fragment of interest was extracted from the agarose gel and purified using QIAquick Gel Extraction Kit (QIAGEN).The PCR-fragment VDNHPMLMNLLKDN-(GGGGS)×3-LBD-LucC was treated with 3 U of restriction enzyme *Sal*I and *Not*I (37°C, 1 hour) and then purified by ethanol precipitation to yield a insert fragment. Approximately 1 µg of pIRES2-LucN-VDLSETHPLLWTLLSSTEGDSM-(GGGGS)×3-LBD-LucC vector was treated with 3 U each of restriction enzyme *Sal*I and *Not*I (37°C, 1 hour) followed by dephosphorylation treatment of the cleaved ends with Calf Intestinal Alkaline Phosphatase (TaKaRa) and purification by ethanol precipitation to yield vector fragments. The insert and vector fragments were mixed in molar ratios of about 3:1 to 10:1, and an equal volume of Ligation High Ver. 2 (TOYOBO) was added to the mixture, followed by a reaction at 16°C for 1 hour. *E.coli* HST08 strain was then transformed with calcium chloride method and applied to an LB agar medium containing 30 µg/mL kanamycin. Several of the obtained *E. coli* colony were selected, inoculated into 2 mL of LB liquid culture medium (containing 30 µg/mL of kanamycin), and shaking culture was performed at 37°C and 200 rpm for 16 hours. The plasmid was then extracted using QIAprep Spin Miniprep Kit (QIAGEN), a portion of which was treated with 1 U of restriction enzyme *Xho*I and *Not*I*,* and the introduction of insert was confirmed by electrophoresis. The resulting plasmid DNAs were named pIRES2-LucN-VDNHPMLMNLLKDN-(GGGGS)×3-LBD-LucC. PCR was performed using primer 19 and 20 (SEQ ID NO: 19 and 20) with pIRES2-LucN-VDNHPMLMNLLKDN-(GGGGS)×3-LBD-LucC as template for preparation of LucN-VDNHPMLMNLLKDN-(GGGGS)×3-LBD(R274)-LucC biosensor with R274L type mutation in LBD in biosensor. PCR was performed under the condition of Reactions 7 (94°C, 2 min later, 98°C, 10 sec, 60°C, 30 sec, 68°C, 3 min 30 sec, 16 cycles; template DNA 10 ng, MgSO₄ 1.5 mM, dNTPs 0.2 mM, KOD-plus neo-DNA polymerase (TOYOBO) 0.2 U, 10 x PCR Buffer for KOD plus neo), primer 0.3 µM, final volume 20 µL. To PCR products was added 5 U of restriction enzyme *Dpn*I and treated at 37°C for 3 hours, then purified by ethanol precipitation and dissolved in 5 µl of ultrapure water. *E.coli* HST08 strain was then transformed with calcium chloride method and applied to an LB agar medium containing 30 µg/mL kanamycin. Several of the obtained *E.coli* colony were selected, inoculated into 2 mL of LB liquid culture medium (containing 30 µg/mL of kanamycin), and shaking culture was performed at 37°C and 200 rpm for 16 hours. Subsequently, plasmid was extracted using QIAprep Spin Miniprep Kit (QIAGEN), and the resulting plasmid DNA was cloned into *Xho*I and *Not*I sites of pIRES2-AcGFP vectors, respectively, in a manner similar to that of Example 5, after sequence verification, and it was named pIRFS2-LucN-VDNHPMLMNLLKDN-(GGGGS)×3-LBD(R274L)-LucC.
SEQ ID NO: 17:
   5'-ATAGTCGACAACCACCCGATGCTCATGAACCTTC-3'
SEQ ID NO: 18:
   5'-ATATGCGGCCGCTTACACGGCGATCTTGCCGCCCTTCTTG-3'
SEQ ID NO: 19:
   5'-CATCATGTTGCTCTCCAATGAGTCCTTCAC-3'
SEQ ID NO: 20:
   5'-ACTCATTGGAGAGCAACATGATGACCTCAATG-3'

### EXAMPLE 7: Preparation of LucN-VDNHPMLMNLLKDN-(GGGGS)×3-LBD(A165-215 aa)- LucC biosensor

For crystal structure analysis of VDRs (1-427 aa), one with deletion of 50 amino acids (165-215 aa) is used for stability reasons (Reference document E). Therefore, a DNA fragment in which 165-215 aa in LBD was deleted was obtained by inverse PCR using primer 21 and 22 (SEQ ID NO: 21 and 22) with pIRES2-LucN-VDNHPMLMNLLKDN-(GGGGS)×3-LBD-LucC as template. Inverse PCR was performed under the conditions: Reaction 8 (94°C, 2 min, 98°C 10 sec, 68°C, 7 min, 12 cycles; template DNA 10 ng, dNTPs 0.2 mM, KOD-plus DNA polymerase (TOYOBO) 0.2 U, 10 x Buffer for iPCR, primer 0.3 µM each, and 10 µL final volume. After confirming the amplified product of about 6 kbp with 1 µL of PCR products by agarose gel electrophoresis, 5 U of restriction enzyme *Dpn*I was added to 10 µL of PCR products, and the methylated template DNAs were digested by reacting at 37°C for 2 hours. 1 µL of *Dpn*I treated solution, 2.5 U of T4 Polynucleotide Kinase, 2.5 µL of Ligation high, and 3.5 µL of sterile distilled water were mixed, and Total volume was adjusted to 7.5 µL, followed by ligation reaction at 16°C for 1 hour. After sequencing analysis, the resulting plasmid DNA was named pIRES2-LucN-VDNHPMLMNLLKDN-(GGGGS)×3-LBD (Δ165-215 aa)-LucC.
SEQ ID NO: 21:
   5'-TCTGTGACCCTAGAGCTGTCCCAGC-3'
SEQ ID NO: 22:
   5'-CCCTCCACCATCATTCACACGAAC-3 '

Reference document E: N. Rochel, J.M. Wurtz, A. Mitschler, B. Klaholz, D. Moras, The crystal structure of the nuclear receptor for vitamin D bound to its natural ligand, Molecular cell, 5 (2000) 173-179.

### EXAMPLE 8: Preparation of LucN-NHPMLMNLLKDN, LucN-VDNHPMLMNLLKDN and LBD-LucC and LBD(R274L)-LucC biosensors

LucN-NHPMLMNLLKDN, LucN-VDNHPMLMNLLKDN, LBD-LucC and LBD (R274L)-LucC fragments were amplified by PCR using primer 23 and 24 (SEQ ID NO: 23 and 24), primer 25 and 26 (SEQ ID NO: 25 and 26), with pIRES2-LucN-VDNHPMLMNLLKDN-(GGGGS)×3-LBD-LucC, pIRES2-LucN-NHPMLMNLLKDN-(GGGGS)×3-LBD-LucC and pIRES2-LucN-VDNHPMLMNLLKDN-(GGGGS)×3-LBD(R274L)-LucC prepared in Examples 5 and 6, respectively, as template. PCR was performed under the condition: Reaction 9 (94°C 2 min later, 98°C 10 sec, 56°C 30 sec, 68°C 50 sec, 30 cycles; template DNA 10 ng, MgSO₄ 1.5 mM, dNTPs 0.2 mM, KOD-plus neo-DNApolymerase (TOYOBO) 0.2 U, 10 x PCR Buffer for KOD plus neo), primer 0.3 µM, final volume 10 µL. PCR products was subjected to electrophoresis and DNA fragment of interest was extracted from agarose gel and purified using QIAquick Gel Extraction Kit (QIAGEN). PCR fragments LucN-NHPMLMNLLKDN, LucN-VDNHPMLMNLLKDN, LBD-LucC and LBDs (R274L)-LucC were treated with 3 U of restriction enzyme *Xho*I and *Not*I (37°C, 1 hour) were purified by ethanol precipitation, which served as insert fragments. Insert fragments (LucN-NHPMLMNLLKDN, LucN-VDNHPMLMNLLKDN, LBD-LucC and LBD (R274L)-LucC)) were cloned into *Xho*I/*Not*I sites of the vector fragment pIRES2-AcGFP clone prepared in Example 5 to obtain pIRES2-LucN-NHPMLMNLLKDN, pIRES2-LucN-VDNHPMLMNLLKDN, pIRES2-LBD-LucC and pIRES2-LBD(R274L)-LucC as final products. The structure of biosensor is shown in Fig. 2.

SEQ ID NO: 23:
   5'-AATTCTCGAGATGGAAGATGCCAAAAACATTAAG-3'
SEQ ID NO: 24:
   5'-ATATGCGGCCGCTAATTATCTTTAAGAAGGTTCATGAGC-3'
SEQ ID NO: 25:
   5'-ATCTCGAGATGCGGCCCAAGCTGTCTGAGGAGCAGCAG-3 '
SEQ ID NO: 26:
   5'-ATATGCGGCCGCTTACACGGCGATCTTGCCGCCCTTCTTG-3'

### EXAMPLE 9: Transduction of single molecular type and double molecular type biosensors into COS-7 cells

COS-7 cells derived from African green monkey were suspended in a DMEM media containing 10 % FBS (with phenol red) and cells from 0.7 to 1.0×10⁶ were seeded on 10 cm cultivation dishes. After cultivation for 24 hours at 37 °C with 5 % CO₂, gene transfer was performed into the COS-7 cells with lipofection. 10µg of plasmid DNA encoding of single molecular type and double molecular type biosensor prepared were suspended in 1 ml of Opti-MEM medium to form DNA solutions. In separate tubes, 20 µl of Lipofectamine 2000 Transfection Reagent (Invitrogen) and 1 ml of Opti-MEM medium were suspended, mixed with the prepared DNA solution, allowed to stand at room temperature for 20 minutes, and then dropped into the cells. The cells were cultivated at 5 % CO₂ at 37 °C for 24 hours.

### EXAMPLE 10: Plating on 96 well plate

Twenty-four hours after gene transfer, the medium was removed and the cells were washed with 1×PBS. The cells were stripped by trypsinization and collected in a 15 ml conical tube, followed by centrifugal separation at 600 rpm for 3 minutes. After supernatant was removed, the cells suspended on a DMEM containing 10% CS-FBS (non-hormone-contained media) were seeded with 1.5×10⁴ of cell count per well and was cultivated 24 hours at 5 % CO₂ and 37 °C.

### EXAMPLE 11: Measurements of luciferase light emission in single molecular type and double molecular type biosensors expression cells

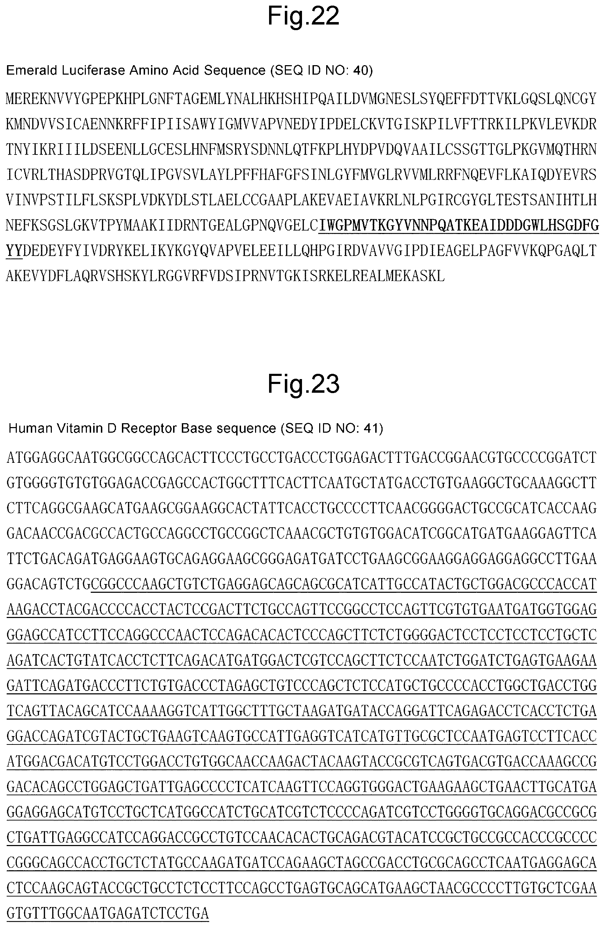

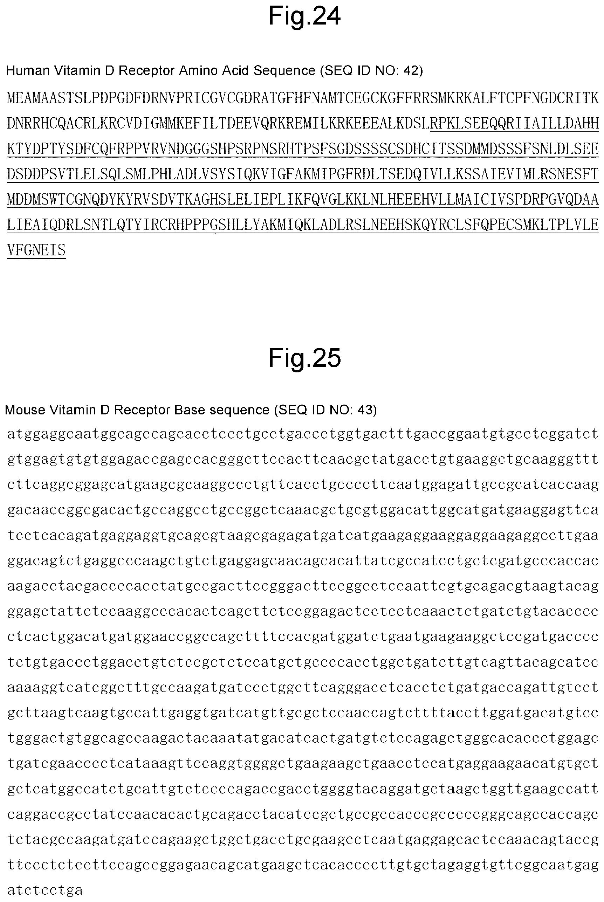

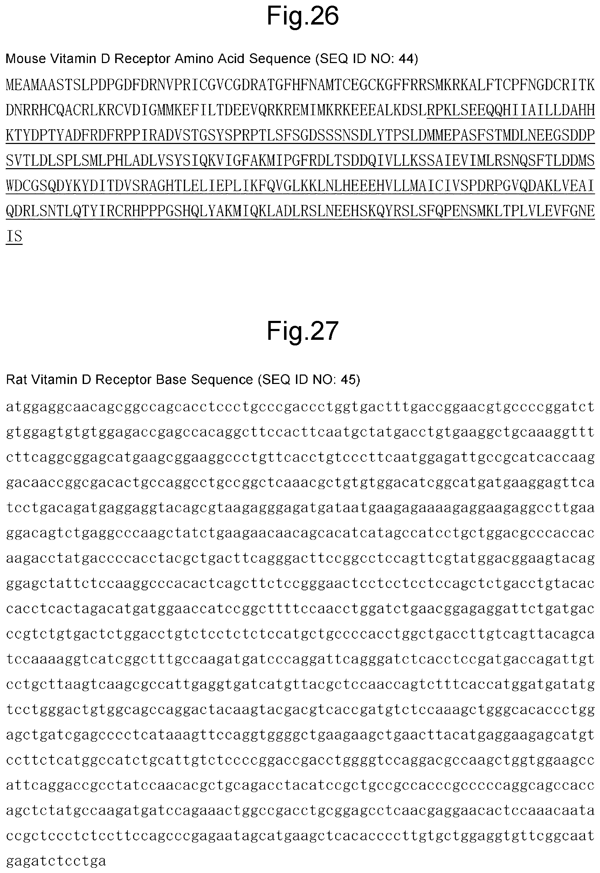

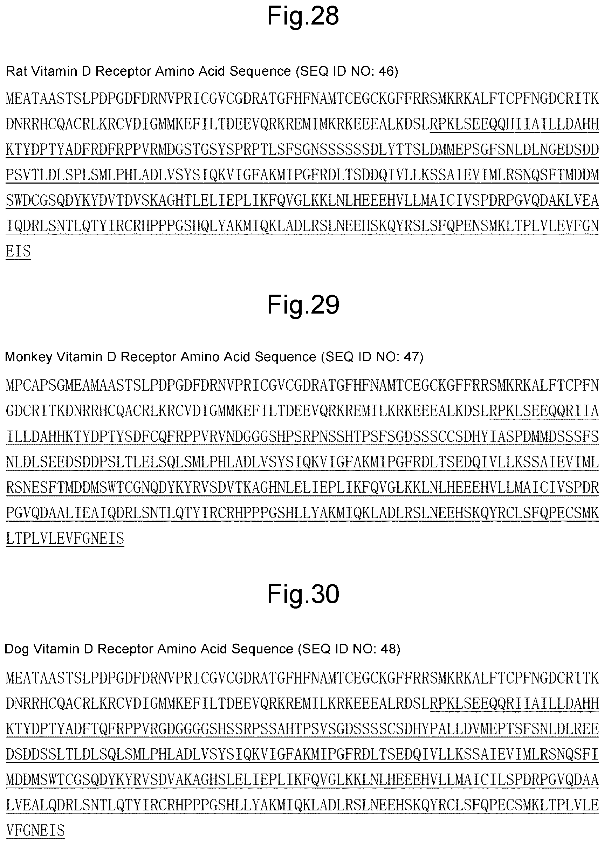

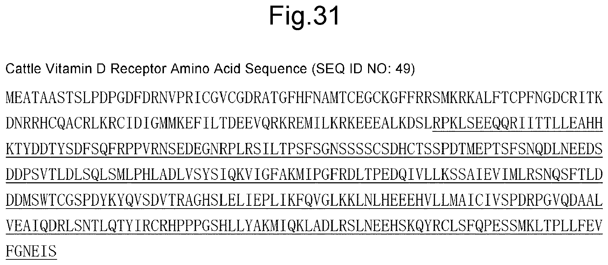

Forty-eight hours after gene transfer, the medium was changed to L-15 medium (phenol-red-free) containing a final concentration of 0.5 mM D-Luciferin Monosodium *Salt* (Thermo Scientific) and allowed to stand at room temperature for 30-60 minutes. VDR agonist (25(OH)D₃ or 1α,25(OH)₂D₃) was administered at 0 minutes prior to administration, and light emission measurements were performed over time after VDR agonist was administered to the respective wells. Ethanol (EtOH) brought in used as a solvent for VDR agonist was at 0.1% of final concentration. Microplate reader Infinite 200 Pro (TECAN) was used as an instrument for measuring light emission. Principle of variation in light emission of single molecular type and double molecular type biosensors after VDR agonist administration is shown in Figs. 3 and 4. Note that H12 in Figs. 3 and 4 is a helix related to VDR, and its sequence is an area embedded in the LBD array and is included in SEQ ID NO: 41-49 (Figs 23-31). When ligand binds to ligand binding pockets of LBDs, H12 undergoes a structure change and acts like a cap to wrap ligand. H12 is also critical for transcriptional coactivator binding. The presence of H12 in the biosensor also has a significant effect on the three-dimensional position of LucN and LucC when the structure change occurs after ligand coupling, which is critical to variation in light emission.

### EXAMPLE 12: Measurements of luciferase light emission in COS-7 cells expressing LucN-(GGGGS)×3-LBD-LucC or LucN-NHPMLMNLLKDN-(GGGGS)×3-LBD-LucC biosensor

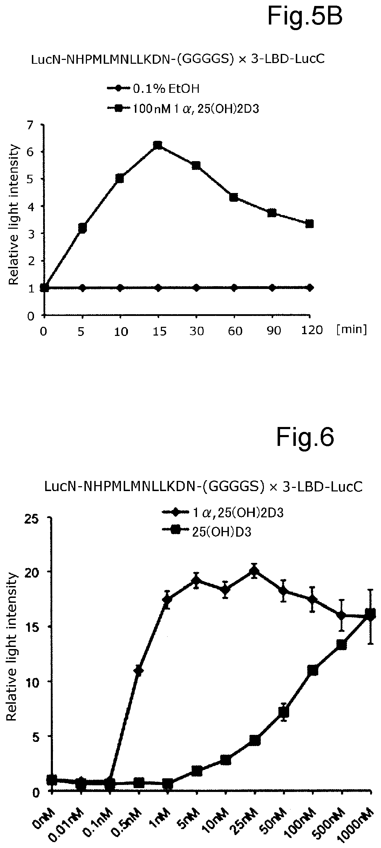

Relative changes in light emission over 120 minutes after 100 nM 1α,25(OH)₂D₃ was added to COS-7 cells expressing LucN-(GGGGS)×3-LBD-LucC or LucN-NHPMLMNLLKDN-(GGGGS)×3-LBD-LucC biosensor were compared. The amount of variation in light emission was plotted by dividing the amount of light emission at 5, 10, 15, 30, 60, 90, and 120 minutes after the addition of 100 nM 1α,25(OH)₂D₃ by the amount of light emission at 0 minute before the addition of 100 nM 1α,25(OH)₂D₃ and by the amount of light emission in the 0.1% EtOH addition group. In LucN-(GGGGS)×3-LBD-LucC biosensor, light emission amount reduced by about 40% after addition of 100 nM 1α,25(OH)₂D₃ (Fig. 5A). In LucN-NHPMLMNLLKDN-(GGGGS)×3-LBD-LucC biosensor, light emission increased by about 6-fold (Fig. 5B).

Binding of the 1α,25(OH)₂D₃ to the LBD of LucN-(GGGGS)×3-LBD-LucC biosensor changes the structure to one which loses light emission capability, whereas LucN-NHPMLMNLLKDN-(GGGGS)×3-LBD-LucC biosensor also contains interaction between LXXLL sequence (NHPMLMNLLKDN) and the LBD, in addition to the change of the LBD structure. Therefore, it is considered that these two structure changes greatly contribute to the restoration of light emission capability. Finally, we succeeded in constructing a biosensor that increases light emission responsive to the target 1α,25(OH)₂D₃. In the subsequent experiments, this biosensor was used as the basis.

### EXAMPLE 13: Responsiveness of LucN-NHPMLMNLLKDN-(GGGGS)×3-LBD-LucC biosensor to 25(OH)D₃ and 1α,25(OH) ₂D₃

Various concentrations (0-1000 nM) of 25(OH)D₃ and 1α,25(OH)₂D₃ were added to the COS-7 cells expressing LucN-NHPMLMNLLKDN-(GGGGS)×3-LBD-LucC biosensor, and the concentration dependency was compared from relative light intensity after 90 minutes. The amount of variation in light emission was plotted by dividing the amount of light emission at 90 minutes after addition of 25(OH)D₃ and 1α,25(OH)₂D₃ by the amount of light emission at 0 minutes prior to addition of 25(OH)D₃ and 1α,25(OH)₂D₃ and by the amount of light emission in the 0 nM addition group. As a result, both the 25(OH)D₃ and the 1α,25(OH)₂D₃ increased light emission in a concentration-dependent manner. However, the 1α,25(OH)₂D₃ concentration reaching maximal light emission was at 1nM, whereas 25(OH)D₃ required 1000 nM (Fig. 6). This difference is believed to reflect the difference in affinity between the 25(OH)D₃ and the 1α,25(OH)₂D₃. Thus, LucN-NHPMLMNLLKDN-(GGGGS)×3-LBD-LucC biosensor can evaluate the difference in affinity of VDR agonist.

### EXAMPLE 14: Selection of promotor for expressing biosensor protein and optimization of LXXLL sequence

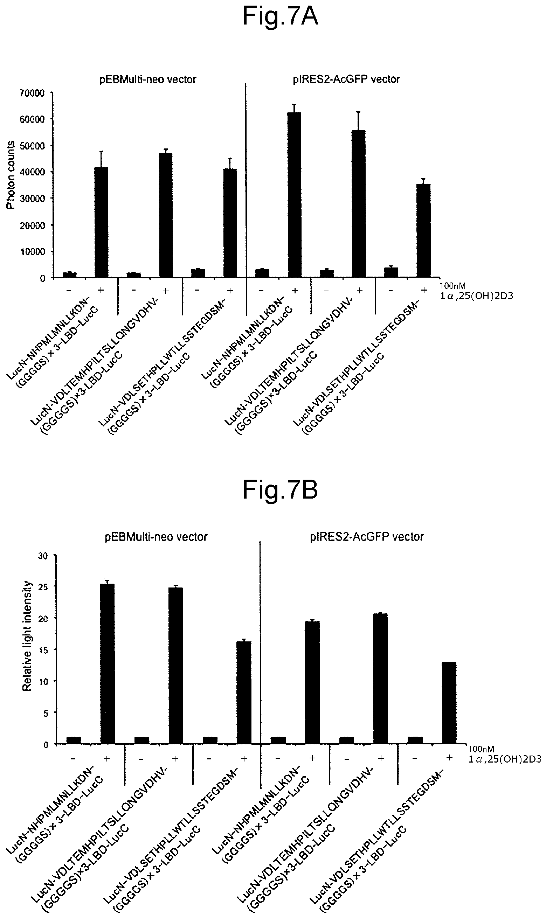

In the present study, there are two types of expression vectors used in mammal cell expression system: pEBMulti-neo and pIRES2-AcGFP vectors. The former induces gene expression by CAG promotor and the latter by CMV promotor. Both promotors can constantly express a protein, and the expression quantity does not change by the addition of VDR agonist. A 100 nM 1α,25(OH)₂D₃ was added respectively to COS-7 cells expressing plasmid DNAs (pEBMulti-neo-LucN-NHPMLMNLLKDN-(GGGGS) ×3-LBD-LucC, pIRES2-LucN-NHPMLMNLLKDN-(GGGGS)×3-LBD-LucC, pEBMulti-neo-LucN-VDLSETHPLLWTLLSSTEGDSM-(GGGGS)×3-LBD-LucC, pIRES2-LucN-VDLSETHPLLWTLLSSTEGDSM-(GGGGS)×3-LBD-LucC, pEBMulti-neo-LucN-VDLTEMHPILTSLLQNGVDHV-(GGGGS)×3-LBD-LucC, pIRES2-LucN-VDLTEMHPILTSLLQNGVDHV-(GGGGS)×3-LBD-LucC) prepared in Example 5, and light emission and relative light intensity after 90 minutes were compared. As a result, light emission amount was higher in the plasmid DNAs cloned into pIRES2-AcGFP and expressed under CMV promotor control (Fig. 7A). In contrast, relative light intensities were larger in the plasmid DNAs cloned into pEBMulti-neo and expressed under CAG promotor control (Fig. 7B). LXXLL sequence showed a light emission increase of about 20-25 fold when NHPMLMNLLKDN and VDLTEMHPILTSLLQNGVDHV were used, but a light emission increase of about 10-15 fold when VDLSETHPLLWTLLSSTEGDSM was used, the change was slightly smaller. From the above, it is considered that the difference in promotor does not greatly affect the performance of biosensor, but the difference in LXXLL sequence affects variation in light emission amount. Subsequent experiments used pIRES2-AcGFP vectors cloned with a biosensor.

### EXAMPLE 15: Comparison of performances of LucN-NHPMLMNLLKDN-(GGGGS)×3-LBD-LucC and LucN-VDNHPMLMNLLKDN-(GGGGS)×3-LBD-LucC biosensors

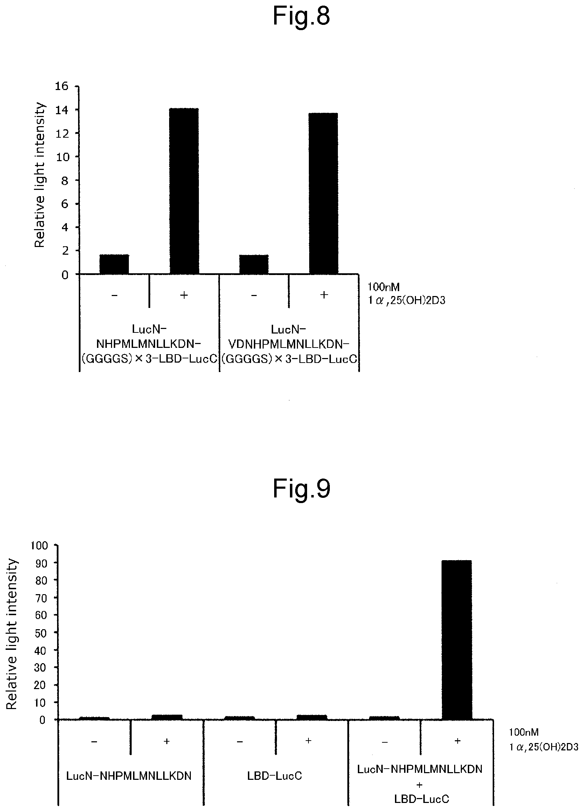

A 100 nM 1α,25(OH)₂D₃ was added to COS-7 cells expressing LucN-NHPMLMNLLKDN-(GGGGS)×3-LBD-LucC and LucN-VDNHPMLMNLLKDN-(GGGGS)×3-LBD-LucC biosensor, respectively, and relative light intensity after 60 minutes was compared. As a result, light emission amount was increased by about 14-fold in both biosensors, and no significant differences were observed (Fig. 8). This suggests that *Sal*I site between LucN and NHPMLMNLLKDN (amino acid sequence VD) does not affect the performance of biosensor.

### EXAMPLE 16: Responsiveness of double molecular type LucN-NHPMLMNLLKDN+ LBD-LucC biosensor to 1α,25(OH)₂D₃

A 100 nM 1α,25(OH)₂D₃ was added to COS-7 cells expressing LucN-NHPMLMNLLKDN or LBD-LucC alone, co-expressing LucN-NHPMLMNLLKDN and LBD-LucC, and relative light intensity after 60 minutes was compared. As a result, light emission could not be detected by adding 1α,25(OH)₂D₃ in COS-7 cells expressing LucN-NHPMLMNLLKDN or LBD-LucC alone. In contrast, COS-7 cells, in which LucN-NHPMLMNLLKDN and LBD-LucC were co-expressed, increased in light emission responsive to 1α,25(OH)₂D₃ (Fig. 9). This increased light emission suggests that LucN and LucC are reconstituted and light emission is restored when an interaction occurs between LXXLL sequence in biosensor protein and LBD.

### EXAMPLE 17: Comparisons of 1α,25(OH)₂D₃ concentrations dependency of single molecular type and double molecular type biosensors

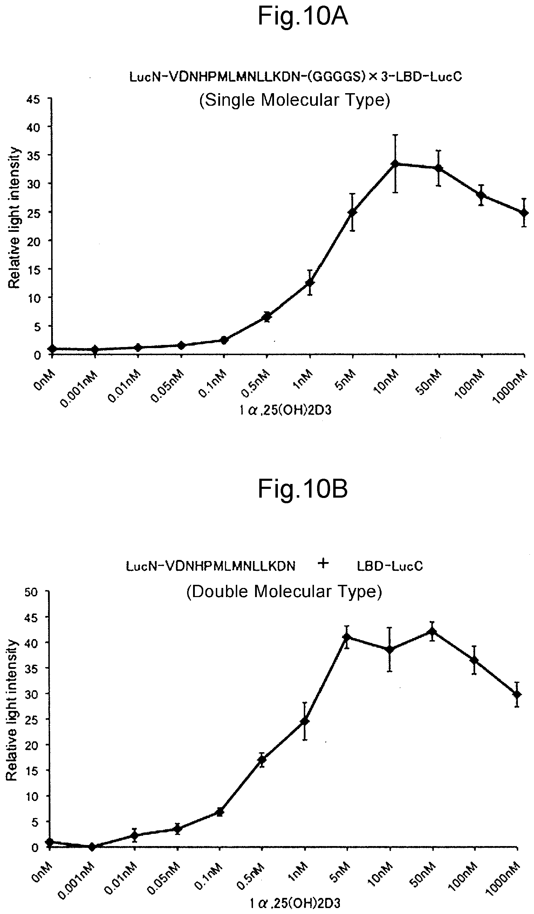

COS-7 cells expressing LucN-VDNHPMLMNLLKDN-(GGGGS)×3-LBD-LucC alone and COS-7 cells co-expressing LucN-NHPMLMNLLKDN and LBD-LucC were supplemented with various concentrations (0-1000 nM) of 1α,25(OH)₂D₃, and the concentration dependencies were compared from relative light intensity after 60 minutes. As a result, both single molecular type and double molecular type biosensors increased light emission amount in a manner dependent on the 1α,25(OH)₂D₃ concentration, and the concentration curves were almost identical (Fig. 10).

### EXAMPLE 18: Comparison of dependencies of LucN-VDNHPMLMNLLKDN-(GGGGS)×3-LBD-LucC AND LucN-VDNHPMLMNLLKDN-(GGGGS)×3-LBD[A165-215 aa]-LucC biosensors on 1α,25(OH)₂D₃ concentrations

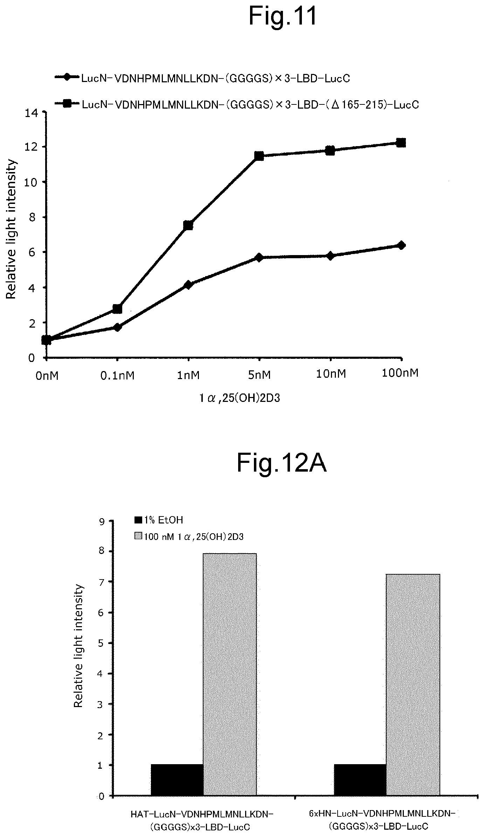

COS-7 cells expressing LucN-VDNHPMLMNLLKDN-(GGGGS)×3-LBD-LucC or LucN-VDNHPMLMNLLKDN-(GGGGS)×3-LBD[AI65-215 aa]-LucC biosensor were supplemented with various concentrations (0-100 nM) of 1α,25(OH)₂D₃, and the concentration dependency was compared from relative light intensity after 60 minutes. The results showed that both biosensors increased light emission in a concentration-dependent manner, whereas LucN-VDNHPMLMNLLKDN-(GGGGS)×3-LBD[A165-215 aa]-LucC biosensor, which lacked 50 amino acids in the LBD, had higher variation in light emission levels (Fig. 11).

### EXAMPLE 19: Preparation of vectors for expression of LucN-VDNHPMLMNLLKDN-(GGGGS)×3-LBD-LucC, LucN-VDNHPMLMNLLKDN-(GGGGS)×3-LBD(R274L)-LucC, LucN-VDNHPMLMNLLKDN-(GGGGS)×3-LBD(A165-215 aa)-LucC, LucN-VDNHPMLMNLLKDN, LBD-LucC and LBD(R274L)-LucC biosensor proteins in E.coli

To express biosensor proteins in *E.coli,* approximately 1 µg each of pIRES2-LucN-VDNHPMLMNLLKDN-(GGGGS)×3-LBD-LucC, pIRES2-LucN-VDNHPMLMNLLKDN-(GGGGS)×3-LBD(R274L)-LucC, pIRES2-LucN-VDNHPMLMNLLKDN-(GGGGS)×3-LBD (Δ165-215 aa)-LucC, pIRES2-LucN-VDNHPMLMNLLKDN, pIRES2-LBD-LucC and pIRES2-LBD(R274L)-LucC plasmid DNAs were treated with 3 U each of restriction enzymes *Xho*I and *Not*I (37°C, 1 hour) and subjected to electrophoresis. DNA fragment of interest was extracted from agarose gel and purified using QIAquick Gel Extraction Kit (QIAGEN) to yield insert fragments, LucN-VDNHPMLMNLLKDN-(GGGGS)×3-LBD-LucC, LucN-VDNHPMLMNLLKDN-(GGGGS)×3-LBD(R274L)-LucC, LucN-VDNHPMLMNLLKDN-(GGGGS)×3-LBD(Δ165-215 aa)-LucC, LucN-VDNHPMLMNLLKDN, LBD-LucC and LBD(R274L)-LucC.

Double strand fragments produced were introduced into *Nco*I/*Bam*HI sites of pET-11d (Novagen Corp.) vector by mixing with 10µM of primers 27 and 28 (SEQ ID NO: 27 and 28) respectively, 10×PNK buffer, 1 mM ATP, 1 U T4 PNK and reacting at 37°C for one and a half hours, 95°C for 10 minutes, 75°C for 10 minutes and then lowering the temperatures to 37°C to produce a pET-11d-N-6×His vector. In order to insert *Not*I site into pET-11d-N-6 × His vector, double strand fragments prepared were incorporated into *Xho*I site of pET-11d-N-6 × His vector by mixing 10µM of primers 29 and 30 (SEQ ID NO: 29 and 30), respectively, 10×PNK buffer, 1 mM ATP, 1 U T4 PNK and reacting at 37°C for 1 hour and a half, 10 minutes at 95°C and 10 minutes at 75°C and lowering the temperature to 37°C to prepare a pET-11d-N-6×His-*Xho*I/*Not*I vector. Proteins, including 6×His tags, may form inclusions within *E.coli,* and 6×HN or HAT tags may solve this issue. Therefore, inverse PCR was performed using primer 31 and 32 (SEQ ID NO: 31 and 32) and primer 33 and 34 (SEQ ID NO: 33 and 34), respectively, for preparation of 6×HN tags and HAT tags. Inverse PCR was carried out under the condition of Reaction 5 (94°C for 2 min, 98°C for 10 sec, 68°C for 6 min, 12 cycles; template DNA 10 ng, dNTPs 0.2 mM, KOD-plus DNA polymerase (TOYOBO) 0.2 U, 10 x Buffer for iPCR, primer 0.3 µM each, and 10 µL final volume. After amplification product of about 6kbp was confirmed with 1 µL of PCR products by agarose gel electrophoresis, the methylated template DNAs were digested by adding 5U of restriction enzyme *Dpn*I to 10 µL of PCR products and reacting at 37°C for 2 hours. 1 µL of *Dpn*I treated solution, 2.5 U of T4 Polynucleotide Kinase, 2.5 µL of Ligation high and 3.5 µL of sterile distilled water were mixed, and total volume was adjusted to 7.5 µL, followed by ligation reaction at 16°C for 1 hour. The resulting plasmid DNAs were sequenced and pET-11d-N-6×HN- *Xho*I/*Not*I and pET-11d-N-HAT-*Xho*I/*Not*I vectors were obtained. Approximately 1 µg of pET-11d-N-6×HN- *Xho*I/*Not*I and pET-11d-N-HAT- *Xho*I/*Not*I vectors were treated respectively with 3 U of restriction enzyme *Xho*I and *Not*I (37°C, 1 hour), followed by dephosphorylation of the cleaved ends with Calf Intestinal Alkaline Phosphatase (TaKaRa), and purified by ethanol precipitation to yield vector fragments. The insert, and vector fragments were mixed in molar ratios of about 3:1 to 10:1, and an equal volume of Ligation High Ver. 2 (TOYOBO) was added to the mixture, followed by a reaction at 16°C for 1 hour. *E. coli* HST08 strain was then transformed with calcium chloride method and applied to an LB agar medium containing 100 µg/mL of ampicillin. Several of the obtained *E.coli* colony were selected, inoculated into 10 mL of LB liquid culture medium (containing 100 µg/mL of ampicillin), and subjected to shaking culture at 37°C and 200 rpm for 16 hours. Plasmids were then extracted using QIAprep Spin Miniprep Kit(QIAGEN), a portion of which was treated with 1 U of restriction enzyme *Xho*I and *Not*I*,* and the introduction of insert was confirmed by electrophoresis. The resulting plasmid DNAs were named pET-11d-N-6×HN-LucN-VDNHPMLMNLLKDN-(GGGGS)×3-LBD-LucC, pET-11d-N-6×HN-LucN-VDNHPMLMNLLKDN-(GGGGS)×3-LBD(R274L)-LucC, pET-11d-N-6×HN-LucN-VDNHPMLMNLLKDN-(GGGGS)×3-LBD(Δ165-215 aa)-LucC, pET-11d-N-HAT-LucN-VDNHPMLMNLLKDN-(GGGGS)×3-LBD-LucC, pET-11d-N-6×HN-LucN-VDNHPMLMNLLKDN, pET-11d-N-6×HN-LBD-LucC and pET-11d-N-6×HN-LBD(R274L)-LucC, respectively.

SEQ ID NO: 27:
   5'-CATGCGCGGAAGCCATCACCATCACCATCACGGATCCCTCGAGAGGCCT-3'
SEQ ID NO: 28:
   5'-GATCAGGCCTCTCGAGGGATCCGTGATGGTGATGGTGATGGCTTCCGCG-3'
SEQ ID NO: 29:
   5'-TCGAGGAATTCGCGGCCGCGTCGACG-3'
SEQ ID NO: 30:
   5'-TCGACGTCGACGCGGCCGCGAATTCC-3'

SEQ ID NO: 31:
5'-
CACAACGCTGCAGGTGATGACGATGATAAGGGATCCCTCGAGAGGCCTGATC
CGGC-3'

SEQ ID NO: 32:
5'-
ATTATGATTATGATTATGATTATGATTATGACCCATGGTATATCTCCTTCTTAAAG
TTAAAC-3'

SEQ ID NO: 33:
5'-
CACGCTCATGCCCACAACAAGATCGATGACGATGACAAAGGATCCCTCGAGA
GGCCTGATCCGGC-3'

SEQ ID NO: 34:
5'-
ATTATGATTATGATTATGATTATGATTATGACCCATGGTATATCTCCTTCTTAAAG
TTAAAC-3'

### EXAMPLE 20: Expression of LucN-VDNHPMLMNLLKDN-(GGGGS)×3-LBD-LucC, LucN-VDNHPMLMNLLKDN-(GGGGS)×3-LBD(R274L)-LucC, LucN-VDNHPMLMNLLKDN-(GGGGS)×3-LBD(Δ165-215 aa)-LucC, LucN-VDNHPMLMNLLKDN, LBD-LucC and LBD (R274L)-LucC proteins in E.coli BL21(DE)3 or SoluBL21 strains

*E.coli* BL21(DE3) or SoluBL21 strains were transformed with calcium chloride method using pET-11d-N-6×HN-LucN-VDNHPMLMNLLKDN-(GGGGS)×3-LBD-LucC, pET-11d-N-6×HN-LucN-VDNHPMLMNLLKDN-(GGGGS)×3-LBD(R274L)-LucC, pET-11d-N-6×HN-LucN-VDNHPMLMNLLKDN-(GGGGS)×3-LBD (Δ165-215 aa)-LucC, pET-11d-N-HAT-LucN-VDNHPMLMNLLKDN-(GGGGS)×3-LBD-LucC, pET11d-N-6×HN-LucN-VDNHPMLMNLLKDN, pET-11d-N-6×HN-LBD-LucC and pET-11d-N-6×HN-LBD(R274L)-LucC plasmid DNAs and applied to LB-agar medium containing 100 µg/mL ampicillin. Several of the obtained *E.coli* colony were selected, inoculated into 2 mL of LB liquid culture medium (containing 100 µg/mL of ampicillin), and subjected to shaking culture at 37°C and 200 rpm for 16 hours. A2 mL *E.coli* culture was inoculated into 500 mL of LB liquid culture medium (containing 100 µg/mL ampicillin) and cultivated to OD₆₀₀=0.4 at 37°C 0.1 mM isopropyl-1-thio-β-D-galactopyranoside [IPTG (NACALAI TESQUE, INC.)] was added and the proteins were expressed by cultivation at 15°C for 3 hours. After cultivation, centrifugal separation was performed at 4°C, 5000×g for 10 minutes to collect bacterial cells. The bacterial cells were suspended in a solution containing 25 mM Tris-HCl, pH 7.4, 10 mM DTT, and protease inhibitor Cocktail (NACALAI TESQUE, INC.), followed by a 15 second × 7 ultrasonic crushing in ice. Thereafter, centrifugal separation was performed at 4°C, 20,000×g for 30 minutes, and supernatant was collected to form biosensor protein solutions.

### EXAMPLE 21: Luciferase Assays in in vitro System

After dispensing 50 µl/well of biosensor protein prepared in Example 20 to 96 well plate, 1α,25(OH)₂D₃ was added in various concentrations, and incubated at room temperature for 5-10 minutes. Then, 50µL of luciferin solution [25 mM Tris-HCl (pH 7.4), 20 mM MgSO₄, and 2 mM D-Luciferin, 4 mM ATP] was added, and light emission amount was measured at any point in time. Ethanol (EtOH) brought in, which is used as a solvent for VDR ligand, was at 1% of final concentration. Microplate reader Infinite 200 Pro (TECAN) was used as an instrument for measuring light emission.

### EXAMPLE 22: Comparison of expression amounts and responsiveness of HAT-LucN-VDNHPMLMNLLKDN-(GGGGS)×3-LBD-LucC AND 6xHN-LucN-VDNHPMLMNLLKDN-(GGGGS)×3-LBD-LucC biosensor to 1α,25(OH)₂D₃

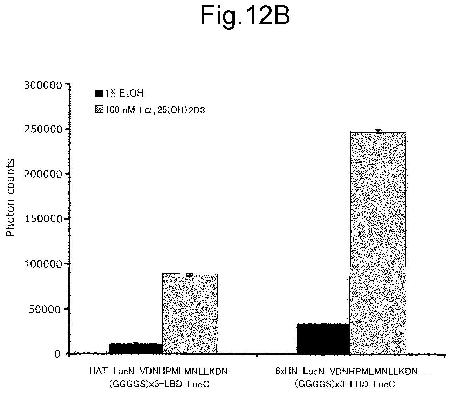

After dispensing 50 µl/well of HAT-LucN-VDNHPMLMNLLKDN-(GGGGS)×3-LBD-LucC or 6xHN-LucN-VDNHPMLMNLLKDN-(GGGGS)×3-LBD-LucC biosensor protein to 96 well plate, 200 nM 1α,25(OH)₂D₃ was added and incubated for 5-10 minutes at room temperature. After that, 50µL of luciferin solution [25 mM Tris-HCl (pH 7.4), 20 mM MgSO₄, and 2 mM D-Luciferin, 4 mM ATP] were added, and light emission amount and relative light intensity were compared after 10 minutes. The final concentration of 1α,25(OH)₂D₃ was 100 nM, and the brought-in ethanol (EtOH) used as solvent of 1α,25(OH)₂D₃ was at 1% of final concentration. Both biosensors responded to 1α,25(OH)₂D₃ by increasing the relative light emission intensity by about 8 (Fig. 12A). Light emission levels of 6xHN-LucN-VDNHPMLMNLLKDN-(GGGGS)×3-LBD-LucC biosensor protein was more than twice comparing to those of HAT-LucN-VDNHPMLMNLLKDN-(GGGGS)×3-LBD-LucC (Fig. 12B).

### EXAMPLE 23: Optimization of light emission measurement period in 6xHN-LucN-VDNHPMLMNLLKDN-(GGGGS)×3-LBD-LucC biosensor

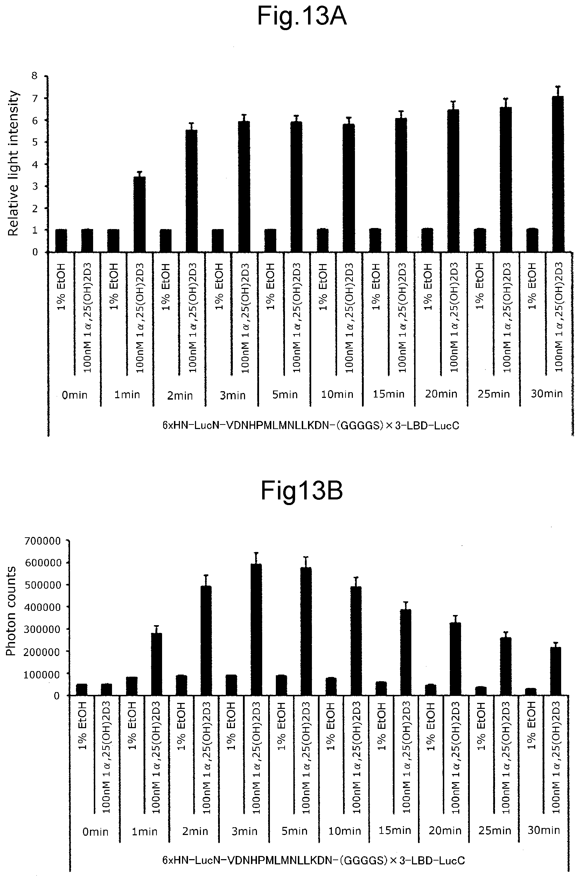

After dispensing 50 µl/well of 6xHN-LucN-VDNHPMLMNLLKDN-(GGGGS)×3-LBD-LucC biosensor protein into 96 well plate, 200 nM 1α,25(OH)₂D₃ was added and incubated at room temperature for 5-10 minutes. After that, 50 µL of luciferin solution [25 mM Tris-HCl (pH 7.4), 20 mM MgSO₄, and 2 mM D-Luciferin, 4 mM ATP] was added, and light emission amount and relative light intensity after 0-30 minutes were compared. The final concentration of 1α,25(OH)₂D₃ was 100 nM, and the brought-in ethanol (EtOH) used as solvent of 1α,25(OH)₂D₃ was at 1% of final concentration. In response to the 1α,25(OH)₂D3, the relative light emission intensity increased by about 6 and after 2 minutes, reached the equilibrium condition (Fig. 13A). However, light emission levels peaked at 3-5 minutes after addition of luciferin solutions and then decreased (Fig. 13B). Therefore, 5 minutes after adding luciferin was taken as the duration of light emission measurements.

### EXAMPLE 24: Comparison of affinity of 1α,25(OH)₂D₃ and 25(OH)D₃ using 6xHN-LucN-VDNHPMLMNLLKDN-(GGGGS)×3-LBD-LucC biosensor

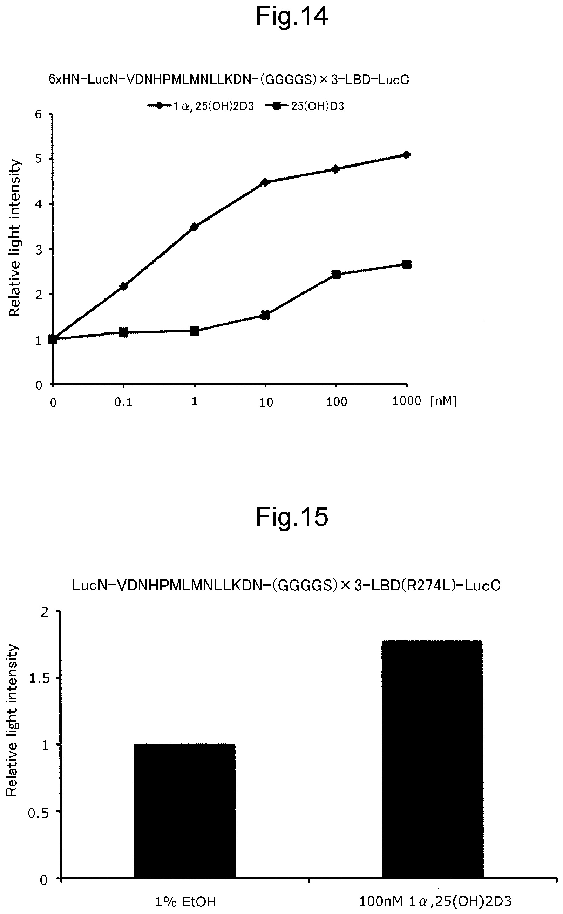

After dispensing 50 µl/well of 6xHN-LucN-VDNHPMLMNLLKDN-(GGGGS)×3-LBD-LucC biosensor protein into 96 well plate, various concentrations (0-1000 nM final concentration) of 1α,25(OH)₂D₃, and 25(OH)D₃ were added and incubated for 5-10 minutes at room temperature. Then, 50µL of luciferin solutions [25 mM Tris-HCl (pH 7.4), 20 mM MgSO₄, and 2 mM D-Luciferin, 4 mM ATP] were added, and relative light intensity after 5 min was compared. Ethanol (EtOH) brought in, which is used as a solvent for 1α,25(OH)₂D₃ and 25(OH)D₃, was at 1% of final concentration. The relative light emission amount increased depending on the concentrations of 1α,25(OH)₂D₃ and 25(OH)D₃, but light emission increase of 25(OH)D₃ was lower than that of 1α,25(OH)₂D₃ (Fig. 14). This difference is believed to reflect the difference in affinity between the 25(OH)D₃ and the 1α,25(OH)₂D₃.

### EXAMPLE 25: Responsiveness of 6xHN-LucN-VDNHPMLMNLLKDN-(GGGGS)×3-LBD(R274L)-LucC biosensor to 1α,25(OH)D₃

After dispensing 50 µl/well of 6xHN-LucN-VDNHPMLMNLLKDN-(GGGGS)×3-LBD-LucC or 6xHN-LucN-VDNHPMLMNLLKDN-(GGGGS)×3-LBD(R274L)-LucC biosensor protein to 96 well plate, 200 nM of 1α,25(OH)₂D₃ was added and incubated for 5-10 minutes at room temperature. Then, 50µL of luciferin solutions [25 mM Tris-HCl (pH 7.4), 20 mM MgSO₄, and 2 mM D-Luciferin, 4 mM ATP] were added, and relative light intensity after 5 min was compared. The final concentration of 1α,25(OH)₂D₃ was 100 nM, and the brought-in ethanol (EtOH) used as solvent of 1α,25(OH)₂D₃ was at 1% of final concentration. As shown in Examples 22-24, when 6xHN-LucN-VDNHPMLMNLLKDN-(GGGGS)×3-LBD-LucC biosensor was used, light emission increase after addition of 100 nM 1α,25(OH)₂D₃ was about 6-8-fold, whereas light emission of 6xHN-LucN-VDNHPMLMNLLKDN-(GGGGS)×3-LBD(R274L)-LucC biosensor increased only about 1.7-fold after addition of 100 nM 1α,25(OH)₂D₃ (Fig. 15). This indicates that inserting R274L mutation into the LBD significantly lowered affinity for the 1α,25(OH)₂D₃.

### EXAMPLE 26: Assessment of changes after adding 100 nM 1α,25(OH)₂D₃ and 100 nM NS-4 Using double molecular type 6xHN-LucN-NHPMLMNLLKDN, 6xHN-LBD-LucC, and 6xHN-LBD(R274L)-LucC biosensors

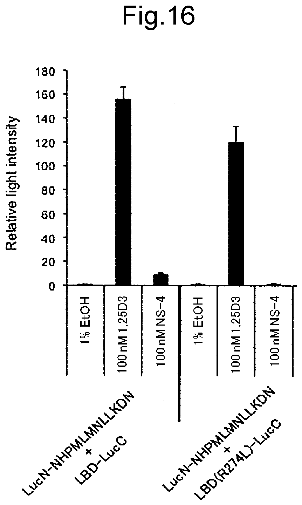

6xHN-LBD-LucC or 6xHN-LBD(R274L)-LucC proteins were dispensed at 96 well plate in 25 µl per well each, followed by the addition of 100 nM 1α,25(OH)₂D₃ or 100 nM NS-4 and incubation at room temperature for 5-10 minutes. Then, 25 µL of 6xHN-LucN-NHPMLMNLLKDN was added to react for 5-10 min, and 50µL of luciferin solution [25 mM Tris-HCl (pH 7.4), 20 mM MgSO₄, and 2 mM D-Luciferin, 4 mM ATP] were added to compare relative light intensity after 5 min. Ethanol (EtOH) brought in used as a solvent of 1α,25(OH)₂D₃ was at 1% of final concentration. In combinations of 6xHN-LucN-NHPMLMNLLKDN and 6xHN-LBD-LucC, adding 100 nM 1α,25(OH)₂D₃ increased the relative light emission by 160-fold, whereas adding 100 nM NS-4 increased the relative light emission by as much as 10-fold (Fig. 16). On the other hand, when 1α,25(OH)₂D₃ was added to the combination of 6xHN-LucN-NHPMLMNLLKDN and 6xHN-LBD(R274L)-LucC, the relative light emission amount increased 120-fold, and the responsiveness decreased by about 25% compared to 6xHN-LBD-LucC. No light emission increased for NS-4. From the above, it is considered that double molecular type is much more sensitive than the above-mentioned 6xHN-LucN-NHPMLMNLLKDN-(GGGGS)×3-LBD(R274L)-LucC biosensor of single molecular type because it increases after adding 1α,25(OH)₂D₃.

### EXAMPLE 27: Cloning of LgBiT and SmBiT

*Xho*I*-*ATG*-*LgBiT-*Sal*I and *Bam*HI-LgBiT-TAG-*Not*I were amplified by PCR using primer 53 and 54 (SEQ ID NO: 53 and 54) and primer 55 and 56 (SEQ ID NO: 55 and 56), respectively, with pBiT.1-N [TK/LgBiT] vector (Promega) as template. PCRs were performed under the condition of Reaction 1 (94°C 2 minutes later, 98°C 10 seconds, 56°C 30 seconds, 68°C 1 minute, 30 cycles; template DNA 10 ng, MgSO₄ 1.5 mM, dNTPs 0.2 mM, KOD-plus neo-DNA polymerase (TOYOBO) 0.2 U, 10 x PCR Buffer for KOD plus neo), primer 0.3 µM each, and a final volume of 10 µL. Electrophoresis of 1 µL of PCR product with 1% agarose gel resulted in a specific amplification at the site of interest (approximately 400 bps) (hereinafter electrophoresis at 1% agarose gel is simply abbreviated as electrophoresis). After amplification of the objective fragment was confirmed by electrophoresis, the PCR product was cloned into pCR Blunt II-TOPO vectors according to the handling instructions of Zero blunt TOPO PCR Cloning Kit (Invitrogen), *E. coli* HST08 strain was transformed by calcium chloride method and applied to LB agar medium (10 g of polypeptone, 5 g of yeast extract, 5 g of NaCl and 15 g of agar dissolved in 1 L of distilled water) containing 30 µg/mL of kanamycin. Several of the obtained *E.coli* colony were selected, inoculated into 2 mL of LB liquid culture medium (containing 30 µg/mL of kanamycin), and shaking culture was performed at 37°C and 200 rpm for 16 hours. The plasmid was then extracted using QIAprep Spin Miniprep Kit (QIAGEN) and plasmid concentrations were measured using a 260 nm absorbance to yield template DNAs. Cycle sequencing reactions were performed using BigDye Terminator v3.1 Cycle Sequencing Kit (Applied Biosystems). Sequence analysis confirmed *Xho*I-ATG-LgBiT-*Sal*I and *Bam*HI-LgBiT-TAG-*Not*I genes. This plasmid was named pCR-Blunt II-TOPO-*Xho*I-ATG-LgBiT-*Sal*I and pCR-Blunt II-TOPO-*Bam*HI-LgBiT-TAG-*Not*I.

Next, 10 µM each, 10×PNK buffer, 1 mM ATP, 1 U T4 PNK of primer 57 and 58 (SEQ ID NO: 57 and 58) and primer 59 and 60 (SEQ ID NO: 59 and 60) were mixed and reacted at 37°C for one and a half hours, 95°C for 10 minutes, 75°C for 10 minutes and then lowering the temperatures to 37°C to produce a double strand fragment of *Xho*I-ATG-SmBiT-*Sal*I and *Bam*HI-SmBiT-TAG-*Not*I, respectively. Primer 57-60 (SEQ ID NO: 57-60) required for the production of double strand fragment encoding SmBit was produced with the permission of Promega.

SEQ ID NO: 53:
   5'-atatctcgagATGGTCTTCACACTCGAAGATTTC-3
SEQ ID NO: 54:
   5'-atatgtcgacACTGTTGATGGTTACTCGGAACAG-3'
SEQ ID NO: 55:
   5'-atatggatccGTCTTCACACTCGAAGATTTCGTTG-3'
SEQ ID NO: 56:
   5'-atatgcggccgCTAACTGTTGATGGTTACTCGGAAC-3'
SEQ ID NO: 57:
   5'-tcgagATGGTGACCGGCTACCGGCTGTTCGAGGAGATTCTGg-3'
SEQ ID NO: 58:
   5'-tcgacCAGAATCTCCTCGAACAGCCGGTAGCCGGTCACCATc-3'
SEQ ID NO: 59:
   5'-gatccGTGACCGGCTACCGGCTGTTCGAGGAGATTCTGtagc-3'
SEQ ID NO: 60:
   5'-ggccgctaCAGAATCTCCTCGAACAGCCGGTAGCCGGTCACg-3'

### EXAMPLE 28: Preparation of pIR-ES2-XhoI-ATG-LgBiT-SalI, pIRES2-BamHI-LgBiT-TAG-NotI, pIRES2-XhoI-ATG-SmBiT-SalI and pIRES2-BamHI-SmBiT-TAG-NotI vectors

After treatment of pCR-Blunt II-TOPO-*Xh*oI-ATG-LgBiT-*Sal*I and pCR-Blunt II-TOPO-*Bam*HI-LgBiT-TAG-*Not*I vectors produced by EXAMPLE 27 with restriction enzyme *Xho*I*lSal*I and *Bam*HI/*Not*I*,* the resulting *Xho*I*-*ATG*-*LgBiT*-Sal*I and *Bam*HI-LgBiT-TAG-*Not*I fragments and double strand fragment of *Xh*oI-ATG-SmBiT-*Sal*I and *Bam*HI-SmBiT-TAG-*Not*I were incorporated into *Xho*I/*Sal*I and *Bam*HI/*Not*I sites of pIRES2-AcGFP vector. The resulting plasmid DNAs were named pIRES2-*Xho*I-ATG-LgBiT-*Sal*I, pIRES2-*BamH*I-LgBiT-TAG-*Not*I, pIRES2-*Xho*I-ATG-SmBiT-*Sal*I and pIRES2-*Bam*HI-SmBiT-TAG-*Not*I vector, respectively.

### EXAMPLE 29: Preparation of pIRES2-XhoI-ATG-LgBiT-SalI-LBD(121-427 aa)-TAG-NotI, pIRES2-XhoI-ATG-LgBiT-SalI-NHPMLMNLLKDN-TAG-NotI, pIRES2-XhoI-ATG-LBD(121-427 aa)-BamHI-LgBiT-TAG-NotI, pIRES2-XhoI-ATG-NHPMLMNLLKDN-BamHI-LgBiT-TAG-NotI, pIRES2-XhoI-ATG-SmBiT-SalI-LBD(121-427 aa)-TAG-NotI, pIRES2-XhoI-ATG-SmBiT-SalI-NHPMLMNLLKDN-TAG-NotI, pIRES2-XhoI-ATG-LBD(121-427 aa)-BamHI-SmBiT-TAG-NotI and pIRES2-XhoI-ATG-NHPMLMNLLKDN-BamHI-SmBiT-TAG-NotI vectors

*Xho*I-ATG-LBD (121-427 aa)-*Bam*HI and *Sal*I-LBD(121-427 aa)-TAG-*Not*I were amplified by PCR using primer 61 and 62 (SEQ ID NO: 61 and 62), primer 63 and 64 (SEQ ID NO: 63 and 64), respectively, with pCR-BluntII-TOPO-hVDR produced by EXAMPLE 1 as template. PCRs were performed under the conditions: Reaction 1 (94°C 2 minutes later, 98°C 10 seconds, 56°C 30 seconds, 68°C 40 seconds, 30 cycles; template DNA 10 ng, MgSO₄ 1.5 mM, dNTPs 0.2 mM, KOD-plus neo-DNA polymerase (TOYOBO) 0.2 U, 10 x PCR Buffer for KOD plus neo), primer 0.3 µM each, and final volume 10 µL. Electrophoresis of 1 µL of PCR product with 1% agarose gel resulted in a specific amplification at the site of interest (approximately 1000 bps). The PCR product was treated with restriction enzyme *Xho*I/*Bam*HI*, Sal*I/*Not*I*,* respectively, and after electrophoresis DNA fragment of interest was extracted from agarose gel and purified using QIAquick Gel Extraction Kit (QIAGEN). 10 µM, 10×PNK buffer, 1 mM ATP, 1 U T4 PNK of primer 65 and 66 (SEQ ID NO: 65 and 66) and primer 67 and 68 (SEQ ID NO: 67 and 68), respectively, were mixed and reacted at 37°C for one and a half hours, 95°C for 10 minutes, 75°C for 10 minutes and then lowering the temperatures to 37°C to produce double strand fragment *Sal*I-NHPMLLLKDN-TAG-*Not*I and *Xho*I-ATG-NHPMLMNLLKDN-*Bam*HI. These were designated as insert fragments.

Insert fragments described above were incorporated into *Sal*I*lNot*I sites of pIRES2-*Xho*I-ATG-LgBiT-*Sal*I and pIRES2-*Xho*I-ATG-SmBiT-*Sal*I vectors and *Xho*I/*Bam*HI sites of pIRES2-*Bam*HI-LgBiT-TAG-*Not*I and pIRES2-*Bam*HI-SmBiT-TAG-*Not*I vectors prepared by EXAMPLE 28, and the resulting plasmid DNAs were named pIRES2-*Xho*I-ATG-LgBiT-*Sal*I-LBD(121-427 aa)-TAG-*Not*I, pIRES2-*Xho*I-ATG-LgBiT-*Sal*I-NHPMLMNLLKDN-TAG-*Not*I, pIRES2-*Xho*I-ATG-SmBIT-*Sal*I-LBD(121-427 aa)-TAG-*Not*I, pIRES2-*Xho*I-ATG-LBD(121-427 aa)-*Bam*HI-LgBiT-TAG-*Not*I, pIRES2-*Xh*oI-ATG-NHPMLMNLLKDN-*Bam*HI-LgBiT-TAG-*Not*I, pIRES2-*Xho*I-ATG-LBD(121-427 aa)-*Bam*HI-SmBiT-TAG-*Not*I, pIRES2-*XHo*I-ATG-NHPMLMNLLKDN-*Bam*HI-SmBiT-TAG-*Not*I and pIRES2-*Xho*I-ATG-SmBiT-*Sal*I-NHPMLMNLLKDN-TAG-*Not*I vectors.

SEQ ID NO: 61:
   5'-ATCTCGAGatgCGGCCCAAGCTGTCTGAGGAGCAGCAG-3 '
SEQ ID NO: 62:
   5'-aattGGATCCggagatctcattgccaaacacttcg-3'
SEQ ID NO: 63:
   5'-atatgtcgaccggcccaagctgtctgaggagcagc-3'
SEQ ID NO: 64:
   5'-atatgcggccgctcaggagatctcattgccaaacac-3'
SEQ ID NO: 65:
   5'-tcgacaaccacccgatgctcatgaaccttcttaaagataattaagc-3'
SEQ ID NO: 66:
   5'-ggccgcttaattatctttaagaaggttcatgagcatcgggtggttg-3'
SEQ ID NO: 67:
   5'-tcgagatgaaccacccgatgctcatgaaccttcttaaagataatg-3'
SEQ ID NO: 68:
   5'-gatccattatctttaagaaggttcatgagcatcgggtggttcatc-3'

### EXAMPLE 30: Preparation of pIRES2-XhoI-ATG-LgBiT-(GGGGS)×3-SalI-NHPMLMNLLKDN-TAG-NotI and pIRES2-XhoI-ATG-LBD(121-427 aa)-BamHI-(GGGGS)×3-SmBiT-TAG-NotI vectors

Primer 69 and 70 (SEQ ID NO: 69 and 70) and primer 71 and 72 (SEQ ID NO: 71 and 72) were mixed with 10 µM, 10×PNK buffer, 1 mM ATP and 1 U T4 PNK and reacted at 37°C for one and a half hours, 95°C for 10 minutes, 75°C for 10 minutes and then lowering the temperatures to 37°C to prepare double strand fragments *Xho*I-(GGGGS)×3-*Sal*I and *Bam*HI-(GGGGS)×3-BglII). Double strand fragments *Xho*I-(GGGGS)×3-*Sal*I and *Bam*HI-(GGGGS)×3-BglII) was respectively incorporated into *Sal*I or *Bam*HI sites of pIRES2-*Xho*I-ATG-LgBiT-*Sal*I-NHPMLMNLLKDN-TAG-*Not*I and pIRES2*-Xho*I-ATG-LBD(121-427 aa)-*Bam*HI-SmBiT-TAG-*Not*I vectors prepared in EXAMPLE 29, and the resulting plasmid DNAs were named pIRES2-*Xho*I-ATG-LgBiT-(GGGGS)×3-*Sal*I-NHPMLMNLLKDN-TAG-*Not*I and pIRES2-*Xho*I-ATG-LBD(121-427 aa)*-Bam*HI-(GGGGS)×3-SmBiT-TAG-*Not*I vectors. 
SEQ ID NO: 69:
5'-tcgagGGAGGTGGGGGTAGTGGAGGGGGAGGTAGCGGTGGCGGTGGTAGTg-
3'
SEQ ID NO: 70:
   5'-tcgacACTACCACCGCCACCGCTACCTCCCCCTCCACTACCCCCACCTCCc-3'

SEQ ID NO: 71:
5'-gatccGGTGGCGGTGGTAGTGGGGGCGGAGGTAGCGGAGGTGGGGGTAGTa-
3'

SEQ ID NO: 72:
   5'-gatctACTACCCCCACCTCCGCTACCTCCGCCCCCACTACCACCGCCACCg-3'

### EXAMPLE 31: Preparation of pIRES2-XhoI-ATG-[LBD(121-427 aa)R274L]-BamHI-SmBiT-TAG-NotI vector

*Xho*I-ATG-LBD(121-427 aa)*-Bam*HI was amplified by PCR using primer 61 and 62 (SEQ ID NO: 61 and 62) with pIRES2-LBD(R274L)-LucC vector produced in EXAMPLE 8 as template. PCR were performed under the conditions: Reaction 1 (94°C 2 minutes later, 98°C 10 seconds, 56°C 30 seconds, 68°C 40 seconds, 30 cycles; template DNA 10 ng, MgSO₄ 1.5 mM, dNTPs 0.2 mM, KOD-plus neo-DNA polymerase (TOYOBO) 0.2 U, 10 x PCR Buffer for KOD plus neo), primer 0.3 µM each, and final volume 10 µL. Electrophoresis of 1 µL of the PCR product with 1% agarose gel resulted in a specific amplification at the site of interest (approximately 1000 bps). The PCR product was treated with restriction enzyme *Xho*I/*Bam*HI and after electrophoresis DNA fragment of interest was extracted from agarose gel and purified using QIAquick Gel Extraction Kit (QIAGEN). The insert fragment described above were incorporated into *Xho*I/*Bam*HI sites of pIRES2-*Bam*HI-SmBiT-TAG-*Not*I vector produced by EXAMPLE 28, and the resulting plasmid DNA was named pIRES2-*Xho*I-ATG-[LBD(121-427 aa)R274L]-*Bam*HI-SmBiT-TAG-*Not*I vector.

### EXAMPLE 32: Preparation of pIRES2-XhoI-ATG-[LBD(121-427 aa Δ165-215 aa)]-BamHI-SmBiT-TAG-NotI vector

*Xho*I-ATG-[LBD(121-427 aa Δ165-215 aa)]-*Bam*HI was amplified by PCR using primer 61 and 62 (SEQ ID NO: 61 and 62) with pIRES2-LucN-VDNHPMLMNLLKDN-(GGGGS)×3-LBD(Δ165-215 aa)-LucC produced by EXAMPLE 7 as template. PCR was performed under the conditions: Reaction 1 (94°C 2 minutes later, 98°C 10 seconds, 56°C 30 seconds, 68°C 40 seconds, 30 cycles; template DNA 10 ng, MgSO₄ 1.5 mM, dNTPs 0.2 mM, KOD-plus neo-DNA polymerase (TOYOBO) 0.2 U, 10 x PCR Buffer for KOD plus neo), primer 0.3 µM each, and final volume 10 µL. Electrophoresis of 1 µL of the PCR product with 1% agarose gel resulted in a specific amplification at the site of interest (approximately 1000 bps). The PCR product was treated with restriction enzyme *Xho*I/*Bam*HI and after electrophoresis DNA fragment of interest was extracted from agarose gel and purified using QIAquick Gel Extraction Kit (QIAGEN). The above-described insert fragment was incorporated into *Xho*I/*Bam*HI sites of pIRES2-*Bam*HI-SmBiT-TAG-*Not*I vector produced by EXAMPLE 28, and the resulting plasmid DNA was named pIRES2-*Xho*I-ATG-[LBD(121-427 aa Δ165-215 aa)]-*Bam*HI-SmBiT-TAG-*Not*I vector.

### EXAMPLE 33: Preparation of pIRES2-XhoI-ATG-[LBD(121-427 aa Δ165-215 aa)R274L]-BamHI-SmBiT-TAG-NotI vector

PCR was performed using primer 19 and 20 (SEQ ID NO: 19 and 20) and pIRES2-*Xho*I-ATG-[LBD(121-427 aa Δ165-215 aa)R274L]-*Bam*HI-SmBiT-TAG-*Not*I vector prepared in EXAMPLE 32 as template. PCR was performed at 94°C 2 minutes later, 98°C 10 seconds, 60°C 30 seconds, 68°C 3 min 30 seconds, 16 cycles; template DNA 10 ng, MgSO₄ 1.5 mM, dNTPs 0.2 mM, KOD-plus neo-DNApolymerase (TOYOBO) 0.2 U, 10 x PCR Buffer for KOD plus neo, primer 0.3 µM each, final volume 20 µL. The PCR product was treated with 5 U of restriction enzyme *Dpn*I at 37°C for 3 hours, then purified by ethanol precipitation and dissolved in 5 µl of ultrapure water. *E.coli* HST08 strain was then transformed with calcium chloride method and applied to an LB agar medium containing 30 µg/mL kanamycin. Several of the obtained *E.coli* colony were selected, inoculated into 2 mL of LB liquid culture medium (containing 30 µg/mL of kanamycin), and shaking culture was performed at 37°C and 200 rpm for 16 hours. The plasmid was then extracted using QIAprep Spin Miniprep Kit (QIAGEN) and the obtained plasmid DNA was confirmed by sequencing. The resulting plasmid DNA was named pIRES2-*Xho*I-ATG-[LBD(121-427 aa Δ165-215 aa)R274L]-*Bam*HI-SmBiT-TAG-*Not*I vector.

### EXAMPLE 34: Preparation of pIRES2-XhoI-ATG-LBD(121-427 aa)-BamHI-SmBiT-EcoRI-LgBiT-SalI-NHPMLMNLLKDN-TAG-NotI vector

*Xho*I-ATG-LBD(121-427 aa)-*Bam*HI-SmBiT was amplified by PCR using primer 25 and 73 (SEQ ID NO: 25 and 73) with pIRES2-*Xho*I-ATG-LBD(121-427 aa)-*Bam*HI-SmBiT-TAG-*Not*I produced in EXAMPLE 29 as template. LgBiT-*Sal*I-NHPMLMNLLKDN-TAG was amplified by using primer 74 and 24 (SEQ ID NO: 74 and 24) with pIRES2-*Xho*I-ATG-LgBiT-*Sal*I-NHPMLMNLLKDN-TAG-*Not*I as template. PCR was performed at 94°C 2 minutes later, 98°C 10 seconds, 56°C 30 seconds, 68°C 30 seconds, 30 cycles ; template DNA 10 ng, MgSO₄ 1.5 mM, dNTPs 0.2 mM, KOD-plus neo-DNA polymerase (TOYOBO) 1 U, 10 x PCR Buffer for KOD plus neo, primer 0.3 µM each, final volume 20 µL. 1 µl of each PCR product (*Xho*I-ATG-LBD (121-427 aa)-*Bam*HI-SmBiT and LgBiT-*Sal*I-NHPMLMNLLKDN-TAG) was amplified and confirmed by electrophoresis. Then *Xho*I-ATG-LBD(121-427 aa)-*Bam*HI-SmBiT and LgBiT-*Sal*I-NHPMLMNLLKDN-TAG were ligated by overlap-PCR by using a mixture of 20 ng of each PCR product and primer 25 and 24 (SEQ ID NO: 25 and 24). PCR was performed at 94°C 2 minutes later, 98°C 10 seconds, 56°C 30 seconds, 68°C 60 seconds, 30 cycles; PCR product each 20 ng, MgSO₄ 1.5 mM, dNTPs 0.2 mM, KOD-plus neo-DNA polymerase (TOYOBO) 1 U, 10 x PCR Buffer for KOD plus neo, primer each 0.3 µM, final volume 20 µL. Electrophoresis of the PCR product revealed a specific amplification at the site of interest (approximately 1.5 kb). The PCR product was cloned into pCR Blunt II-TOPO vectors by the methods described above and after sequence analysis, the plasmid DNA was named pCR Blunt II-TOPO-*Xho*I-ATG-LBD(121-427 aa)-*Bam*HI-SmBiT-*Eco*RI-LgBiT-*Sal*I-NHPMLMNLLKDN-TAG. The insert fragment was then excised by *Xho*I/*Sal*I and incorporated into *Xho*I*lSal*I sites of pIRES2-*Xho*I-ATG-LgBiT-*Sal*I-NHPMLMNLLKDN-TAG-*Not*I, and the resulting plasmid DNA was named pIRES2-*Xho*I-ATG-LBD(121-427 aa)-*Bam*HI-SmBiT-*Eco*RI-LgBiT-*Sal*I-NHPMLMNLLKDN-TAG*-Not*I*.*

SEQ ID NO: 73
5'-
CAACGAAATCTTCGAGTGTGAAGACgaattcCAGAATCTCCTCGAACAGCCGGT
AG-3'

SEQ ID NO: 74
5'-
CTACCGGCTGTTCGAGGAGATTCTGgaattcGTCTTCACACTCGAAGATTTCGTT
G-3'

### EXAMPLE 35: Preparation of pIRES2-XhoI-ATG-LgBiT-SalI-NHPMLMNLLKDN-EcoRI-LBD(121-427 aa)-BamRI-SmBiT-TAG-NotI

*Xho*I-LgBiT-*Sal*I-NHPMLMNLLKDN was amplified by PCR using primer 53 and 75 (SEQ ID NO: 53 and 75) with pIRES2-*Xho*I-ATG-LgBiT-*Sal*I-NHPMLMNLLKDN-TAG-*Not*I prepared in EXAMPLE 29 as template. LBD(121-427 aa)-*Bam*HI-SmBiT-TAG was amplified using primer 76 and 60 (SEQ ID NO: 76 and 60) with pIRES2-*Xho*I-ATG-LBD(121-427 aa)-*Bam*HI-SmBiT-TAG-*Not*I as template. PCR was performed at 94°C 2 minutes later, 98°C 10 seconds, 56°C 30 seconds, 68°C 30 seconds, 30 cycles ; template DNA 10 ng, MgSO₄ 1.5 mM, dNTPs 0.2 mM, KOD-plus neo-DNApolymerase (TOYOBO) 1 U, 10 x PCR Buffer for KOD plus neo, primer 0.3 µM each, final volume 20 µL. After amplification of the PCR product (*Xho*I-LgBiT-*Sal*I-NHPMLMNLLKDN and LBD(121-427 aa)-*Bam*HI-SmBiT-TAG) was confirmed by electrophoresis of 1 µl of each product, *Xho*I-LgBiT-*Sal*I-NHPMLMNLLKDN and LBD(121-427 aa)*-Bam*HI-SmBiT-TAG were ligated by overlap-PCR by using a mixture of primer 53 and 60 (SEQ ID NO: 53 and 60) and 20 ng each of the PCR product. PCR was performed at 94°C 2 minutes later, 98°C 10 seconds, 56°C 30 seconds, 68°C 60 seconds, 30 cycles; PCR product each 20 ng, MgSO₄1.5 mM, dNTPs 0.2 mM, KOD-plus neo-DNA polymerase (TOYOBO) 1 U, 10 x PCR Buffer for KOD plus neo, primer each 0.3 µM, final volume 20 µL. Electrophoresis of the PCR product revealed a specific amplification at the site of interest (approximately 1.5 kb). The PCR product was cloned into pCR Blunt II-TOPO vector by the methods described above and after sequence analysis, the plasmid DNA was named pCR Blunt II-TOPO-*Xho*I-LgBiT-*Sal*I-NHPMLMNLLKDN-*Eco*RI-LBD(121-427 aa)-*Bam*HI-SmBiT-TAG. The insert fragment was then excised by *Xho*I/*Bam*HI and incorporated into *Xho*I/*Bam*HI site of pIRES2-*Xho*I-ATG-LBD(121-427 aa)*-Bam*HI-SmBiT-TAG-*Not*I, and the resulting plasmid DNA was named pIRES2-*Xho*I-LgBiT-*Sal*I-NHPMLMNLLKDN-*Eco*RI-LBD(121-427 aa)-*Bam*HI-SmBiT-TAG-*Not*I.

SEQ ID NO: 75
   5'-gctgctcctcagacagcttgggccgGAATTCattatctttaagaaggttcatgagc-3'
SEQ ID NO: 76
   5'-gctcatgaaccttcttaaagataatGAATTCcggcccaagctgtctgaggagcagc-3'

### EXAMPLE 36: pIRES2-XhoI-ATG-LgBiT-SalI-NHPMLMNLLKDN-EcoRI-(GGGGS)×3-EcoRI-LBD(121-427 aa)-BamHI-SmBiT-TAG-NotI and pIRES2-XhoI-LgBiT-SalI-NHPMLMNLLKDN-EcoRI-(GGGGS)×3-EcoRI-LBD(121-427 aa)-BamHI-SmBiT-TAG-NotI

Primer 77 and 78 (SEQ ID NO: 77 and 78) 10 µM, 10×PNK buffer, 1 mM ATP, 1 U T4 PNK were mixed and reacted at 37°C for one and a half hours, 95°C for 10 minutes, 75°C for 10 minutes and then lowering the temperatures to 37°C to produce double strand fragment *Eco*RI-(GGGGS)×3-*Eco*RI): *Eco*RI*-*(GGGGS)×3-*Eco*RI was incorporated into *Eco*RI sites of pIRES2-*Xho*I-ATG-LBD(121-427 aa)-*Bam*HI-SmBiT-*Eco*RI-LgBiT-*Sal*I-NHPMLMNLLKDN-TAG-*Not*I and pIRES2-*Xho*I-LgBiT-*Sal*I-NHPMLMNLLKDN-*Eco*RI-LBD(121-427 aa)-*Bam*HI-SmBiT-TAG-*Not*I produced in EXAMPLE 34 and 35, and the resulting plasmid DNAs were named pIRES2-*Xho*I-ATG-LgBiT-*Sal*I-NHPMLMNLLKDN-*Eco*RI-(GGGGS)×3-*Eco*RI-LBD(121-427 aa)-*Bam*HI-SmBiT-TAG-*Not*I and pIRES2-*Xho*I-LgBiT-*Sal*I-NHPMLMNLLKDN-*Eco*RI-(GGGGS)×3-*Eco*RI-LBD(121-427 aa)-*Bam*HI-SmBiT-TAG-*Not*I. 
SEQ ID NO: 77
5'-aattcGGAGGTGGCGGAAGCGGTGGCGGCGGGAGTGGAGGAGGCGGTAGCg-
3'
 SEQ ID NO: 78
5'-aattcGCTACCGCCTCCTCCACTCCCGCCGCCACCGCTTCCGCCACCTCCg-3'

### EXAMPLE 37: Preparation of pIRES2-XhoI-ATG-LgBit-SalI-HVEMHPLLMGLLMESQWGA-TAG-NotI, pIRES2-XhoI-ATG-LgBit-SalI-DAASKHKQLSELLRGGSGSS-TAG-NotI, pIRES2-XhoI-ATG-LgBit-SalI-GHEPLTLLERLLMDDKQAV-TAG-NotI, pIRES2-XhoI-ATG-LgBit-SalI-KVSQNPILTSLLQITGNGG-TAG-NotI, pIRES2-XhoI-ATG-LgBit-SalI-YSQTSHKLVQLLTTTAEQQ-TAG-NotI, pIRES2-XhoI-ATG-LgBit-SalI-ESKDHQLLRYLLDKDEKDL-TAG-NotI, pIRES2-XhoI-ATG-LgBit-SalI-QAQQKSLLQQLLTE-TAG-NotI, pIR-ES2-XhoI-ATG-LgBit-SalI-EAEEPSLLKKLLLAPANTQ-TAG-NotI, pIRES2-XhoI-ATG-LgBit-SalI-LPYEGSLLLKLLRAPVEEV-TAG-NotI, pIRES2-XhoI-ATG-LgBit-SalI-HVYQHPLLLSLLSSEHESG-TAG-NotI and pIRIES2-XhoI-ATG-LgBit-SalI-HKILHRLLQEG-TAG-NotI vectors

Primer 79 and 80 (SEQ ID NO: 79 and 80), primer 81 and 82 (SEQ ID NO: 81 and 82), primer 83 and 84 (SEQ ID NO: 83 and 84), primer 85 and 86 (SEQ ID NO: 85 and 86), primer 87 and 88 (SEQ ID NO: 87 and 88), primer 89 and 90 (SEQ ID NO: 89 and 90), primer 91 and 92 (SEQ ID NO: 91 and 92), primer 93 and 94 (SEQ ID NO: 93 and 94), primer 95 and 96 (SEQ ID NO: 95 and 96), primer 97 and 98 (SEQ ID NO: 97 and 98), primer 99 and 100 (SEQ ID NO: 99 and 100), 10 µM, 10×PNK buffer, 1 mM ATP, and 1 U T4 PNK were mixed and reacted at 37°C for one and a half hours, 95°C for 10 minutes, 75°C for 10 minutes and then lowering the temperatures to 37°C to produce double-stranded fragments *Sal*I-HVEMHPLLMGLLMESQWGA-TAG-TVM, *Sal*I-DAASKHKQLSELLRGGSGSS-TAG-*Not*I, *Sal*I-GHEPLTLLERLLMDDKQAV-TAG-*Not*I, *Sal*I-KVSQNPILTSLLQITGNGG-TAG-*Not*I, *Sal*I-YSQTSHKLVQLLTTTAEQQ-TAG-*Not*I, *Sal*I-ESKDHQLLRYLLDKDEKDL-TAG-*Not*I, *Sal*I-QAQQKSLLQQLLTE-TAG-*Not*I, *Sal*I-EAEEPSLLKKLLLAPANTQ-TAG-*Not*I, *Sal*I-LPYEGSLLLKLLRAPVEEV-TAG-*Not*I, *Sal*I-HVYQHPLLLSLLSSEHESG-TAG-*Not*I, *Sal*I-HKILHRLLQEG-TAG-*Not*I, respectively. These double-stranded fragments were incorporated respectively into *Sal*I/*Not*I sites of pIRES2-*Xho*I-ATG-LgBiT-*Sal*I produced in EXAMPLE 28, and the resulting plasmid DNAs were named pIRES2-*Xho*I-ATG-LgBit-*Sal*I-HVEMHPLLMGLLMESQWGA-TAG-*Not*I, pIRES2-*Xho*I-ATG-LgBit-*Sal*I-DAASKHKQLSELLRGGSGSS-TAG-*Not*I, pIRES2-*Xho*I-ATG-LgBit-*Sal*I-GHEPLTLLERLLMDDKQAV-TAG-*Not*I, pIRES2-*Xho*I-ATG-LgBit-*Sal*I-KVSQNPILTSLLQITGNGG-TAG-*Not*I, pIRES2-*Xho*I-ATG-LgBit-*Sal*I-YSQTSHKLVQLLTTTAEQQ-TAG-*Not*I, pIRES2-*Xho*I-ATG-LgBit-*Sal*I-ESKDHQLLRYLLDKDEKDL-TAG-*Not*I, pIRES2-*Xho*I-ATG-LgBit-*Sal*I-QAQQKSLLQQLLTE-TAG-*Not*I, pIRES2-*Xho*I-ATG-LgBit-*Sal*I-EAEEPSLLKKLLLAPANTQ-TAG-*Not*I, pIRES2-*Xho*I-ATG-LgBit-*Sal*I-LPYEGSLLLKLLRAPVEEV-TAG-*Not*I, piRES2-*Xho*I-ATG-LgBit-*Sal*I-HVYQHPLLLSLLSSEHESG-TAG-*Not*I and pIRES2-*Xho*I-ATG-LgBit-*Sal*I-HKILHRLLQEG-TAG-*Not*I vectors.

SEQ ID NO: 79
   5'-TCGACcatgtcgagatgcacccactcctgatgggacttctcatggaatcgcagtggggcgccTAGC-3'
SEQ ID NO: 80
   5'-GGCCGCTAggcgccccactgcgattccatgagaagtcccatcaggagtgggtgcatctcgacatgG-3'
SEQ ID NO: 81
   5'-TCGACgctgcttccaaacataaacaactgtcggagcttctacgaggaggcagcggctctagtTAGC-3'
SEQ ID NO: 82
   5'-GGCCGCTAactagagccgctgcctcctcgtagaagctccgacagttgtttatgtttggaagcagcG-3'
SEQ ID NO: 83
   5'-TCGACggacacgaaccactcacgcttctggaacgactgctaatggacgacaagcaggcggtcTAGC-3'
SEQ ID NO: 84
   5'-GGCCGCTAgaccgcctgcttgtcgtccattagcagtcgttccagaagcgtgagtggttcgtgtccG-3'
SEQ ID NO: 85
   5'-TCGACagcaaggtgtctcagaacccaattcttaccagtttgttgcaaatcacagggaacggggggTAGC-3'
SEQ ID NO: 86
   5'-GGCCGCTAccccccgttccctgtgatttgcaacaaactggtaagaattgggttctgagacaccttgctG-3'
SEQ ID NO: 87
   5'-TCGACaaatactctcaaaccagtcacaaactagtgcagcttttgacaacaactgccgaacagcagTAGC-3'
SEQ ID NO: 88
   5'-GGCCGCTActgctgttcggcagttgttgtcaaaagctgcactagtttgtgactggtttgagagtatttG-3'
SEQ ID NO: 89
   5'-TCGACaaagaatcaaaagaccatcagctcctacgctatcttttagataaagatgagaaagatttaTAGC-3'
SEQ ID NO: 90
   5'-GGCCGCTAtaaatctttctcatctttatctaaaagatagcgtaggagctgatggtcttttgattctttG-3'
SEQ ID NO: 91
   5'-TCGACcaggcccagcagaagagcctccttcagcagctactgactgaaTAGC-3'
SEQ ID NO: 92
   5'-GGCCGCTAttcagtcagtagctgctgaaggaggctcttctgctgggcctgG-3'
SEQ ID NO: 93
   5'-tcgacgaggcagaagagccgtctctacttaagaagctcttactggcaccagccaacactcagtagc-3'
SEQ ID NO: 94
   5'-ggccgctactgagtgttggctggtgccagtaagagcttcttaagtagagacggctcttctgcctcg-3'
SEQ ID NO: 95
   5'-tcgacctaccatacgaaggcagtctacttctcaagctacttagagccccagttgaggaggtgtaggc-3'
SEQ ID NO: 96
   5'-ggccgcctacacctcctcaactggggctctaagtagcttgagaagtagactgccttcgtatggtagg-3'
SEQ ID NO: 97
   5'-tcgaccacgtttatcagcatcctctacttctcagcctacttagctcagagcacgagtcaggttagc-3'
SEQ ID NO: 98
   5'-ggccgctaacctgactcgtgctctgagctaagtaggctgagaagtagaggatgctgataaacgtgg-3'
SEQ ID NO: 99
   5'-tcgaccataaaattctacaccggctcttacaggagggttagc-3'
SEQ ID NO: 100
   5'-ggccgctaaccctcctgtaagagccggtgtagaattttatgg-3'

### EXAMPLE 38: Cloning into E.coli expression vectors

pIRES2-*Xho*I-ATG-LgBiT-*Sal*I-NHPMLMNLLKDN-TAG-*Not*I and pIRES2-*Xho*I ATG-LBD(121-427 aa)-*Bam*HI-SmBiT-TAG-*Not*I prepared in EXAMPLE 29, pIRES2-*Xho*I-ATG-[LBD(121-427 aa)R274L]-*Bam*HI-SmBiT-TAG-*Not*I prepared in EXAMPLE 31, pIRES2-*Xho*I-LgBiT-*Sal*I-NHPMLMNLLKDN-*Eco*RI-LBD(121-427 aa)-*Bam*HI-SmBiT-TAG-*Not*I produced in EXAMPLE 35 were treated with *Xho*I*lNot*I and insert fragments were excised and integrated into *Xho*I*lNot*I sites of pET-11d-N-6×His-*Xho*I/*Not*I vectors produced in EXAMPLE 19. The resulting plasmid DNAs were named pET-11d-N-6×His-*Xho*I-ATG-LgBiT-*Sal*I-NHPMLNINLLKDN-TAG-*Not*I, pET-11d-N-6×His-*Xho*I-ATG-LBD(121-427 aa)-*Bam*HI-SmBiT-TAG-*Not*I, pET-11d-N-6×His-*Xho*I-ATG-[LBD(121-427 aa)R274L]-*Bam*HI-SmBiT-TAG-*Not*I, and pET-11d-N-6×His-*Xho*I-LgBiT-*Sal*I-NHPMLMNLLKDN-*Eco*RI-LBD(121-427 aa)-*Bam*HI-SmBiT-TAG-*Not*I. Structures of biosensor produced in EXAMPLEs 27 to 38 are shown in Fig. 32.

### EXAMPLE 39: Light emission measurements in NanoLuciferase type biosensor expression cells

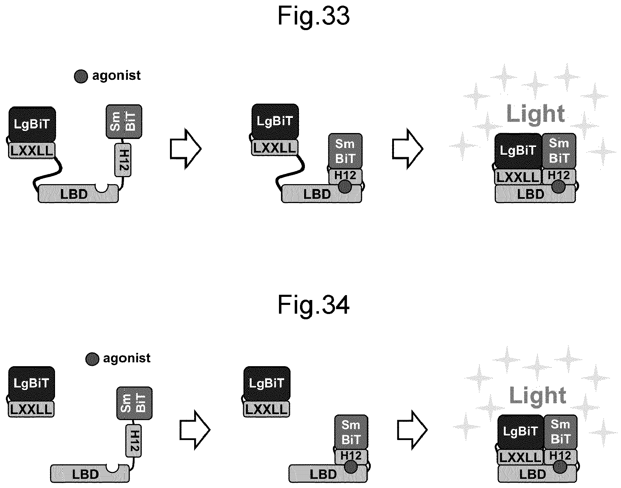

Plasmid DNAs encoding NanoLuciferase type of biosensor were transferred into COS-7 cells and plated at 96 well plate. The procedures described in EXAMPLE 9 and 10 were followed. Forty-eight hours after gene transfer, the medium was changed to phenol-red-free L-15 medium (Gibco), allowed to stand at room temperature for 30 minutes, and 60 minutes after addition of VDR agonist, 25(OH)D₃ or 1α,25(OH)₂D₃, light emission substrate solution (Nano-Glo Live Cell Assay System, Promega) was added and measured. Ethanol (EtOH) brought in used as a solvent for VDR agonist was at a final concentration of 0.1%. Microplate reader Infinite 200 Pro (TECAN) was used as an instrument for measuring light emission. Principle of variation in light emission of single molecular type and double molecular type biosensor after adding VDR agonist is shown in Figs. 33 and 34. For H12 described in Figs. 33 and 34, refer to the descriptions of Figs. 3 and 4 in EXAMPLE 11.

### EXAMPLE 40: Selection of double molecular type NanoLuciferase-type biosensor

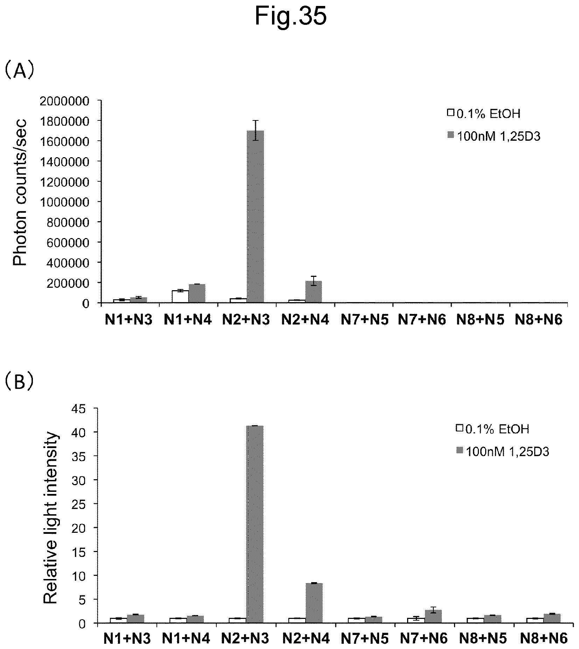

The light emission (Fig. 35(A)) and relative changes in light emission (Fig. 35(B)) 60 minutes after adding 100 nM 1α,25(OH)₂D₃ to COS-7 cells expressing biosensor in the combinations of Groups A and B or Groups C and D of Fig. 32 were compared. The relative change in light emission was obtained by dividing the amount of light emission at 60 minutes after addition of 100 nM 1α,25(OH)₂D₃ by the amount of light emission in the 0.1% EtOH addition group. In the case of mammal cell line, since the expression amount of proteins changes depending on experiments and light emission amount fluctuates depending on the expression amount, the relative change of light emission amount is described in parallel in order to correct this point. However, in *in vitro* assay system of *E.coli* expression system, which will be described later, there is no change in the expression amount of proteins, and the relative change of light emission amount and the relative change of light emission amount correspond to each other.

Combinations of N2+N3 showed the largest light emission and light emission relative changes after the addition of 100 nM 1α,25(OH)₂D₃, and the change was about 40 times larger. Binding of the 1α,25(OH)₂D₃ to the LBD in biosensor resulted in a structure change, followed by interaction of LgBiT and SmBiT during interaction between LXXLL sequence (NHPMLMNLLKDN) and the LBD, which resulted in restoration of light emission. Light intensity was more than 100 times higher than conventional firefly luciferase types.

### EXAMPLE 41: Performance Comparisons of biosensor using LgBiT-(GGGGS)×3-SalI-NHPMLMNLLKDN and LBD(121-427 aa)-BamHI-(GGGGS)×3-SmBiT

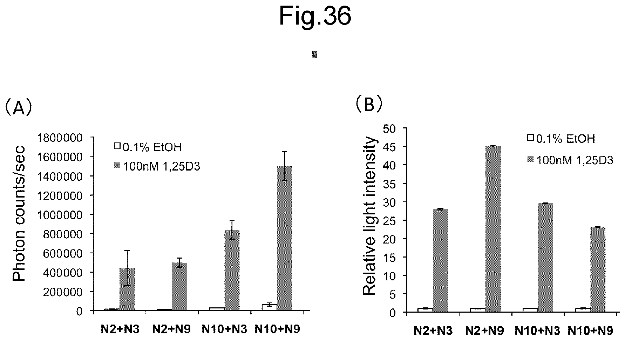

The light emission (Fig. 36(A) and relative changes of light emission (Fig. 36(B), 60 minutes after 100 nM 1α,25(OH)₂D₃ was added to COS-7 cells expressing biosensor were compared.

On the basis of the combinations of biosensor N2+N3 with large light emission and relative light intensity shown in EXAMPLE 40, N10 and N9 with (GGGGS3)×3 linkers inserted in each biosensor were prepared and compared with each other for the difference in light emission amounts. In N10+N9, the amount of light emission increased, but the relative change was comparable to that in N2+N3. On the other hand, for N2+N9, light emission amount was identified as N2+N3, but was highest for relative light intensity. These results suggest that the presence or absence of the (GGGGS3)×3 linker changes the distances and orientations of LgBiT and SmBiT in biosensor and affects light emission amount.

### EXAMPLE 42: Optimization of LXXLL Sequences in biosensor

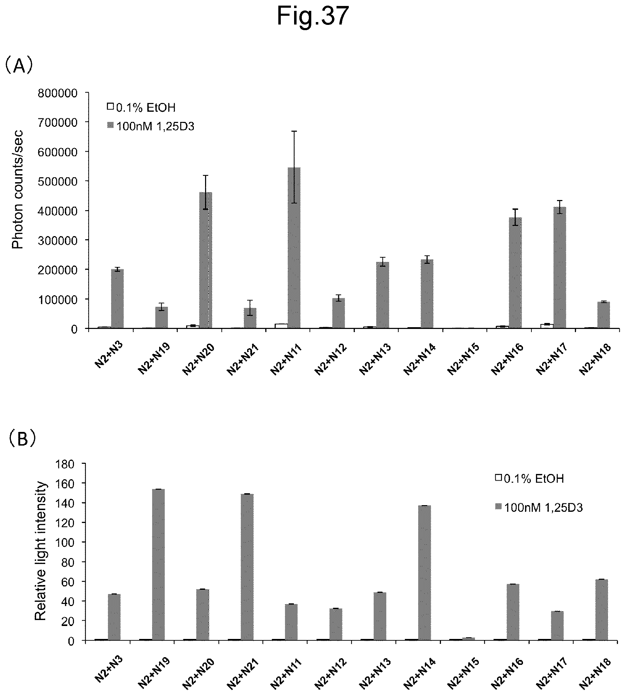

Based on the combination of N3+N2, a 100 nM 1α,25(OH)₂D₃ was added to COS-7 cells expressing N11-21 produced in EXAMPLE 37 and N2 respectively, and then light emission amount (Fig.37(A)) and relative change of light emission amount (Fig.37(B)) after 60 minutes were compared.

In N2+N20, N2+N11, N2+N16, and N2+N17, the amounts of light emission after adding 100 nM 1α,25(OH)₂D₃ were greater than N2+N3. Among them, there was no combination in which the relative change amount was larger than that of N2+N3, but all showed an increase of about 40 times, which was the same as that of N2+N3. On the other hand, combinations in which the relative change amount was larger than that of N2+N3 were N2+N19, N2+N21, and N2+N14. Among them, there was no combination in which light emission amount was larger than N2+N3, but the same degree of combination was N2+N14. These results suggest that N2+N20, N2+N11, N2+N16, N2+N17 and N2+N14 are likely to lead to increased sensitivities of biosensor. Considering the results of EXAMPLE 41, inserting a (GGGGS3)×3 linker between LgBiT and LXXLL sequence in these biosensor may further increase light emission amount and improve the performance.

### EXAMPLE 43: Optimization of LBD in double molecular type biosensor and Comparisons of R274L type biosensor

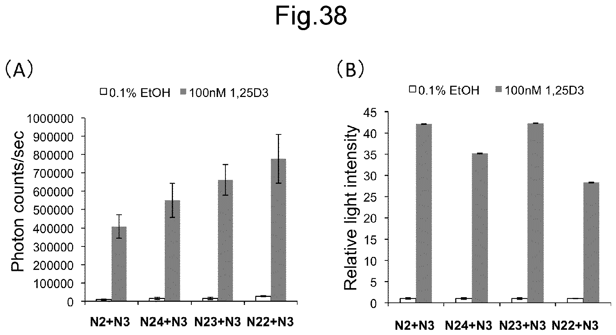

The light emission (Fig. 38(A)) and relative changes of light emission (Fig. 38(B)) 60 minutes after adding 100 nM 1α,25(OH)₂D₃ to COS-7 cells in which combinations of N22-24 produced by EXAMPLE 31-33 and N3, N22+N3, N23+N3, or N24+N3, were expressed respectively, were compared.

N23 and N24 with the deletion of 165-215 aa of LBDs in biosensor tended to have higher levels of light emission, whereas N2 and N22, which were not deleted, were similar when compared with the relative changes. In R274L mutation type LBD, since affinity of the 1α,25(OH)₂D₃ is lowered, the relative changes of N22 and N24 are smaller than those of N2 and N23.

### EXAMPLE 44: Light emission of single molecular type NanoLuciferase biosensor

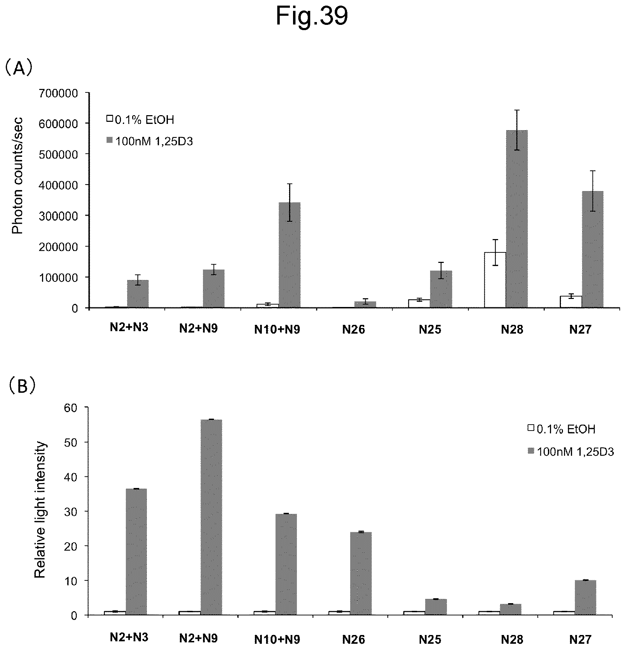

The light emission (Fig. 39(A)) and relative changes of light emission (Fig. 39(B)) 60 minutes after adding 100 nM 1α,25(OH)₂D₃ to COS-7 cells expressing single molecular type NanoLuciferase-type biosensor N25-28 produced by EXAMPLE 34-36, respectively, were compared.

N25 and N26 were smaller than double molecular type N2+N3 and N10+9 in terms of light emission amount after adding 100 nM 1α,25(OH)₂D₃, whereas N27 and N28 containing the (GGGGS3) ×3 linker were similar to or larger than double molecular type. However, when comparing the relative changes, N26 was increased by a factor of 20, while N25, N27, and N28 were increased by a factor of 10 or less, which were results inferior to those of double molecular type. Based on these facts, single molecular type biosensor is expected to be further improved and devised in comparison with double molecular type biosensor.

### EXAMPLE 45: Expression of LgBiT-SalI-NHPMLMNLLKDN, LBD(121-427 aa)-BamHI-SmBiT, [LBD(121-427 aa)R274L]-BamHI-SmBiT, and LgBiT-SalI-NHPMLMNLLKDN-EcoRI-LBD(121-427 aa)-BamHI-SmBiT proteins in E.coli BL21(DE)3 or SoluBL21 strain

*E.coli* BL21 (DE3) strain was transformed by calcium chloride method using pET-11d-N-6×His-*Xho*I-ATG-LgBiT-*Sal*I-NHPMLMNLLKDN-TAG-*Not*I, pET-11d-N-6×His-*Xho*I-ATG-LBD(121-427 aa)-*Bam*HI-SmBiT-TAG-*Not*I, pET-11d-N-6×His-*Xho*I-ATG-[LBD(121-427 aa)R-274L]-*Bam*HI-SmBiT-TAG-*Not*I and pET-11d-N-6×His-*Xho*I-LgBiT-*Sal*I-NHPMLMNLLKDN-*Eco*RI-LBD(121-427 aa)-*Bam*HI-SmBiT-TAG-*Not*I plasmid DNAs produced in EXAMPLE 38 and applied to an LB agar medium containing 100 µg/mL of ampicillin. Several of the obtained *E.coli* colony were selected, inoculated into 2 mL of LB liquid culture medium (containing 100 µg/mL of ampicillin), and shaking culture was performed at 37°C and 200 rpm for 12 hours. 2 mL of *E.coli* solution was succeeded in 500 mL of LB liquid culture medium (containing 100 µg/mL of ampicillin) and cultivated at 37°C to OD₆₀₀=0.4, and then 1 mM isopropyl-1-thio-β-D-galactopyranoside [IPTG (NACALAI TESQUE, INC.) was added and cultivated at 15°C for 3 hours to express proteins. After cultivation, centrifugal separation was performed at 4°C, 5000×g for 10 minutes to collect bacterial cells. Bacterial bodies were suspended in the solution containing 25 mM Tris-HCl, pH 7.4, 10 mM DTT, and protease cocktails of inhibitor (NACALAI TESQUE, Inc.) and then ultrasonic crushing was performed in ice for 15 sec×7 times. Thereafter, centrifugal separation was performed at 4°C, 20,000×g for 30 minutes, and supernatant was collected to obtain biosensor protein solutions N29, N30, N31 and N32.

### EXAMPLE 46: Luciferase Assays in in vitro System

After 40 µl/well of N29+N31, N30+N31, or N32 biosensor protein prepared in EXAMPLE 45 was dispensed into 96 well plate, 25(OH)D₃ or 1α,25(OH)₂D₃ was added at various concentrations, and incubated at room temperature for 5-10 minutes. Thereafter, 10 µl of light emission substrate solution (Nano-Glo Live Cell Assay System, Promega) was added and measured. Ethanol (EtOH) brought in, which is used as a solvent for VDR ligand, was at a final concentration of 1%. Microplate reader Infinite 200 Pro (TECAN) was used as an instrument for measuring light emission.

### EXAMPLE 47: Comparisons of double molecular type and single molecular type NanoLuciferase-type biosensor in In vitro Systems

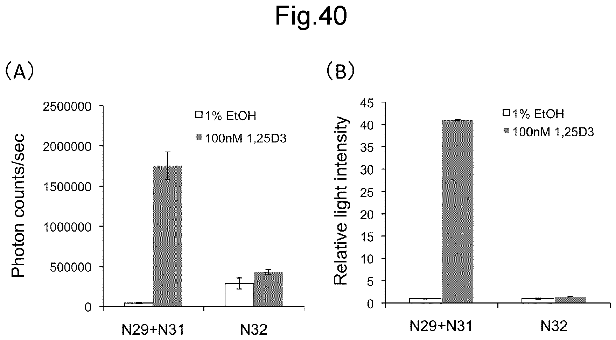

After plating double molecular type N29+N31 and single molecular type N32 biosensor to 96 well plate, light emission 10 minutes after adding 100 nM 1α,25(OH)₂D₃ was measured by the methods shown in EXAMPLE 46, and light emission amounts (Fig. 40(A)) and relative light intensity (Fig. 40(B)) were calculated and compared.

Double molecular type N29+N31 biosensor, similar to the results in mammal cell expression system, also had a large light emission amount after adding 100 nM 1α,25(OH)₂D₃, and about 40 times in light emission variation amount, but N32 biosensor in single molecular type did not increase light emission much. For this reason, double molecular type N29+N31 type biosensor was used in the subsequent experiments.

### EXAMPLE 48: Performance of double molecular type N29 + N31 biosensor

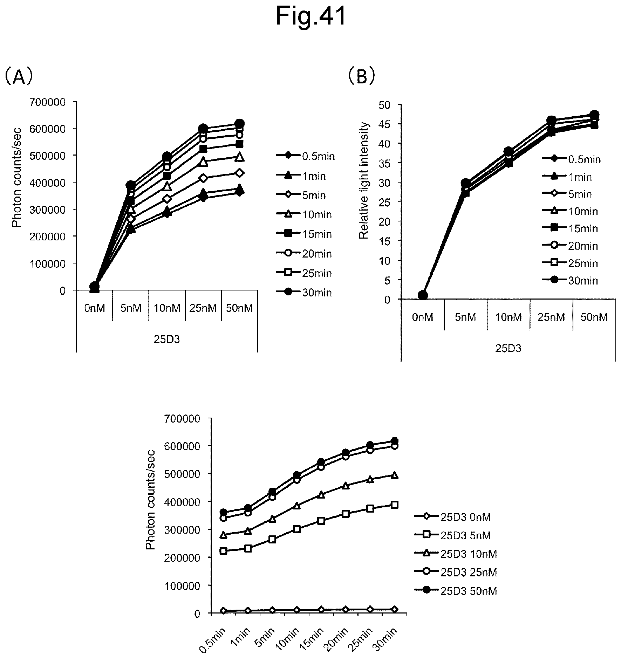

After plating double molecular type N29+N31 to 96 well plate, 0-50 nM of 25(OH)D₃ was added and incubated for 10 minutes at room temperature. Thereafter, 30 seconds, 1, 5, 10, 15, 20, 25, and 30 minutes after adding a light emission substrate solution, light emission was measured by the methods shown in EXAMPLE 46, and the amounts of light emission (Fig. 41(A)) and relative light intensity (Fig. 41(B)) were calculated and compared.

Light emission increased in a 25(OH)D₃ concentration-dependent manner, reaching maximal increase at around 50 nM. Light emission amount increases gradually after the light emission substrate solution is added, but light emission amounts at 25 and 30 minutes are almost the same, so it is considered to be maximal around 30 minutes (Fig. 41A). However, when comparing relative light intensity at the respective measurement points, the concentration curves agreed with each other, so that the concentration curves can be measured at any time from 0.5 to 30 minutes after the addition of substrate solution (Fig. 41B).

### EXAMPLE 48: Detection limit of double molecular-type N29+N31 biosensor to 25(OH)D₃

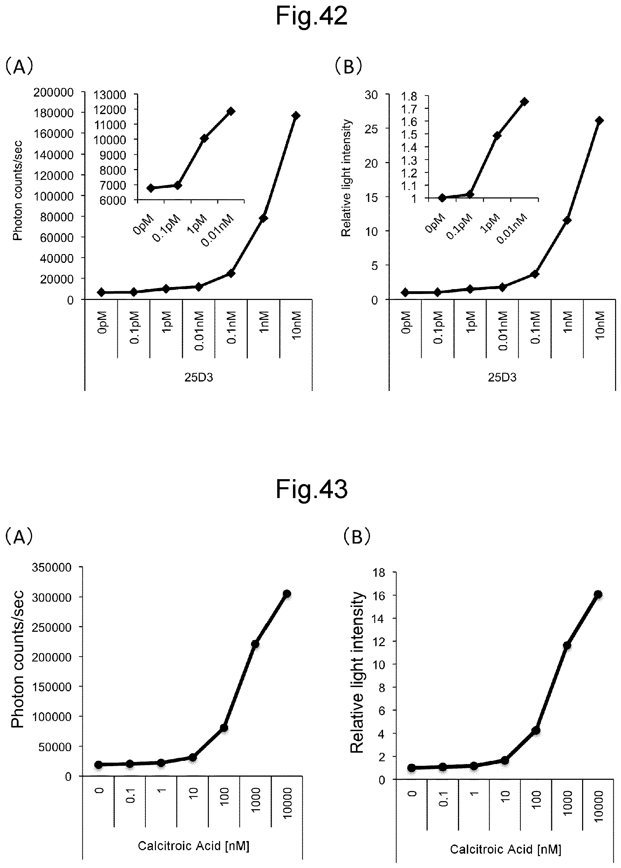

After plating double molecular type N29+N31 to 96 well plate, 0-10 nM of 25(OH)D₃ was added and incubated for 10 minutes at room temperature. Thereafter, 10 minutes after adding the light emission substrate solution, light emission was measured in the manner shown in EXAMPLE 46, and the amounts of light emission (Fig. 42(A)) and relative light intensity (Fig. 42(B)) were calculated and compared.

Detection limit is 1 pM because the amount of light emission increased from 0.1 pM 25(OH)D₃ in a concentration-dependent manner, with a relative change of about 1.5-fold at 1 pM.

### EXAMPLE 49: Detecting calcitroic acid with double molecular type N29+ N31 biosensor

Affinity of calcitroic acid, a metabolite of 1α,25(OH)₂D₃, was evaluated using double molecular type N29+N31 biosensor. After plating double molecular type N29+N31 to 96 well plate, 0-10000 nM calcitroic acid was added and incubated for 10 minutes at room temperature. Thereafter, 10 minutes after adding the light emission substrate solution, light emission was measured in the manner shown in EXAMPLE 46, and the amounts of light emission (Fig. 43(A)) and relative light intensity (Fig. 43(B)) were calculated and compared. As a result, it was clarified that affinity was extremely low compared with 25(OH)D₃ because calcitroic acid of 10 nM or more was required for increasing light emission. Calcitroic acid is considered negligible for detecting blood 25(OH)D₃ or 1α,25(OH)₂D₃ concentrations by the biosensor of the present invention, since it is metabolite contained in urine in trace amounts and VDR affinity of calcitroic acid is 1/1000 or less of 1α,25(OH)₂D₃.

### EXAMPLE 50: Vitamin D extracted from rat blood plasma metabolite

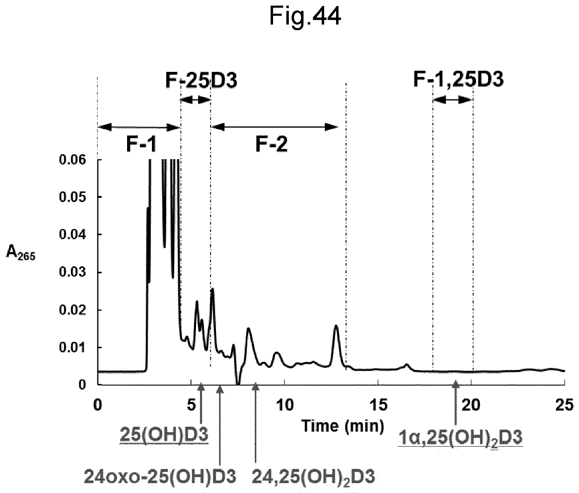

To 2.2 mL of blood plasma collected from rat, 2.2 mL of acetonitrile was added, mixed by inversion, and left for 10 minutes. After vigorous stirring, centrifugation was carried out at 1,500 g for 10 minutes and supernatant was dried. To the dry matter, 2 mL of ethyl acetate and 1 mL of distilled water were added, stirred vigorously, centrifuged at 1,500 g for 10 minutes, and ethyl acetate layer (upper layer) was transferred to separate tubes. To the aqueous layer (lower layer) was again added 2 ml of ethyl acetate, the same procedure was repeated again, and ethyl acetate layer was added to the tubes described above. The resulting ethyl acetate layer was dried, 30 µL of acetonitrile was added to the dry matter, centrifuged at 20,000 g for 10 minutes, and then 25 µL of supernatant was applied to normal phase HPLC. A fraction of 4.5 to 6.5 minutes of detection time corresponding to 25-hydroxyvitamin D3 (F-25D3), a fraction of 18.5 to 21.5 minutes corresponding to 1α, 25D3 (F-1, 25D3), and a fraction of 4.5 minutes from 0 minutes of detection time (F-1), and a fraction of 13.0 minutes from 6.5 minutes of detection time (F-2) were collected, respectively (see Fig. 44), and each fraction was dissolved in 20 µL of ethanol after drying. Of the resulting ethanolic solutions, 0.5 µL were analyzed by Nano Luc assays and 10 µL by LC/MS/MS. Normal phase HPLC conditions are as follows:

### Normal phase HPLC

Column: Zorbax RX-SIL (4.6 x 250 nm, 5 µm), Column temperature: 25°C
Eluent: hexane/isopropanol (9:1), flow rate: 0.7 mL/min, detector wavelength: 254 nm

Normal phase HPLC-chart of the rat blood plasma extract and the results of the fractions collected are shown in Fig. 44. Arrows below the horizontal axis indicate the times at which various vitamin D reference standards were detected.

### EXAMPLE 51: Determination of 25-hydroxyvitamin D3 and 1α,25-hydroxyvitamin D3 using LC/MS/MS

The dried F-25D3, F-1, 25D3 fractions were dissolved in 20 µL of ethanol solution, and to 10 µL of this solution, 10µL (0.5 ng) of 50 ng/mL (125 nM) of d6-25(OH)D3 ethanol solution as internal standard substance and 30 µL of 0.1 mg/mL of an ethyl acetate solution of DMEQ-TAD (4-[2-(6,7-dimethoxy-4-methyl-3-oxo-3, 4-dihydroquinoxalyl)ethyl]-1, 2,4-triazoline-3, 5-dione) (Wako) were added. After shaking lightly and allowing to stand at room temperature for 30 minutes, 30 µL of 0.1 mg/mL of DMEQ-TAD ethyl acetate was added again, shaking lightly, and left to stand at room temperature for 1 hour. Ethanol 200 µL was added, shaken, and left at room temperature for 10 minutes. After drying the resulting solution, the dried solution was dissolved in 40 µL of acetonitrile, of which 10 µL was applied to LC/MS/MS. The 25-hydroxyvitamin D3 reference standard was also subjected to derivatization by DMEQ-TAD in the same manner as described above, and then analyzed by LC/MS/MS. LC/MS/MS analytical conditions are as follows.

### LC/MS/MS

### <LC condition>

Columns: CAPCELL PAK C18 UG120 S5: 4. 6 mm i. d. ×250 mm
Mobile phase: A: distilled water +0.1% formic acid, B: acetonitrile +0.1% formic acid B-30% (0-5 seconds) →30-70% (5-34 minutes) → 100% (34-37 minutes)
Flow rate: 1.0 mL/min

### <MS/MS condition>

Device: API 3200-QTrap
Modes: APCI-positive
Detection: MRM (precursor ion/product ion)
   - DMEQ-TAD-25(OH)D₃ -m/z 746.6/468.3
   - DMEQ-TAD-1α, 25(OH)₂D₃- m/z 762.6/484.3
   - DMEQ-TAD-d6-25(OH)D₃---m/z 752.6/468.3 (internal standard)

Analytical results showed that the 25-hydroxyvitamin D3 concentration in 20 µl ethanolic solutions of dried F-25D₃ was 148 nM and the calculated blood concentration was 1.35 nM. On the other hand, 1α,25-hydroxyvitamin D3 concentrations in 20 µL ethanolic solutions of F-1,25D₃ dried product were below detection limit.

### EXAMPLE 52: Detecting 25-hydroxyvitamin D3 and 1α,25-hydroxyvitamin D3 in rat blood plasma using double molecular type N29+ N31 biosensor

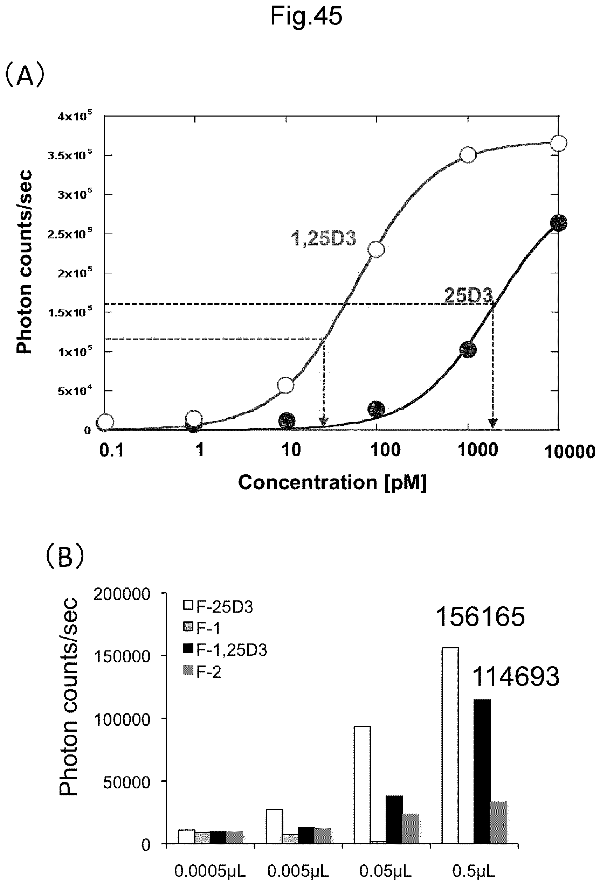

After double molecular type N29+N31 was plated at 96 well plate, 0.1, 1, 10, 100, 1000, 10000 pM 25 (OH) D₃ or 1α,25(OH)₂D₃ andF-1, F-25D₃, F-2, F-1,25D₃ fraction extracted and sampled in EXAMPLE 50 were added in 0.5 µL per well for 10 minutes. Then, light emission 10 minutes after adding the light emission substrate solution was measured as shown in EXAMPLE 46, and 25(OH)D₃ and 1α,25(OH)₂D₃ in the F-25D3, F-1,25D3 fractions were calculated from the standard curve (Fig.45A) and the value of light emission (Fig.45B). As a result, the concentration of 25(OH)D₃ was calculated to be 1.91 nM, the concentration of 1α,25(OH)₂D₃ was calculated to be 26 pM, and the concentration of 25(OH)D₃ was close to the concentration analyzed by LC/MS/MS in EXAMPLE 51.

### INDUSTRIAL APPLICABILITY

The present invention has utility in the field related to detecting and searching for substance with binding property to vitamin D binding domain of VDR.

### Sequence Listing Free Text

SEQ ID NO: 1-34: PCR-specific primers
SEQ ID NO: 35: <Firefly luciferase gene sequence>
SEQ ID NO: 36: <Firefly luciferase amino acid sequence>
SEQ ID NO: 37: < *Renilla reniformis* luciferase gene sequence >
SEQ ID NO: 38: < *Renilla reniformis* luciferase amino acid sequence>
SEQ ID NO: 39: <Emerald luciferase gene sequence>
SEQ ID NO: 40: <Emerald luciferase amino acid sequence>
SEQ ID NO: 41: <Human Vitamin D Receptor base sequence>
SEQ ID NO: 42: <Human Vitamin D Receptor amino acid sequence>
SEQ ID NO: 43: <Mice Vitamin D Receptor base sequence>
SEQ ID NO: 44: <Mice Vitamin D Receptor amino acid sequence>
SEQ ID NO: 45: <Rat Vitamin D Receptor base sequence>
SEQ ID NO: 46: <Rat Vitamin D Receptor amino acid sequence>
SEQ ID NO: 47: <Monkey vitamin D Receptor amino acid sequence>
SEQ ID NO: 48: <Canine Vitamin D Receptor amino acid sequence>
SEQ ID NO: 49: <Bovine Vitamin D Receptor amino acid sequence>
SEQ ID NO: 50 to 52: LX¹X²LL sequence-related array
SEQ ID NO: 53-100: Various primers
SEQ ID NO: 101: NLuc base sequence
SEQ ID NO: 102: LgBiT base sequence
SEQ ID NO: 103: SmBiT base sequence
SEQ ID NO: 104: NLuc amino acid sequence
SEQ ID NO: 105: LgBiT amino acid sequence
SEQ ID NO: 106: SmBiT amino acid sequence
SEQ ID NO: 107: *Oplophorus gracilirostris* (deep-sea shrimp) luciferase gene sequence
SEQ ID NO: 108: *Oplophorus gracilirostris* luciferase amino acid sequence
SEQ ID NO: 109 to 119: LX¹X²LL sequence-related array

## Claims

1. A fused protein of any one of (1) to (3) below:
(1) a fused protein comprising in random order N terminal domain of luciferase (hereinafter referred to as LucN), at least one vitamin D binding domain of vitamin D receptor (hereinafter referred to as VDR) and C terminal domain of luciferase (hereinafter referred to as LucC), and further comprising in random order one or both of LX¹X²LL sequences, wherein X¹ is M, W, T, S, E, V, R, Q, K, L or H and X² is N, T, S, G, E, R, Q, Y or K, and a linker sequence,
(2) a fused protein comprising in random order LucN or LucC and LX¹X²LL sequence, wherein X¹ is M, W, T, S, E, V, R, Q, K, L or H and X² is N, T, S, G, E, R, Q, Y or K, wherein the fused protein may comprise further in random order a linker sequence,
(3) a fused protein comprising in random order at least one vitamin D binding domain of VDR and LucC or LucN, wherein the fused protein may comprise further in random order a linker sequence.

2. The fused protein according to claim 1, wherein the linker sequence is at least one selected from the group consisting of a GGGGS sequence and an EAAAK sequence, preferably 3 repeating sequences of GGGGS, (GGGGS)×3.

3. The fused protein according to claim 1 or 2, wherein the fused protein (1) or (2) further comprises an insertion sequence A of 1 to 20 of amino acid number between LucN or LucC and LX¹X²LL sequence.

4. The fused protein according to any one of claims 1 to 3, wherein the fused protein (1) or (2) further comprises an insertion sequence B of 1 to 20 of amino acid number between LX¹X²LL sequence and linker sequence.

5. The fused protein according to any one of claims 1 to 4, wherein the luciferase is selected from the group consisting of Firefly luciferase; *Pyrophorus plagiophthalamus* luciferase; Emerald luciferase; *Renilla reniformis* luciferase; *Cypridina noctiluca* luciferase; *Gaussia princeps* luciferase; *Oplophorus gracilirostris* luciferase; and variant of *Oplophorus gracilirostris* luciferase.

6. The fused protein according to any one of claims 1 to 4, wherein Firefly luciferase has an amino acid sequence denoted by SEQ ID NO: 36 of Sequence Listing, and the N terminal domain has an amino acid sequence of number 1 to 415 and the C terminal domain has an amino acid sequence number 416 to 550.

7. The fused protein according to any one of claims 1 to 4, wherein *Renilla reniformis* luciferase has an amino acid sequence denoted by SEQ ID NO: 38 of Sequence Listing, and the N terminal domain has a number 1 to 229 amino acid sequence and the C terminal domain has a number 230 to 311 amino acid sequence.

8. The fused protein of any one of claims 1 to 4, wherein Emerald luciferase has an amino acid sequence denoted by SEQ ID NO: 40 of Sequence Listing, and the N terminal domain has an amino acid sequence of number 1 to n and the C terminal domain has an amino acid sequence of number n+1 to 542, and n is an integer of any of 388 to 422.

9. The fused protein according to any one of claims 1 to 4, wherein the variant of *Oplophorus gracilirostris* luciferase (hereinafter referred to as NLuc) has an amino acid sequence denoted by SEQ ID NO: 104 of Sequence Listing, and the N terminal domain of NLuc has an amino acid sequence denoted by SEQ ID NO: 105 of Sequence Listing and the C terminal domain of NLuc has an amino acid sequence denoted by SEQ ID NO: 106 of Sequence Listing.

10. The fused protein according to any one of claims 1 to 9, wherein the VDR is a VDR of human, mouse, rat, monkey, dog, or cattle.

11. The fused protein according to claim 10, wherein the human VDR has an amino acid sequence denoted SEQ ID NO: 42 of Sequence Listing of which vitamin D binding domain has an amino acid sequence of numbers 121 to 427,
the mouse VDR has an amino acid sequence denoted by SEQ ID NO: 44 of Sequence Listing, vitamin D binding domain of which has an amino acid sequence of numbers 121 to 422,
the Rat VDR has an amino acid sequence denoted SEQ ID NO: 46 of Sequence Listing, vitamin D binding domain of which has an amino acid sequence of numbers 121 to 423,
the Monkey VDR has an amino acid sequence denoted SEQ ID NO: 47 of Sequence Listing, vitamin D binding domain of which has an amino acid sequence of numbers 121 to 434,
the Dog VDR has an amino acid sequence denoted SEQ ID NO: 48 of Sequence Listing, vitamin D binding domain of which has an amino acid sequence of numbers 121 to 427,
the cattle VDR has an amino acid sequence denoted by SEQ ID NO: 49 of Sequence Listing, vitamin D binding domain of which has an amino acid sequence of numbers 121 to 426.

12. The fused protein according to any one of claims 1 to 11, wherein the amino acid sequence of vitamin D binding domain has mutation.

13. The fused protein according to any one of claims 1 to 12, which has two vitamin D binding domains.

14. The fused protein according to any one of claims 1 to 13, further comprising two amino acids of any of VD, GS, VE, RS, or EF between any of LucN, LucC, LX¹X²LL sequence, linker sequence, insertion sequence A and insertion sequence B.

15. A DNA encoding amino acid sequence of the fused protein according to any one of claims 1 to 14.

16. A plasmid comprising the DNA according to claim 15 in a vector.

17. A transformed cell which is a cell into which the plasmid according to claim 16 has been introduced.

18. The transformed cell according to claim 17, wherein said cell is a cell derived from a mammal or an insect.

19. A screening method of a substance that shows binding property to vitamin D receptor (hereinafter referred to as VDR) comprising
cultivating the transformed cell according to claims 17 or 18 in the presence of a substrate of luciferase of which DNA is contained in the transformed cell, Mg²⁺, ATP and oxygen, and candidate substance, wherein a fused protein of the luciferase is fused protein (1) or fused protein (2) and fused protein (3), wherein when the fused protein (2) comprises LucN, the fused protein (3) comprises LucC and when the fused protein (2) comprises LucC, the fused protein (3) comprises LucN;
measuring light emission in cultivation and/or after cultivation;
assessing binding of candidate substance to VDR based on the light emission; and
selecting a substance exhibiting binding property to VDR.

20. The sequence method according to claim 19, wherein a DNA encoding amino acid sequence of vitamin D binding domain, which is contained in the plasmid introduced into transformed cell has a mutation in the amino acid sequence of vitamin D binding domain, and a candidate substance which specifically binds to a vitamin D binding domain having a mutation which is involved in disease is screened from the relation between the mutation of amino acid sequence and disease.

21. A screening method of substance that shows binding property to vitamin D receptor (hereinafter referred to as VDR) comprising
subjecting the fused protein according to any one of claims 1 to 14 to interaction with a coexistent substrate of luciferase, Mg²⁺, ATP and oxygen, and candidate substance wherein the fused protein is fused protein (1) or fused protein (2) and fused protein (3), wherein when the fused protein (2) comprises LucN, the fused protein (3) comprises LucC and when the fused protein (2) comprises LucC, the fused protein (3) comprises LucN;
measuring light emission during the interaction and/or after the interaction; assessing binding of candidate substance to VDR based on the light emission; and
selecting a substance exhibiting binding property to VDR.

22. Screening method according to claim 21, wherein an amino acid sequence of the vitamin D binding domain contained in the fused protein has a mutation, and a candidate substance that specifically binds to a vitamin D binding domain having a mutation involved in disease is screened from the relation between mutation of amino acid sequence and disease.

23. The screening method according to any one of claims 19 to 22, wherein the candidate substance is a compound having steroid nucleus, fatty acids, fat-soluble vitamins, or polyphenols.
